# EUROPEAN PATENT SPECIFICATION

(11) **EP 3 762 026 B1**
(45) Date of publication and mention of the grant of the patent: **08.04.2026**
(21) Application number: 19715280.4
(22) Date of filing: 06.03.2019
(51) Int. Cl.: C07K 16/40, A61K 39/00, G01N 33/92, A61P 9/00

(54) **USE OF PCSK9 INHIBITOR FOR REDUCING CARDIOVASCULAR RISK**
VERWENDUNG VON PCSK9 INHIBITOREN ZUR VORBEUGUNG VON HERZ-KREISLAUF-RISIKEN
UTILISATION D'INHIBITEUR DE PCSK9 POUR RÉDUIRE LE RISQUE CARDIOVASCULAIRE

(30) Priority: 06.03.2018 US 201862639407 P; 08.03.2018 US 201862640361 P; 09.03.2018 US 201862641082 P; 12.03.2018 US 201862641918 P; 13.04.2018 US 201862657495 P; 12.06.2018 US 201862683695 P; 22.06.2018 US 201862688622 P; 10.08.2018 US 201862717530 P; 25.09.2018 US 201862736284 P; 10.10.2018 US 201862744008 P; 16.10.2018 US 201862746319 P; 21.11.2018 US 201862770530 P; 04.12.2018 US 201862775219 P; 28.01.2019 US 201962797680 P; 07.02.2019 US 201962802545 P; 15.02.2019 US 201962806313 P; 04.03.2019 EP 19305247
(43) Date of publication of application: 13.01.2021
(73) Proprietor: Sanofi Biotechnology, 75008 Paris (FR); Regeneron Pharmaceuticals, Inc., Tarrytown, NY 10591 (US)
(72) Inventor: SCHWARTZ, Gregory, Denver, Colorado 80220 (US); PORDY, Robert, New York 10591 (US); BESSAC, Laurence, 75008 Paris (FR); HANOTIN, Corinne, 75008 Paris (FR); SASIELA, William, New York 10591 (US); STEG, Philippe, 75008 Paris (FR)
(74) Representative: Zwicker, Jörk
(86) International application number: PCT/US2019/021034
(87) International publication number: WO 2019/173530

(56) References cited:
- WO-A1-2015/142668
- KEREIAKES DEAN J. ET AL: "Efficacy and safety of the proprotein convertase subtilisin/kexin type 9 inhibitor alirocumab among high cardiovascular risk patients on maximally tolerated statin therapy: The ODYSSEY COMBO I study", AMERICAN HEART JOURNAL, vol. 169, no. 6, 1 June 2015 (2015-06-01), AMSTERDAM, NL, pages 906 - 915.e13, XP093249425, ISSN: 0002-8703, DOI: 10.1016/j.ahj.2015.03.004
- BRANDICOURT OLIVIER ET AL.: "Results of ODYSSEY OUTCOMES TrialEvaluation of long-term cardiovascular outcomes after Acute Coronary Syndrome (ACS) during treatment with Praluent® (alirocumab)", 15 November 2015 (2015-11-15), XP002791322, Retrieved from the Internet <URL:https://www.sanofi.com/-/media/Project/One-Sanofi-Web/Websites/Global/Sanofi-COM/Home/common/docs/investors/FINAL_ACC_Praluent_ODYSSEY_OUTCOMES_March10_LBCT_presentation_Web.pdf?la=en&hash=6EDCA3072D73C2ADD56C8DDA044E6275B6CE7502> [retrieved on 20190512]
- UNKNOWN: "ODYSSEY LONG TERM", 13 December 2016 (2016-12-13), XP002791323, Retrieved from the Internet <URL:https://www.wikijournalclub.org/wiki/ODYSSEY_LONG_TERM> [retrieved on 20190515]
- RAY KAUSIK K ET AL: "Reductions in Atherogenic Lipids and Major Cardiovascular Events A Pooled Analysis of 10 ODYSSEY Trials Comparing Alirocumab With Control", CIRCULATION, vol. 134, no. 24, 13 December 2016 (2016-12-13), pages 1931 - 1943, XP002791324, DOI: 10.1161/CIRCULATIONAHA.116.024604
- RAY KAUSIK K ET AL: "Supplemental Material - Reductions in Atherogenic Lipids and Major Cardiovascular Events:A Pooled Analysis of 10 ODYSSEY Trials Comparing Alirocumab With Control", 21 November 2016 (2016-11-21), pages 1 - 23, XP002792195, Retrieved from the Internet <URL:https://www.ahajournals.org/action/downloadSupplement?doi=10.1161%2FCIRCULATIONAHA.116.024604&file=supplemental_material_nov_21.pdf>
- ROBINSON JENNIFER G ET AL: "Efficacy and Safety of Alirocumab in Reducing Lipids and Cardiovascular Events", NEW ENGLAND JOURNAL OF MEDICINE, vol. 372, no. 16, 16 April 2015 (2015-04-16), pages 1489 - 1499, XP002791325, DOI: 10.1056/NEJMoa1501031
- ROBINSON JG, FARNIER M, KREMPF M,: "Efficacy and safety of alirocumab in reducing lipids andcardiovascular eventsSupplementary Appendix:", April 2015 (2015-04-01), pages Frontpg. - 75, XP002792196, Retrieved from the Internet <URL:https://www.nejm.org/doi/suppl/10.1056/NEJMoa1501031/suppl_file/nejmoa1501031_appendix.pdf>
- GIUGLIANO ROBERT P ET AL: "Clinical efficacy and safety of achieving very low LDL-cholesterol concentrations with the PCSK9 inhibitor evolocumab: a prespecified secondary analysis of the FOURIER trial", LANCET (NORTH AMERICAN EDITION), vol. 390, no. 10106, 28 October 2017 (2017-10-28), pages 1962 - 1971, XP002791326

## Description

### FIELD OF THE INVENTION

The present invention relates to the field of therapeutic treatments of diseases and disorders that are associated with elevated levels of lipids and lipoproteins. More specifically, the invention relates to a PCSK9 antibody or antigen-binding fragment thereof for use in reducing the risk of a major cardiovascular event, including mortality, in high cardiovascular risk patients following acute coronary syndrome despite a maximally tolerated statin therapy.

### BACKGROUND

Despite modern therapy including prompt coronary revascularization, dual anti-platelet therapy, and intensive statin treatment, cardiovascular events occur with high frequency following acute coronary syndrome (ACS). Registry data indicates cardiovascular mortality as high as 13% at 5 years, with an overwhelming majority occurring after initial discharge from the hospital. Patients with recent acute coronary syndrome (ACS) are at very high risk for suffering recurrent coronary events in the near term. In approximately 10% of patients with ACS, cardiovascular death, recurrent myocardial infarction, or stroke occurs within 1 year. Based on the results of large clinical trials, early intensive statin therapy has become formally endorsed as a treatment recommendation for patients with ACS. Both epidemiological and pharmacological intervention trials have demonstrated a strong and linear relationship between the levels of low-density lipoprotein cholesterol (LDL-C) and cardiovascular (CV) events. However, many high CV risk patients cannot achieve such levels with currently available lipid lowering drugs. Furthermore, a significant number of high-risk patients even fail to achieve their recommended LDL-C target levels and most CV events are actually not prevented, leaving a substantial "residual risk" for patients. WO 2015/142668 discloses methods for reducing cardiovascular risk using PCSK9 antibody alirocumab in patients with high cardiovascular risk within 12 months following an acute coronary syndrome event despite a maximum tolerated dose statin therapy. Thus, additional pharmacologic therapies for treating patients with high cardiovascular risk, including to reduce mortality.

### BRIEF SUMMARY OF THE INVENTION

The present invention provides a proprotein convertase subtilisin/kexin type 9 (PCSK9) antibody or antigen-binding fragment thereof for use in a method for reducing the risk of major adverse cardiovascular events (MACE) comprising administering to a patient in need thereof a therapeutically effective amount of said PCKS9 antibody or antigen-binding fragment thereof for at least three years, wherein the antibody or antigen-binding fragment thereof comprises complementarity determining regions (CDRs) of a heavy chain variable region (HCVR) and a light chain variable region (LCVR) comprising amino acid sequences set forth in SEQ ID NOs: 1 and 6, respectively, and wherein the patient is a high cardiovascular risk patient who had an acute coronary syndrome (ACS) event between about 1 to about 12 months prior to the administration of the antibody or antigen binding fragment thereof, wherein the patient has an LDL-C level greater than or equal to 100 mg/dL despite treatment with maximally tolerated statin therapy. The present invention is particularly useful for reducing cardiovascular risk and/or events. The invention is based, in part, on clinical trial results disclosed herein that demonstrated, for the first time, that treatment with a PCSK9 inhibitor was associated with a reduced risk of death in high cardiovascular risk patients receiving a maximally tolerated statin therapy.

In certain embodiments, the patient's risk of mortality is reduced by about 15%. In certain other embodiments, the patient's risk of mortality is reduced by about 29%. In certain embodiments, the method increases the survival time of the patient. In certain embodiments, the mortality is cardiovascular disease death ("CV death"). In other embodiments, the mortality is non-cardiovascular death ("non-CV death"). In certain embodiments, the non-CV death is due to pulmonary infection, pulmonary malignancy, gastrointestinal/ hepatobiliary/ pancreatic infection, gastrointestinal/hepatobiliary/pancreatic malignancy, hemorrhage, a neurological process that is not a stroke/hemorrhage, suicide, a non-cardiovascular procedure or surgery, accident or trauma, renal infection, renal malignancy, other non-cardiovascular infection, or other non-cardiovascular malignancy.

In certain embodiments, the patient's risk of coronary heart disease (CHD) death, non-fatal myocardial infarction, unstable angina requiring hospitalization, or ischemic stroke is reduced by about 15%. In certain other embodiments, the patient's risk of CHD death, non-fatal myocardial infarction, unstable angina requiring hospitalization, or ischemic stroke is reduced by about 24%. In certain embodiments, administration of the antibody or antigen-binding fragment thereof increases the time to first occurrence of CHD death, non-fatal myocardial infarction, unstable angina requiring hospitalization, or ischemic stroke in the patient. In certain embodiments, the high cardiovascular risk patient has an Lp(a) level greater than or equal to 20 mg/dL as measured by an automated latex-enhanced nephelometric assay despite treatment with a maximally tolerated statin therapy. In some embodiments, the high cardiovascular risk patient has peripheral artery disease (PAD). In some embodiments, the high cardiovascular risk patient has cerebrovascular disease (CeVD). In some embodiments, the patient has polyvascular disease. In some embodiments, the high cardiovascular risk patient has had a prior coronary artery bypass graft (CABG).

In certain embodiments, the high cardiovascular risk patient has diabetes. In certain embodiments, administration of the antibody or antigen-binding fragment thereof reduces the diabetic patient's risk of coronary heart disease (CHD) death, non-fatal myocardial infarction, unstable angina requiring hospitalization, or ischemic stroke by about 16%.

In certain embodiments, the CHD event comprises CHD death, non-fatal myocardial infarction, unstable angina requiring hospitalization, or ischemia-driven coronary revascularization procedure. In certain embodiments, the patient's risk of a CHD event is reduced by about 12%. In certain other embodiments, the patient's risk of a CHD event is reduced by about 22%. In certain embodiments, administration of the antibody or antigen-binding fragment thereof increases the time to first occurrence of a CHD event in the patient.

In certain embodiments, the major CHD event comprises CHD death or non-fatal myocardial infarction. In certain embodiments, the patient's risk of a major CHD event is reduced by about 12%. In certain other embodiments the patient's risk of a major CHD event is reduced by about 23%. In certain embodiments, the method increases the time to first occurrence of major CHD event in the patient.

In certain embodiments, the patient's risk of CHD death is reduced by about 28%.

In certain embodiments, the cardiovascular event comprises non-fatal a non-fatal CHD event, cardiovascular death, or non-fatal ischemic stroke. In certain embodiments, the non-fatal CHD event comprises non-fatal myocardial infarction, unstable angina requiring hospitalization, or ischemia-driven coronary revascularization procedure. In certain embodiments, the patient's risk of a cardiovascular event is reduced by about 13%. In certain other embodiments, the patient's risk of a cardiovascular event is reduced by about 22%. In certain embodiments, administration of the antibody or antigen-binding fragment thereof increases the time to first occurrence of a cardiovascular event in the patient.

In certain embodiments, the patient's risk of a cardiovascular event is reduced by about 31%. In certain embodiments, administration of the antibody or antigen-binding fragment thereof increases the time to first occurrence of cardiovascular death in the patient.

In certain embodiments, the non-fatal cardiovascular event comprises myocardial infarction, stroke, unstable angina requiring hospitalization, heart failure requiring hospitalization, or an ischemia-driven coronary revascularization procedure. In certain embodiments, the patient's risk of a non-fatal cardiovascular event is reduced by about 15%.

In certain embodiments, the non-fatal CV event comprises myocardial infarction, stroke, unstable angina requiring hospitalization, heart failure requiring hospitalization, or ischemia driven coronary revascularization procedure. In certain embodiments, the patient has experienced one, two, three, four, or more than four non-fatal cardiovascular events. In one embodiment, the initial non-fatal CV event is a first non-fatal CV event and the subsequent non-fatal CV event is a second non-fatal CV event. In another embodiment, the initial non-fatal CV event is a second non-fatal CV event and the subsequent non-fatal CV event is a third non-fatal CV event. In another embodiment, the initial non-fatal CV event is a third non-fatal CV event and the subsequent non-fatal CV event is a fourth non-fatal CV event. In another embodiment, the initial non-fatal CV event is a fourth non-fatal CV event and the subsequent non-fatal CV event is a fifth non-fatal CV event. In certain embodiments, the patient has experienced more than four non-fatal CV events.

In certain embodiments, the non-fatal cardiovascular event comprises non-fatal myocardial infarction, non-fatal stroke, or non-fatal unstable angina. In certain embodiments, the patient's risk of non-fatal cardiovascular events is reduced, thereby leading to a reduction in the patient's risk of non-cardiovascular death. In certain embodiments, the patient's risk of non-cardiovascular death is reduced by about 19%.

In certain embodiments, the patient's risk of mortality, non-fatal myocardial infarction, or non-fatal ischemic stroke is reduced by about 14%. In certain other embodiments the patient's risk of mortality, non-fatal myocardial infarction, or non-fatal ischemic stroke is reduced by about 23%. In certain embodiments, administration of the antibody or antigen-binding fragment thereof increases the time to first occurrence of mortality, non-fatal myocardial infarction, or non-fatal ischemic stroke in the patient. In certain embodiments, the mortality is CV death. In other embodiments, the mortality is non-CV death.

In certain embodiments, the patient's risk of ischemic stroke is reduced by about 27%. In certain other embodiments, the patient's risk of ischemic stroke is reduced by about 40%. In certain embodiments, administration of the antibody or antigen-binding fragment thereof increases the time to first occurrence of ischemic stroke in the patient.

In certain embodiments, the patient's risk of unstable angina requiring hospitalization is reduced by about 39%. In certain other embodiments, the patient's risk of unstable angina requiring hospitalization is reduced by about 52%. In certain embodiments, administration of the antibody or antigen-binding fragment thereof increases the time to first occurrence of unstable angina requiring hospitalization in the patient.

In certain embodiments, the patient's risk of non-fatal myocardial infarction is reduced by about 14%. In one embodiment, the myocardial infarction is a type 1 myocardial infarction. In certain embodiments, the patient's risk of type 1 myocardial infarction is reduced by about 13%. In another embodiment, the myocardial infarction is a type 2 myocardial infarction. In certain embodiments, the patient's risk of type 2 myocardial infarction is reduced by about 23%. In certain embodiments, administration of the antibody or antigen-binding fragment thereof increases the time to first occurrence of non-fatal myocardial infarction in the patient.

In certain embodiments, the patient's risk of having of ischemia-driven coronary revascularization procedure is reduced by about 12%. In certain embodiments, administration of the antibody or antigen-binding fragment thereof increases the rate of occurrence of ischemia-driven coronary revascularization procedure in a high cardiovascular risk patient. In certain embodiments, administration of the antibody or antigen-binding fragment thereof increases the time to first occurrence of ischemia-driven coronary revascularization procedure in the patient.

In certain embodiments, the very high risk patient is selected by having either a history of multiple major ASCVD events, or one major ASCVD event and multiple high risk conditions. In certain embodiments, high risk conditions comprise heterozygous familial hypercholesterolemia, a history of prior coronary artery bypass surgery or percutaneous coronary intervention, diabetes mellitus, hypertension, CKD (eGFR 15-59 mL/min/1.73 m²), current smoking, persistently elevated LDL-C (LDL-C ≥100 mg/dL [≥2.6 mmol/L]) despite maximally tolerated statin therapy and ezetimibe, or a history of congestive heart failure.

In certain embodiments, the Lp(a) level is measured by an automated latex-enhanced nephelometric assay.

In certain embodiments of any one of the foregoing aspects, the patient has established cardiovascular disease. In certain embodiments, the patient has clinical atherosclerotic cardiovascular disease (ASCVD). In certain embodiments, the ACS event is defined by: (1) unstable symptoms of myocardial ischemia occurring at rest or minimal exertion within 72 hours of an unscheduled hospital admission, due to presumed or proven obstructive coronary disease; and (2) at least one of the following: (i) elevated cardiac biomarkers consistent with acute myocardial infarction; and (ii) resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.

In certain embodiments, the PCSK9 inhibitor is administered to the patient in combination with the maximally tolerated statin therapy. In certain embodiments, the maximally tolerated statin therapy comprises a daily dose of about 40 mg to about 80 mg of atorvastatin. In certain embodiments, the maximally tolerated statin therapy comprises a daily dose of about 20 mg to about 40 mg of rosuvastatin. In certain embodiments, the maximally tolerated statin therapy is a low or moderate dose of atorvastatin or rosuvastatin. In certain embodiments, the maximally tolerated statin therapy comprises a statin other than atorvastatin or rosuvastatin. In certain embodiments, the patient has been treated with the maximally tolerated statin therapy for at least two weeks.

In certain embodiments of the invention disclosed herein, the step of administering a PCSK9 antibody or antigen-binding fragment thereof (step (b)) comprises:
(i) administering to the patient one or more initial doses of 75 mg of the antibody or antigen-binding fragment thereof about every two weeks; and
(ii) administering to the patient one or more following doses of 75 mg of the antibody or antigen-binding fragment thereof about every two weeks if the LDL-C level in the patient after step (i) is lower than a threshold level, or administering to the patient one or more following doses of 150 mg of the antibody or antigen-binding fragment thereof about every two weeks if the LDL-C level in the patient after step (i) is greater than or equal to a threshold level.

In certain embodiments, the threshold level is 50 mg/dL. In certain embodiments, step (b) comprises administering to the patient one or more doses of 150 mg of the antibody or antigen-binding fragment thereof about every two weeks. In other embodiments, step (b) comprises administering to the patient one or more doses of 300 mg of the antibody or antigen-binding fragment thereof about every two weeks or about every four weeks.

In certain embodiments, the patient is intolerant to statins or has a history of adverse reactions to statin therapy. In certain embodiments, the patient's risk of coronary heart disease (CHD) death, non-fatal myocardial infarction, unstable angina requiring hospitalization, or ischemic stroke is reduced by about 35%.

Other aspects and embodiments of the present invention are disclosed in the accompanying claims and will further become apparent from a review of the ensuing detailed description.

### BRIEF DESCRIPTION OF THE FIGURES

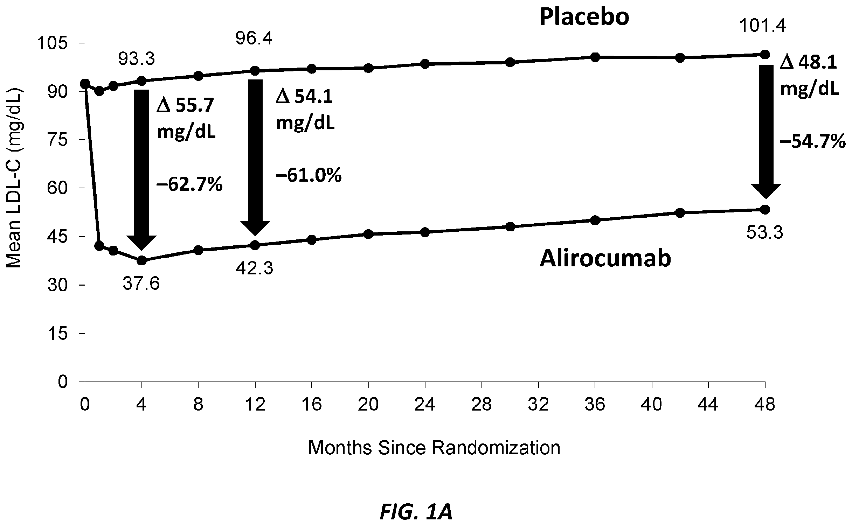

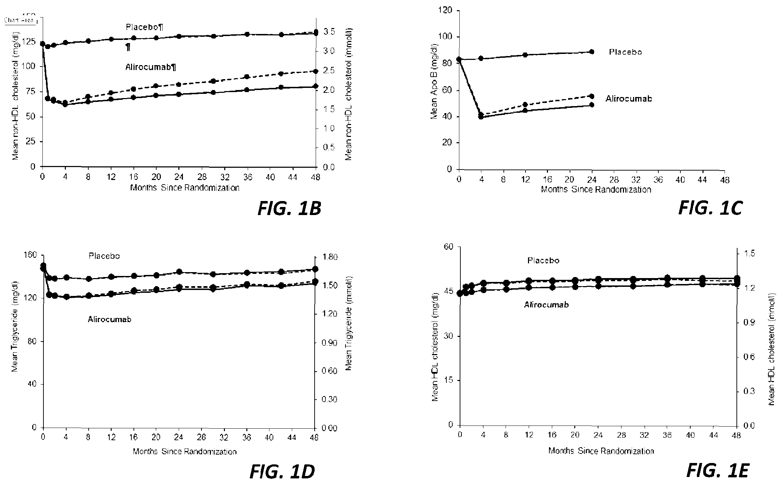

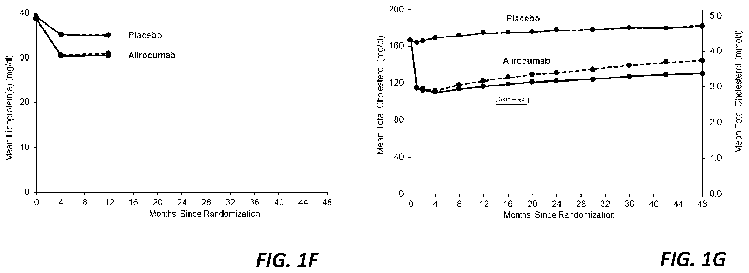

**Fig. 1A** **-** **Fig. 1G** graphically depict marker levels in an on-treatment population. Fig. 1A depicts a graph of the LDL-C values for the on-treatment population over 48 months for patients receiving placebo or alirocumab. **Fig. 1B** **-** **Fig. 1G** depict graphs showing the levels of non-HDL cholesterol (Fig. 1B), ApoB (Fig. 1C), triglyceride (Fig. 1D), HDL cholesterol (Fig. 1E), lipoprotein(a) (Fig. 1F), and total cholesterol (Fig. 1G) for patients receiving placebo or alirocumab. Intention-to-treat (ITT) levels, shown with dashed lines, include all values including those measured after premature treatment discontinuation, blinded alirocumab dose titration, or blinded switch from alirocumab to placebo. On-treatment levels, shown with solid lines, exclude values obtained after premature treatment discontinuation or blinded switch from alirocumab to placebo (but include values obtained after blinded alirocumab down-titration).

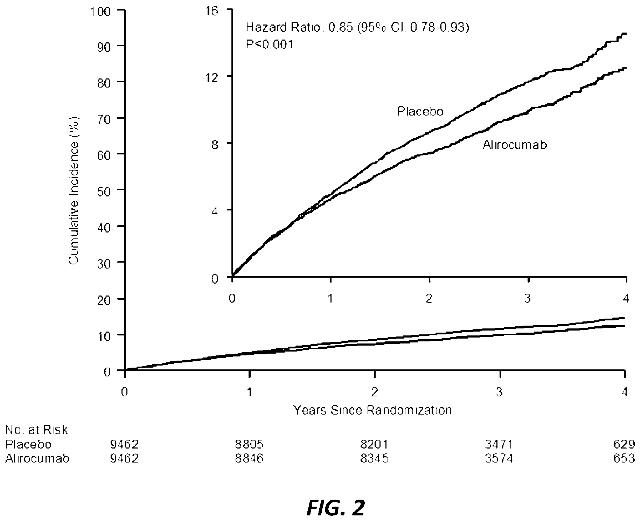

**Fig.** 2 depicts a graph of the primary efficacy endpoint, MACE, in patients treated with alirocumab or placebo. Alirocumab reduced the risk of MACE by 15% (HR=0.85, 0.78 to 0.93, p<0.001). Insets show the same data on an enlarged y axis. P values were calculated with the use of log-rank tests stratified by geographic region.

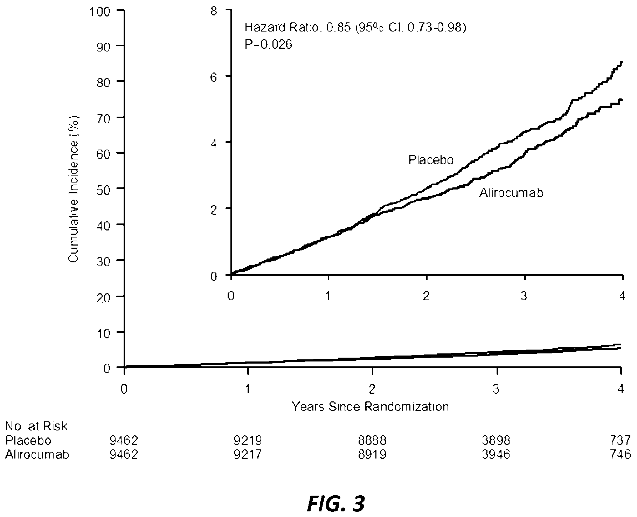

**Fig. 3** depicts a graph, with hazard ratio, of all-cause mortality in patients treated with alirocumab and placebo. Insets show the same data on an enlarged y axis. P values were calculated with the use of log-rank tests stratified by geographic region.

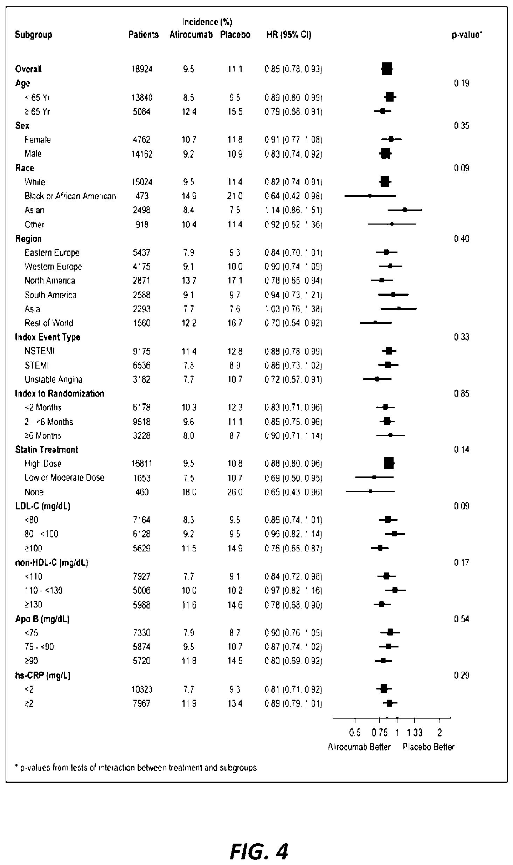

**Fig. 4** depicts the results, including hazard ratios, for analysis of various pre-specified subgroups, including the P-values from tests of interaction between the different subgroups. Pre-specified Cox proportional hazard regression analysis was performed in subgroups of patients with respect to primary outcomes (first occurrence of coronary heart disease death, non-fatal myocardial infarction, ischemic stroke, or hospitalization for unstable angina). Apo, apolipoprotein; Cl, confidence interval; HR, hazard ratio; hs-CRP, high-sensitivity C-reactive protein; NSTEMI, non-ST-segment elevation myocardial infarction; LDL-C, low-density lipoprotein cholesterol; STEMI, ST-segment elevation myocardial infarction.

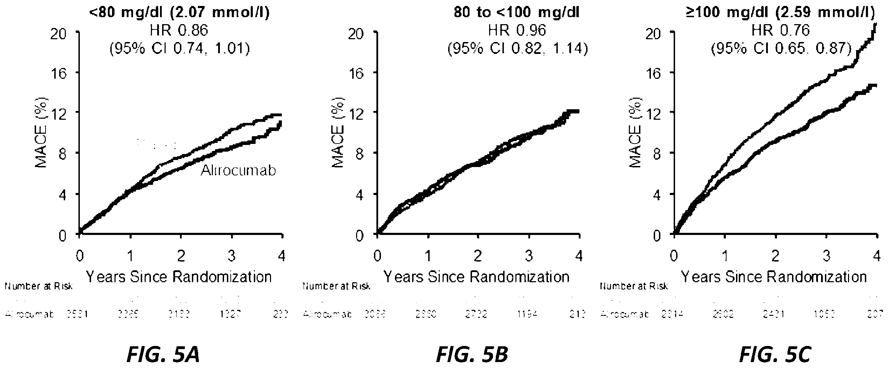

**Fig. 5A** **-** **Fig. 5C** depict a set of graphs, with hazard ratios, of MACE in three different subgroups of patients treated with alirocumab and placebo stratified by baseline LDL-C levels (< 80 mg/dL **(****Fig. 5A****),** 80-100 mg/dL **(****Fig. 5B****),** and ≥ 100 mg/dL **(****Fig. 5C****)).**

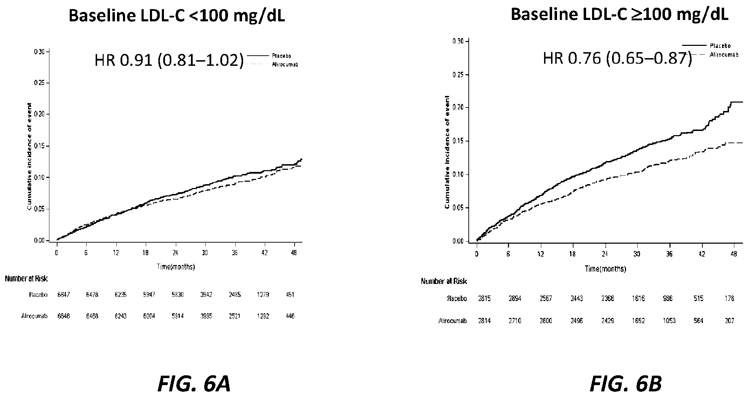

Fig. 6A - Fig. 6B depict a set of graphs, with hazard ratios, of MACE in two different subgroups of patients treated with alirocumab and placebo stratified by baseline LDL-C levels (<100 mg/dL **(****Fig. 6A****)** and ≧ 100 mg/dL **(****Fig. 6B****)).**

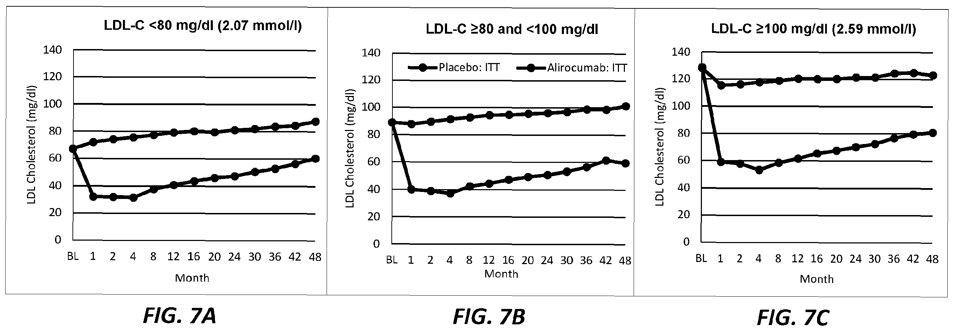

Fig. **7A** **-** **Fig. 7C** show the LDL-C levels examined in subgroups according to baseline LDL cholesterol levels of <80 mg per deciliter **(****Fig. 7A****), 80** to <100 mg per deciliter **(****Fig. 7B****),** or ≥100 mg per deciliter **(****Fig. 7C****).**

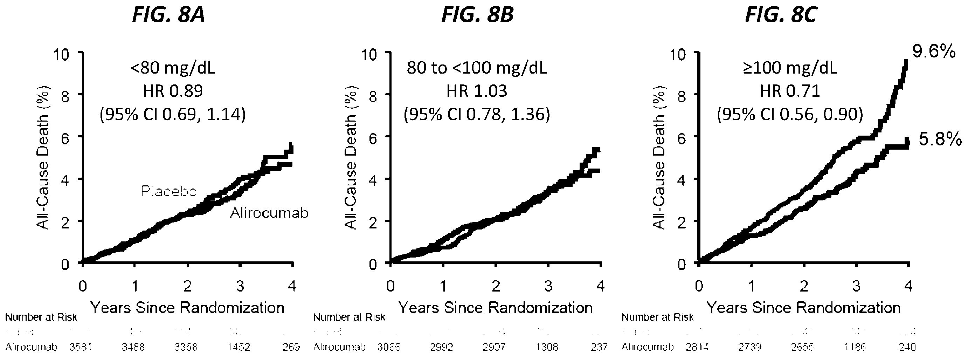

**Fig. 8A** **-** **Fig. 8C** are a set of graphs, with hazard ratios, of all-cause mortality in three different subgroups of patients treated with alirocumab and placebo stratified by baseline LDL-C levels (< 80 mg/dL **(****Fig. 8A****),** 80-100 mg/dL **(****Fig. 8B****),** and ≥ 100 mg/dL **(****Fig. 8C****)).** Relative risk reduction (RRR) interaction p=0.12; absolute risk reduction (ARR) interaction p=0.005.

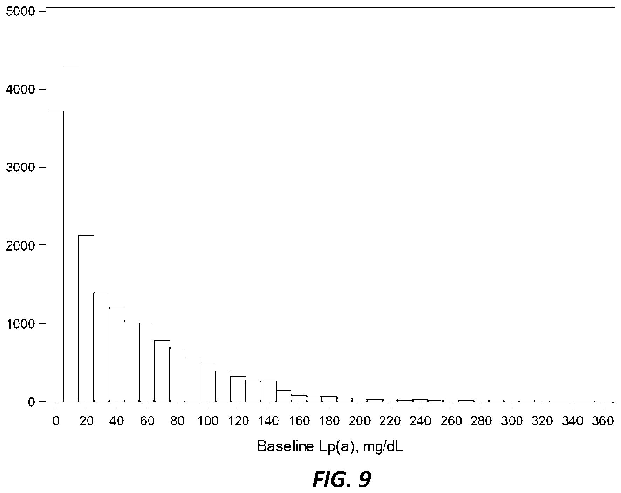

**Fig. 9** is a histogram showing the distribution of baseline Lp(a) levels. An approximate conversion from mg/dL to nmol/L can be computed as Lp(a) mg/dL* 0.357.

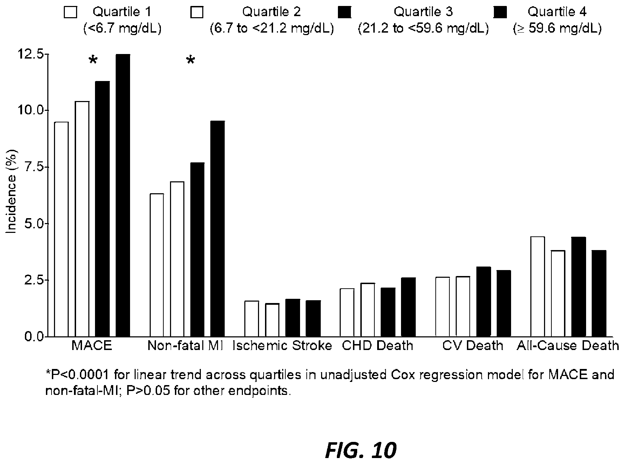

**Fig. 10** is a graph showing incidence of MACE, non-fatal myocardial infarction, ischemic stroke, CHD death, CV death and All-cause death shown by baseline Lp(a) quartiles for the placebo group.

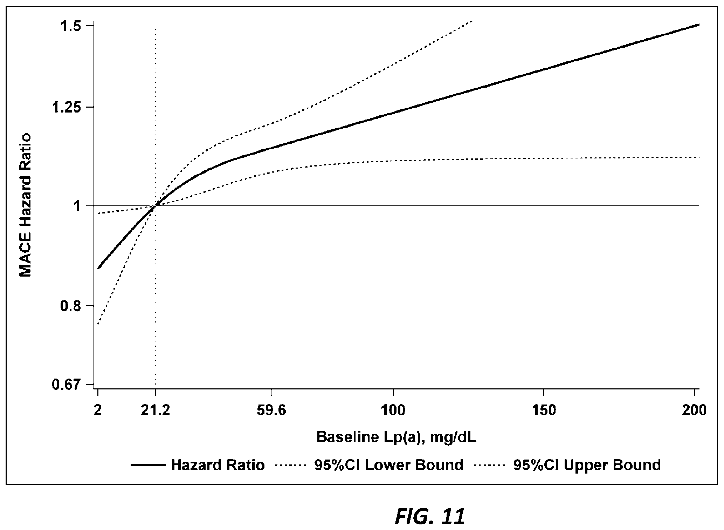

**Fig. 11** is a graph showing a spline of degree 3 (piecewise cubic curve) with natural cubic basis and 3 knots, located at overall 25th percentile (6.7 mg/dL), median (21.2 mg/dL), and 75th percentile (59.6 mg/dL). P-value for spline effect based on score test. Hazard ratio set to 1.00 at the overall baseline median (21.2 mg/dL). P<0.0001 for spline effect.

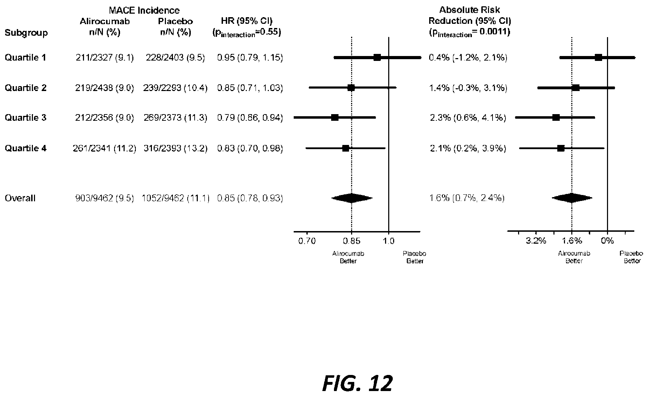

**Fig. 12** is a Forest plot depicting relative and absolute risk reduction with alirocumab compared to placebo. MACE incidence is shown for the alirocumab and placebo groups stratified by baseline quartile of Lp(a) and for the overall population.

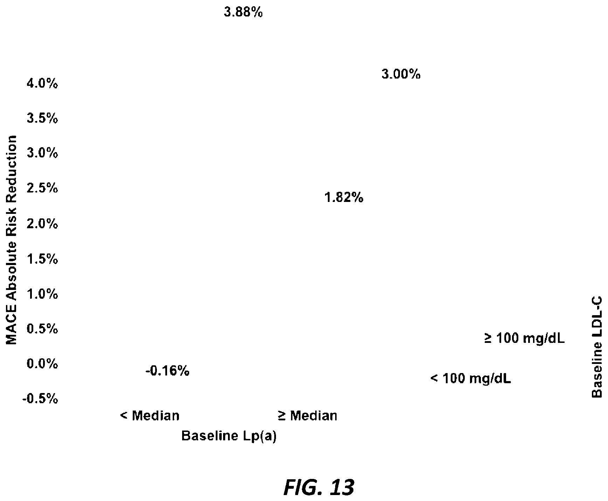

**Fig. 13** is a graph depicting absolute risk reduction in MACE stratified by baseline Lp(a) and baseline LDL-C. Baseline LDL-C uncorrected for Lp(a).

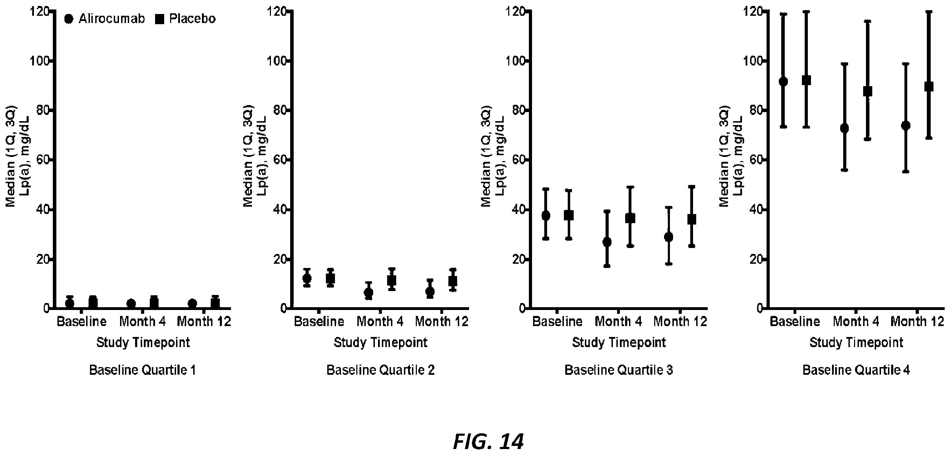

**Fig. 14** depicts distributions of Lp(a) (median and interquartile range) in the alirocumab and placebo groups at baseline, Month 4, and Month 12, stratified by baseline quartile of Lp(a).

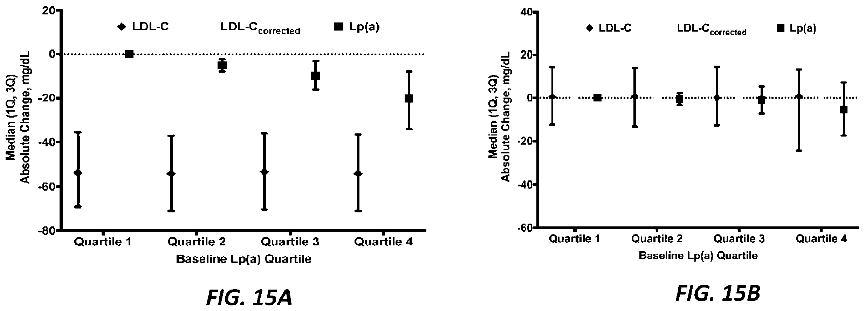

**Fig. 15A****-** **Fig. 15B** depicts median and interquartile range of absolute change from baseline to Month 4 for LDL-Cholesterol, corrected LDL-cholesterol, and Lp(a), stratified by Lp(a) quartile in the alirocumab treatment group **(****Fig. 15A****)** and the placebo group **(****Fig. 15B****).**

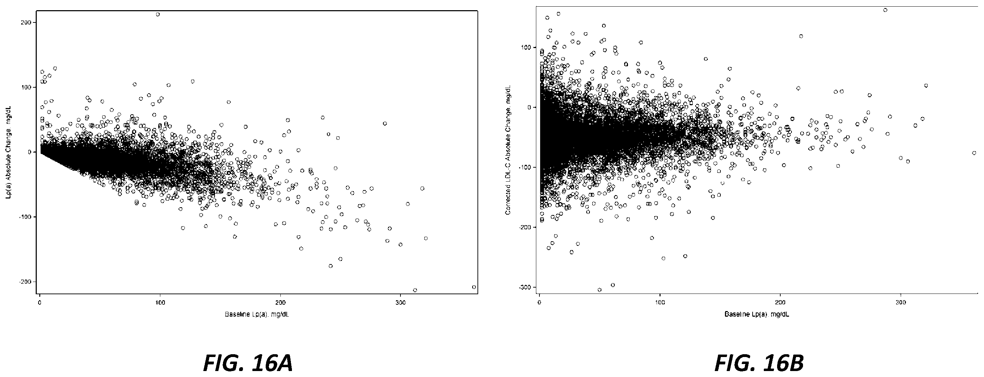

**Fig. 16A****-****Fig. 16B** depicts a plot of the correlation of baseline lipoprotein(a) versus absolute change in lipoprotein(a) **(****Fig. 16A****)** and baseline lipoprotein(a) versus absolute change in LDL-Cc_{orrected} **(****Fig. 16B****).** Pearson correlations between baseline lipoprotein(a) and absolute change in lipoprotein(a) and LDL-C_{corrected} are -0.56 (P<0.0001) and 0.05 (P<0.0001), respectively.

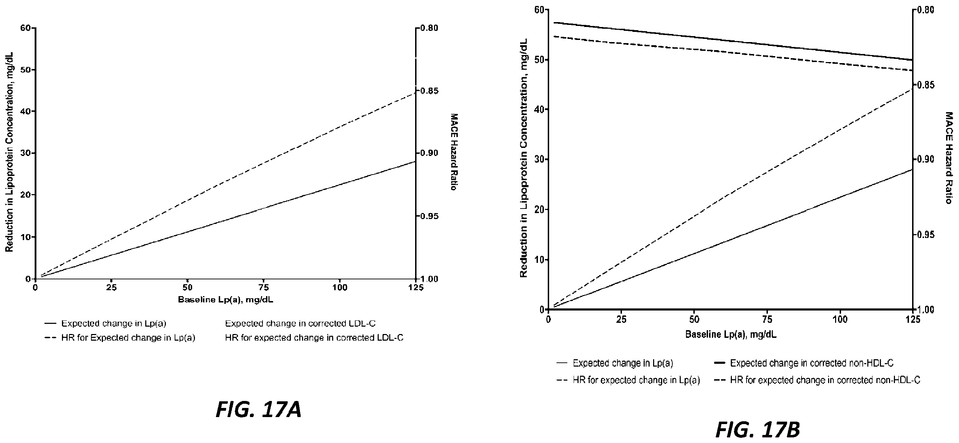

**Fig. 17A****-** **Fig. 17B** depicts the relationship between absolute change in Lp(a) at Month 4 and risk of MACE is shown across the distribution of baseline Lp(a) from 2 to 150 mg/dl. **Fig. 17A** shows the results from a model adjusting for baseline and change from baseline to Month 4 in LDL-C_{corrected} (Model 3A). **Fig. 17B** shows the results from a model adjusting for baseline and change from baseline to Month 4 in non-HDL-C_{corrected} (Model 3B).

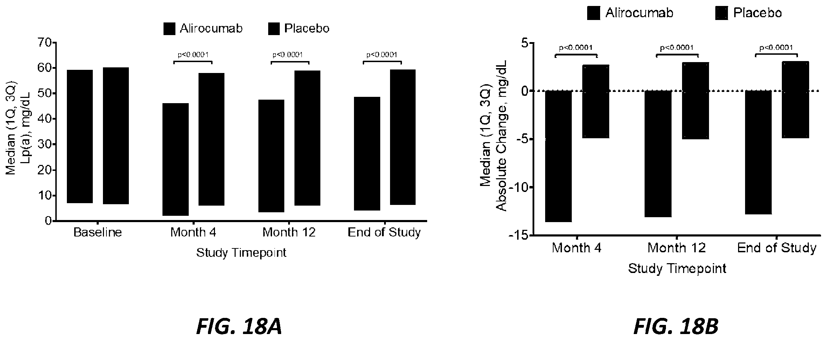

**Fig. 18A** is a graph showing the first quartile ("1Q"), median, and third quartile ("3Q") of the Lp(a) levels in the patients receiving placebo or alirocumab over the course of the study. **Fig. 18B** is a graph showing the first quartile ("1Q"), median, and third quartile ("3Q") of the absolute changes in Lp(a) levels in the patients receiving placebo or alirocumab over the course of the study.

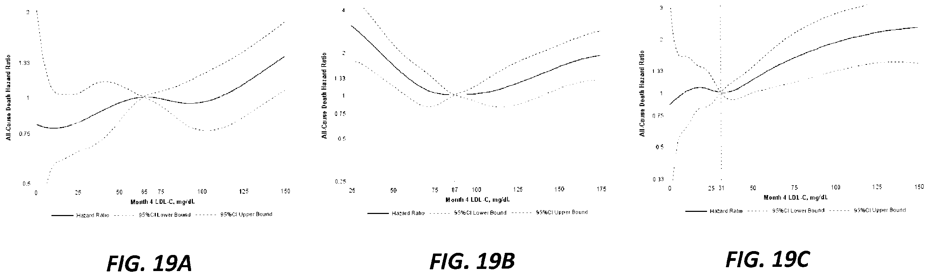

Fig. 19A - Fig. 19C shows all-cause death spline analysis of continuous intention-to-treat month-4 LDL-C for: all patients **(****Fig. 19A****;** * HR is relative to median month-4 LDL-C (1.68 mmol/L [65 mg/dL]), stratified by region. Degree = 3, 3 knots located at month-4 LDL-C quartiles (0.80, 1.68, and 2.38 mmol/L [31, 65, and 92 mg/dL]), p value for model = 0.0070.); placebo-treated patients (Fig. **19B****;** HR is relative to median month-4 LDL-C (2.25 mmol/L [87 mg/dL]), stratified by region. Degree = 3, 3 knots located at month-4 LDL-C quartiles (1.86, 2.25, and 2.64 mmol/L [72, 87, and 102 mg/dL]), p value for model = 0.0007.); and alirocumab-treated patients **(****Fig. 19C****;** HR is relative to median month-4 LDL-C (0.80 mmol/L [31 mg/dL]), stratified by region. Degree = 3, 3 knots located at month-4 LDL-C quartiles (0.52, 0.80, and 1.27 mmol/L [20, 31, and 49 mg/dL]), p value for model = 0.0083. LDL-C=low-density lipoprotein cholesterol; HR=hazard ratio).

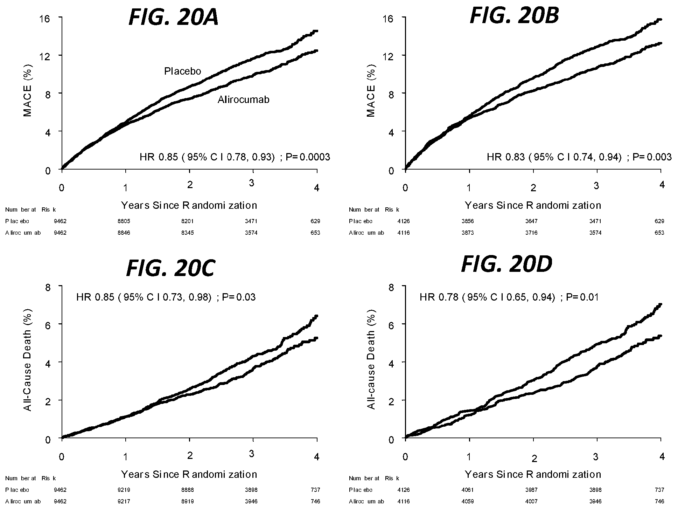

Fig. 20A - Fig. 20D are a set of graphs, with hazard ratios, of MACE **(****Fig. 20A** and **Fig.** 20B) and all-cause mortality **(****Fig. 20C** and **Fig. 20D****)** in the total population **(****Fig. 20A** and **Fig. 20C****)** and patients eligible for ≥ three years of follow up **(****Fig. 20B** and **Fig. 20D****).**

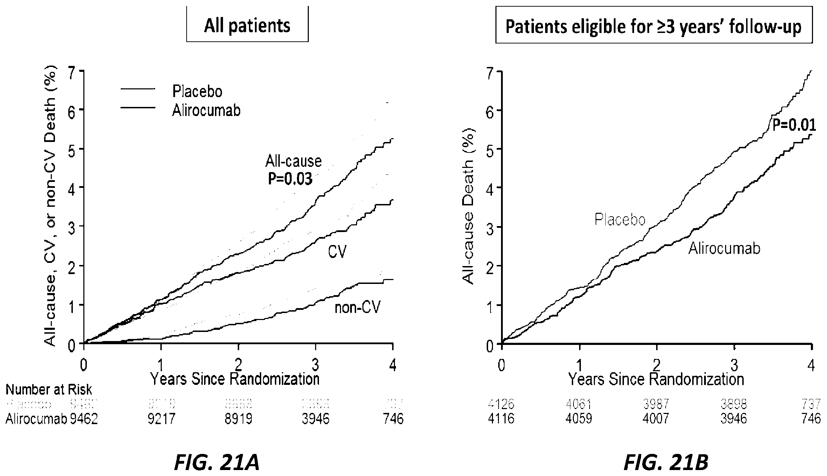

Fig. 21A - Fig 21B graphically depict death data. **Fig. 21A** is a graph showing all-cause death, cardiovascular (CV) death, and non-cardiovascular (non-CV) death in the total population of patients treated with alirocumab or placebo. **Fig. 21B** shows death reduction in patients eligible for ≥3 years follow up.

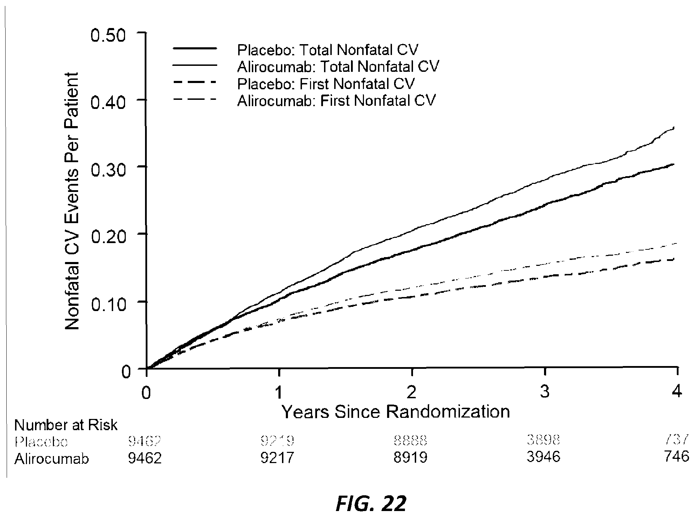

**Fig. 22** depicts mean cumulative functions and Kaplan-Meier curves for non-fatal cardiovascular (CV) events. Mean cumulative function curves depict the expected total number of non-fatal cardiovascular events for a given patient in the placebo and alirocumab groups at a given time after randomization.

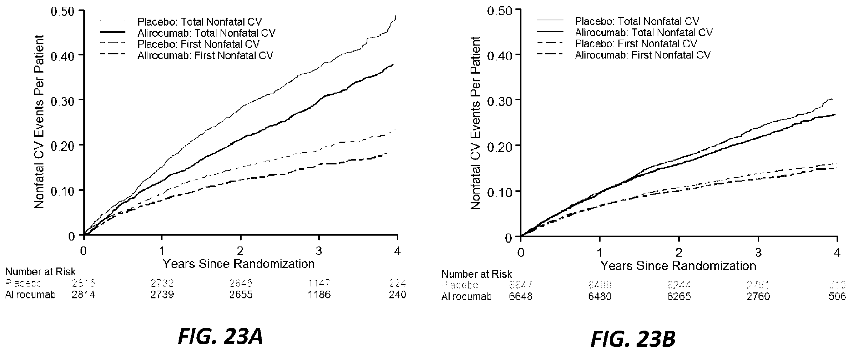

Fig. 23A - Fig. 23B show mean cumulative functions and Kaplan-Maier curves for non-fatal CV events in different patient groups. **Fig. 23A** illustrates non-fatal CV events among patients with baseline LDL-C ≥100 mg/dL (n=5,629). **Fig. 23B** illustrates non-fatal CV events among patients with baseline LDL-C <100 mg/dL (n=13,295).

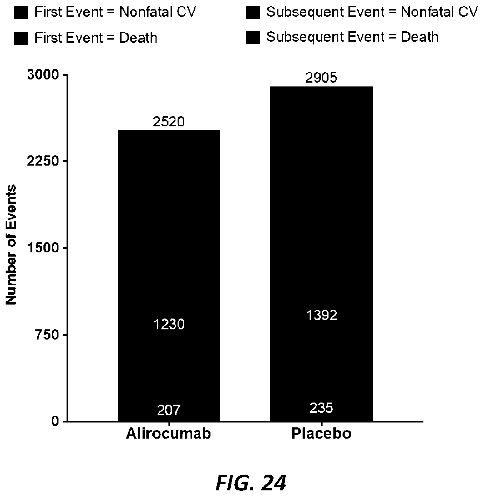

**Fig. 24** depicts first, subsequent, and total CV events and death by treatment group.

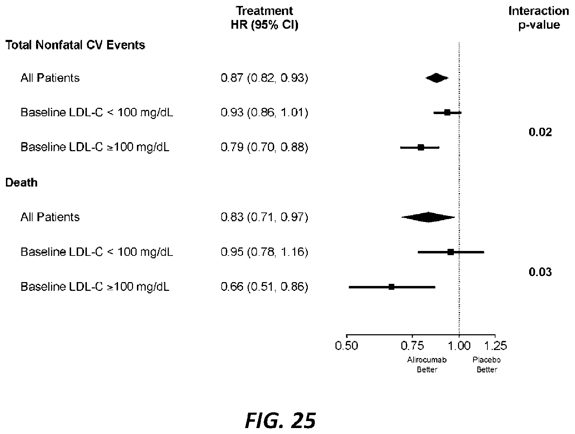

**Fig. 25** depicts a joint frailty model according to baseline LDL-C subgroups.

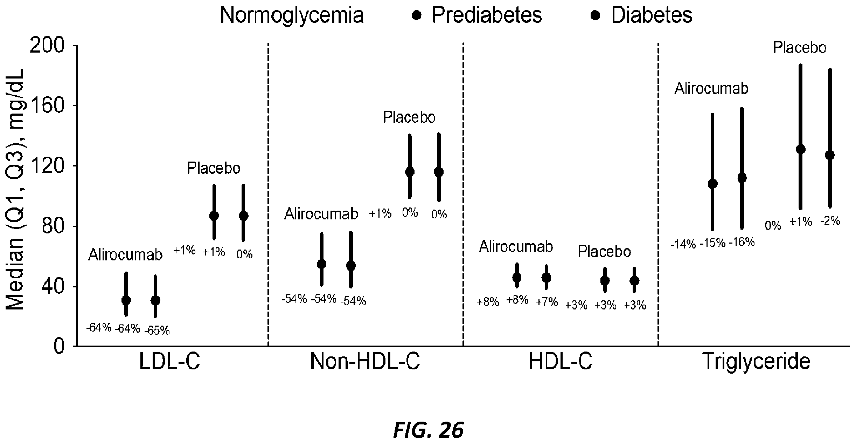

**Fig. 26** depicts a graph showing the lipid levels at 16 weeks after randomization (intention-to-treat analysis) in normoglycemic, pre-diabetic and diabetic patients. Median percent change from baseline is presented below each bar.

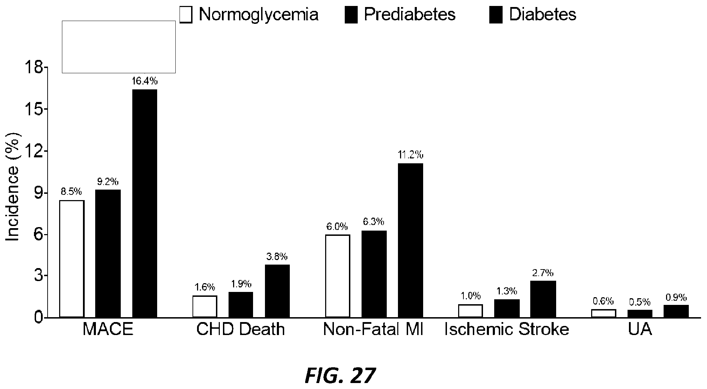

**Fig. 27** depicts a graph showing the incidence of cardiovascular events in normoglycemic, pre-diabetic, and diabetic patients, over a median (Q1, Q3) follow-up period of 2.8 (2.3, 3.4) years. *P<0.0001 for comparison of hazard in people with diabetes vs that in people with normoglycemia or pre-diabetes.

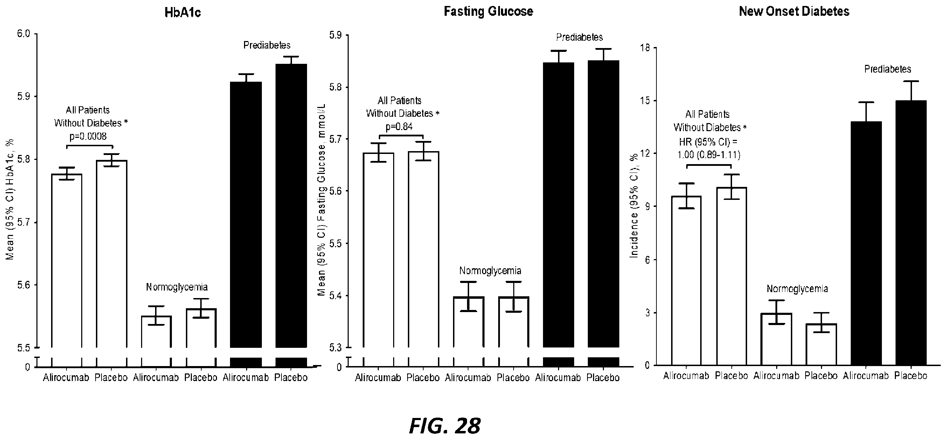

**Fig. 28** is a series of graphs showing post-randomization A1c, fasting glucose, and new-onset diabetes by baseline glucometabolic status, using a repeated-measures mixed effects model for A1c and fasting glucose. Only post-randomization values prior to initiation of diabetes medication were included in the analysis. *Without diabetes = pre-diabetes or normoglycemia.

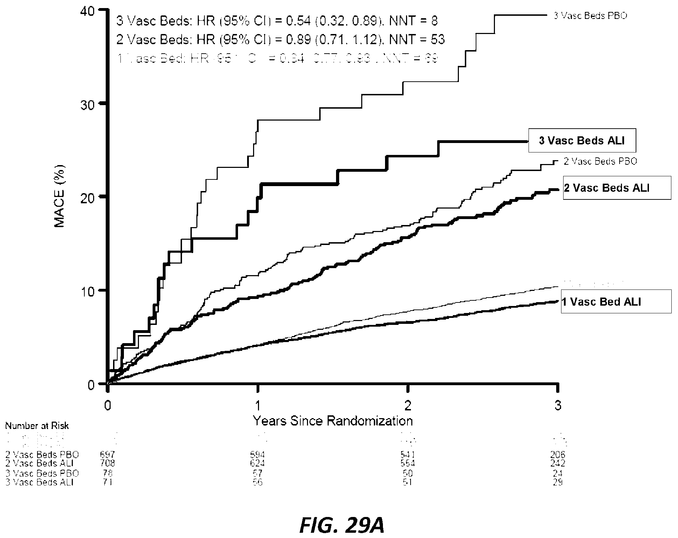

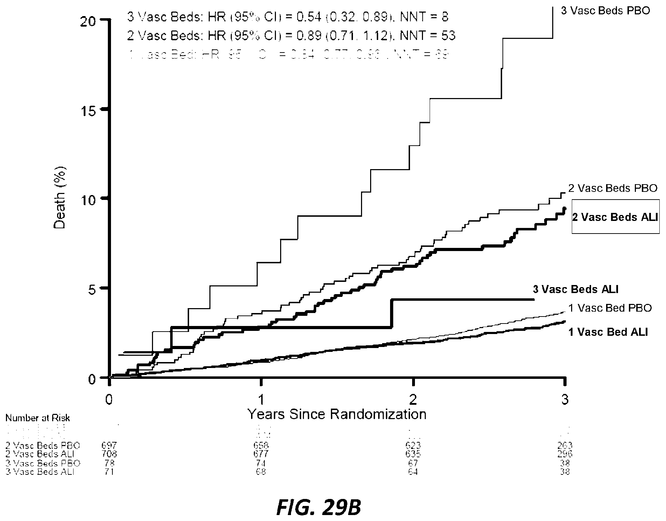

**Fig. 29A** **and** **Fig. 29B** show Kaplan-Meier curves for primary MACE endpoint **(****Fig. 29A****)** and all-cause death **(****Fig. 29B****)** in patients with arterial disease in 1 (coronary artery disease and no PAD or CeVD), 2 (coronary artery disease and PAD or CeVD), or 3 (coronary artery disease and PAD and CeVD) vascular beds following treatment with alirocumab (ALI; in bold line) or placebo (PBO). ARR indicates absolute risk reduction.

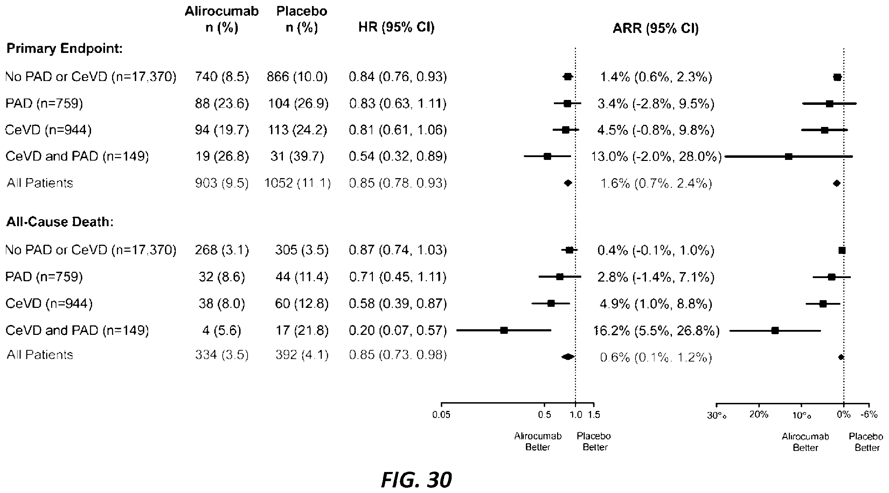

**Fig. 30** depicts hazard ratios of MACE and all-cause death and the corresponding absolute risk reduction (ARR) in patients with monovascular disease (coronary artery disease without known PAD or CeVD), polyvascular disease in two vascular beds (coronary artery disease and either PAD or CeVD), and polyvascular disease in three vascular beds (coronary artery disease with both PAD and CeVD).

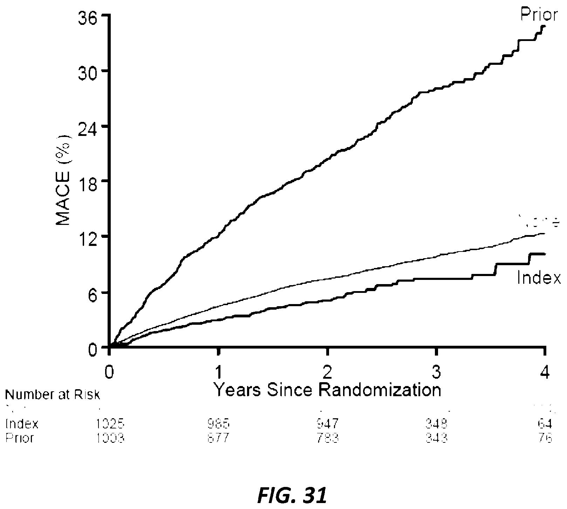

**Fig. 31** depicts Kaplan-Meier curves of occurrence of MACE according to prior coronary artery bypass grafting (CABG) status. Curves represent patients having: (1) no prior CABG ("None"); (2) CABG after the index ACS but before randomization ("Index"); and (3) CABG prior to the index ACS ("Prior").

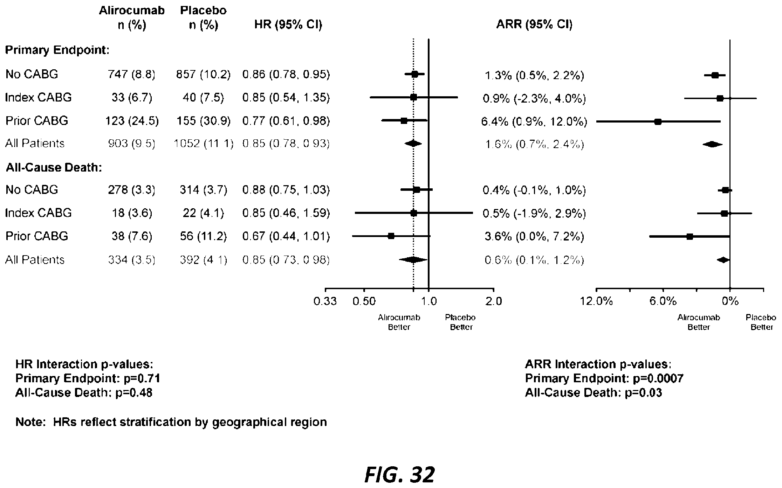

**Fig. 32** depicts hazard ratios of MACE (primary endpoint) and all-cause death and the corresponding absolute risk reduction (ARR) in patients according to prior coronary artery bypass grafting (CABG) status.

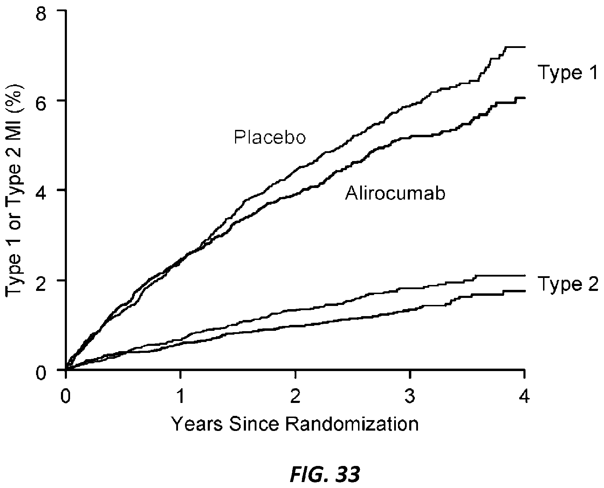

**Fig. 33** depicts Kaplan-Meier curves of occurrence of type 1 or type 2 myocardial infarctions (MI) and the effects of alirocumab over time versus placebo.

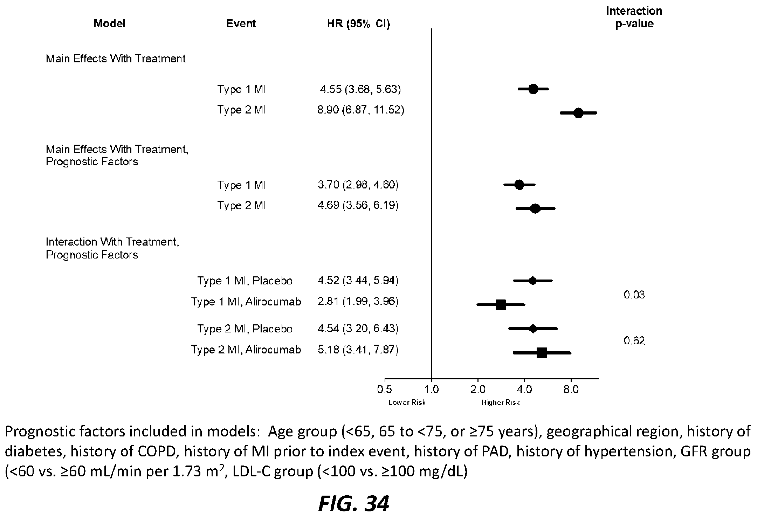

**Fig. 34** depicts a forest plot of type 1 or type 2 myocardial infarction (MI) during follow-up as predictors for all-cause death. Prognostic factors included in models: Age group (<65, 65 to <75, or ≥75 years), geographical region, history of diabetes, history of COPD, history of MI prior to index event, history of PAD, history of hypertension, GFR group (<60 vs. ≥60 mL/min per 1.73 m2, LDL-C group (<100 vs. ≥100 mg/dL).

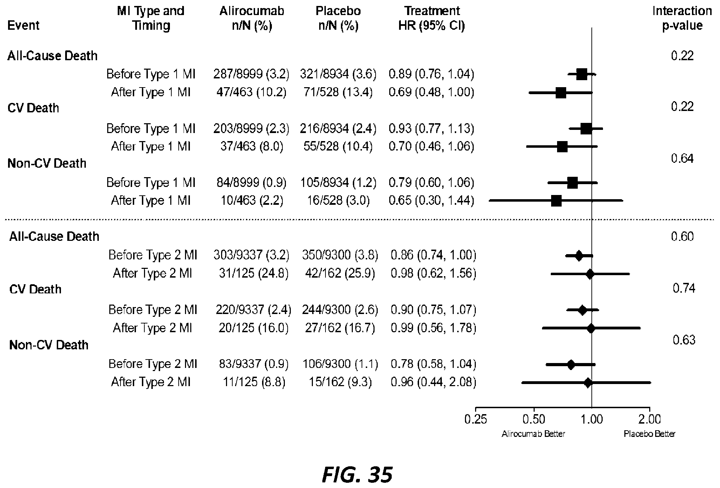

**Fig. 35** depicts hazard ratios of treatment effects on all-cause and cause-specific death before and after type 1 or type 2 myocardial infarction (MI).

### DETAILED DESCRIPTION

Before the present methods are described, it is to be understood that the invention is not limited to particular methods and experimental conditions described, as such methods and conditions may vary. It is also to be understood that the terminology used herein is for the purpose of describing particular embodiments only, and is not intended to be limiting, since the scope of the present methods will be limited only by the appended claims.

### Definitions

Unless defined otherwise, all technical and scientific terms used herein have the same meaning as commonly understood by one of ordinary skill in the art. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

As used herein, "high cardiovascular risk patient" refers to a patient with hypercholesterolemia and/or elevated levels of at least one atherogenic lipoprotein. In certain embodiments, a high cardiovascular risk patient has hypercholesterolemia and/or elevated levels of at least one atherogenic lipoprotein that are not adequately controlled by a lipid modifying therapy (LMT), including e.g., a maximally tolerated statin therapy. In certain embodiments, the high cardiovascular risk patient is a patient with clinical atherosclerotic cardiovascular disease (ASCVD). Patients with clinical ASCVD encompass patients with ACS, those with history of myocardial infarction, stable or unstable angina or coronary or other arterial revascularization, stroke, transient ischemic attack (TIA), or peripheral artery disease including aortic aneurysm, all of atherosclerotic origin.

As used herein, "acute coronary syndrome event" or "ACS event" refers to unstable symptoms of myocardial ischemia occurring at rest or minimal exertion within 72 hours of an unscheduled hospital admission, due to presumed or proven obstructive coronary disease. In certain embodiments, an ACS event requires at least one of the following criteria to be fulfilled: elevated cardiac biomarkers consistent with acute myocardial infarction, or resting ECG changes consistent with ischemia or infarction along with additional evidence of obstructive coronary disease from regional perfusion imaging or wall motion abnormalities, epicardial coronary stenosis ≧70% by angiography, or need for coronary revascularization related to the event.

As used herein, "major adverse cardiovascular events" or "MACE" refers to one or more of: death from coronary heart disease (CHD death), non-fatal myocardial infarction, unstable angina requiring hospitalization, and fatal or non-fatal ischemic stroke.

As used herein, "mortality," "all-cause mortality," and "all-cause death" are used interchangeably to refer to death caused by any reason.

As used herein, "coronary heart disease event" or "CHD event" refers to death from coronary heart disease, nonfatal myocardial infarction, unstable angina requiring hospitalization, or an ischemia-driven coronary revascularization procedure. The term "major coronary heart disease event" refers to death from coronary heart disease or nonfatal myocardial infarction.

The term "ischemia-driven coronary revascularization" refers to percutaneous coronary intervention (PCI) or coronary artery bypass graft (CABG). For the clinical studies disclosed herein, coronary revascularization procedures performed solely for restenosis at prior PCI site were excluded from this definition. In certain embodiments, ischemia-driven coronary revascularization must be driven by one of the following: a) acute ischemia, b) new or progressive symptoms (angina or equivalent), or 3) new or progressive functional testing abnormalities (e.g., stress testing or imaging).

As used herein, "coronary heart disease death," "CHD death," and "death due to coronary heart disease" are used interchangeably to refer to the subset of cardiovascular deaths for which there is a clear relationship to underlying coronary heart disease, including death secondary to acute myocardial infarction (MI), sudden death, heart failure, complication of a coronary revascularization procedure performed for symptoms, coronary disease progression, or new myocardial ischemia where the cause of death is clearly related to the procedure, unobserved and unexpected death, and other death that cannot definitely be attributed to a nonvascular cause.

As used herein, the terms "cardiovascular event" or "CV event" refer to any non-fatal coronary heart disease event, any cardiovascular death, and any non-fatal ischemic stroke. Exemplary CV events include, but are not limited to, myocardial infarction, stroke, unstable angina requiring hospitalization, heart failure requiring hospitalization, and an ischemia-driven coronary revascularization procedure.

As used herein, the terms "cardiovascular death", "CV death", and "cardiovascular mortality" are used interchangeably to refer to death resulting from an acute myocardial infarction, sudden cardiac death, death due to heart failure, death due to stroke, and death due to other cardiovascular causes. In certain embodiments, the CV death is CHD death. In other embodiments the CV death is selected from the group consisting of heart failure or cardiogenic shock, stroke, ischemic cardiovascular causes, or a cardiovascular cause other than ischemia.

As used herein, the term "non-fatal cardiovascular event" refers to any CV event that does not result in death. In certain embodiments, non-fatal CV events may occur consecutively in time wherein an initial (e.g., first) CV event is followed by a subsequent (e.g., second, third or fourth) event.

As used herein, "non-cardiovascular death" and "non-CV death" are used interchangeably to refer to any death that is not thought to be a cardiovascular death. Examples of non-cardiovascular death include but are not limited to pulmonary infection, pulmonary malignancy, gastrointestinal/hepatobiliary/pancreatic infection, gastrointestinal/hepatobiliary/pancreatic malignancy, hemorrhage, neurological process that is not a stroke/hemorrhage, suicide, a non-cardiovascular procedure or surgery, accident or trauma, renal infection, renal malignancy other non-cardiovascular infection, and other non-cardiovascular malignancy.

As used herein, "non-fatal myocardial infarction" is defined and sub-classified in accordance with ACC/AHA/ESC Universal Definition of Myocardial Infarction (see Thygesen et al., 2012. Third universal definition of myocardial infarction. Journal of the American College of Cardiology. 60(16): 1581-98). For the clinical studies disclosed herein, silent myocardial infarction was not considered part of the primary end point.

As used herein, "type 1 myocardial infarction" refers to spontaneous myocardial infarction related to an event related to atherosclerotic plaque rupture, ulceration, fissuring, erosion, or dissection with resulting intraluminal thrombus in one or more of the coronary arteries, leading to decreased myocardial blood flow or distal platelet emboli with ensuing myocyte necrosis (see Thygesen et al., 2012. JACC 60(16): 1581-98). In some embodiments, a type 1 myocardial infarction is defined as a detection of a rise and/or fall of cardiac troponin (cTn) with at least one value above the 99th percentile upper reference limit (URL) and with at least one of the following: symptoms of acute myocardial ischemia, new ischemic ECG changes, development of pathological Q waves, imaging evidence of new loss of viable myocardium or new regional wall motion abnormality in a pattern consistent with an ischemic etiology, or identification of a coronary thrombus by angiography including intracoronary imaging or by autopsy.

As used herein, "type 2 myocardial infarction" refers to instances of myocardial injury with necrosis, where a condition other than coronary artery disease contributes to an imbalance between myocardial oxygen supply and/or demand (see Thygesen et al., 2012. JACC 60(16): 1581-98). In some embodiments, a type 2 myocardial infarction is defined as a detection of a rise and/or fall of cTn with at least one value above the 99th percentile URL, and evidence of an imbalance between myocardial oxygen supply and demand unrelated to coronary thrombosis, requiring at least one of the following: symptoms of acute myocardial ischemia, new ischemic ECG changes, development of pathological Q waves, or imaging evidence of new loss of viable myocardium, or new regional wall motion abnormality in a pattern consistent with an ischemic etiology.

As used herein, "type 3 myocardial infarction" refers to Patients who suffer cardiac death, with symptoms suggestive of myocardial ischemia accompanied by presumed new ischemic electrocardiogram (ECG) changes or new left bundle branch block-but without available biomarker values (see Thygesen et al., 2012. JACC 60(16): 1581-98).

As used herein, "type 4a myocardial infarction" refers to myocardial infarction associated with percutaneous coronary intervention. As used herein, "Type 4b myocardial infarction" refers to myocardial infarction associated with stent thrombosis (see Thygesen et al., 2012. JACC 60(16): 1581-98).

As used herein, "type 5 myocardial infarction" refers to myocardial infarction associated with coronary artery bypass grafting (CABG) (see Thygesen et al., 2012. JACC 60(16): 1581-98).

As used herein, "coronary artery bypass grafting (CABG)" refers to a procedure in which autologous arteries or veins are used as grafts to bypass coronary arteries that are partially or completely obstructed by atherosclerotic plaques (see Alexander & Smith, 2016, Coronary-Artery Bypass Grafting. NEJM 374:1954-64).

As used herein, the terms "unstable angina requiring hospitalization" and "hospitalization for unstable angina" are used interchangeably to refer to: admission to hospital or emergency department with symptoms of myocardial ischemia with an accelerating tempo in the prior 48 hours and/or rest chest discomfort ≧20 min, requiring in addition both of the following: a) new or presumed new ischemic ECG changes, defined by ST depression >0.5 mm in 2 contiguous leads; T-wave inversion >1 mm in 2 contiguous leads with prominent R-wave or R/S>1; ST elevation in >2 contiguous leads >0.2 mV in V2 or V3 in men, >0.15 mV in V2 or V3 in women, or >0.1 mV in other leads; or LBBB; and b) definite contemporary evidence of coronary obstruction by need for coronary revascularization procedure or at least one epicardial stenosis ≧70%. For the clinical trials disclosed herein, coronary revascularization procedures or stenoses due only to restenosis at prior PCI site were excluded.

As used herein, "ischemic stroke" refers to: 1) an acute episode of focal cerebral, spinal, or retinal dysfunction caused by infarction, defined by at least one of the following: a) pathological, imaging, or other objective evidence of acute, focal cerebral, spinal, or retinal ischemic injury in a defined vascular distribution; or b) symptoms of acute cerebral, spinal, or retinal ischemic injury persisting 24 hours or until death, with other etiologies excluded; 2) hemorrhagic infarction, but not stroke caused by intracerebral or subarachnoid hemorrhage; or 3) strokes not otherwise sub-classified.

As used herein, "diabetes" and "diabetic" refer to a group of metabolic diseases in which a person has high blood sugar levels, either because the body does not produce enough insulin, or because cells do not respond to the insulin that is produced. The most common types of diabetes are: (1) type 1 diabetes, where the body fails to produce insulin; (2) type 2 diabetes, where the body fails to use insulin properly, combined with an increase in insulin deficiency over time; and (3) gestational diabetes, where women develop diabetes due to their pregnancy. In certain embodiments, diabetes is defined as a medical history of diabetes type 1 or 2, hemoglobin A1c level ≥6.5%, two values of fasting serum or plasma glucose (FPG) ≥126 mg/dL, or use of diabetes medication. In certain embodiments, diabetes is diagnosed according to the standards recommended by the American Diabetes Association. (see Standards of Medical Care in Diabetes - 2012. Diabetes Care. Jan 2012; 35 Suppl 1:S11-63). In a specific embodiment, diabetes is diagnosed as having a FPG greater than or equal to 126 mg/dL; a two-hour value in the 75-gram oral glucose tolerance test (OGTT) greater than or equal to 200 mg/dL; a hemoglobin A1c level greater than or equal to 6.5%; or a random plasma glucose level greater than or equal to 200 mg/dL in a patient with classic symptoms of hyperglycemia or hyperglycemic crisis. As used herein, "pre-diabetes" refers to A1c ≥5.7% and <6.5% at baseline, or two FPG values >100 mg/dL, but no more than one ≥126 mg/dL. As used herein, "normoglycemia" refers to none of these listed parameters of diabetes or pre-diabetes. As used herein, "new-onset diabetes" refers to any of the following: at least two values of A1c ≥6.5%, at least two FPG measurements ≥126 mg/dL, investigator-reported diabetes-related adverse event, or the initiation of diabetes medication.

As used herein "peripheral artery disease" or "PAD" encompasses the vascular diseases caused primarily by atherosclerosis and thromboembolic pathophysiological processes that alter the normal structure and function of the aorta, its visceral arterial branches, and the arteries of the lower extremity. PAD refers to denote stenotic, occlusive, and aneurysmal diseases of the aorta and its branch arteries, exclusive of the coronary arteries (see Hirsch et al, 2006, ACC/AHA 2005 practice guidelines for the management of patients with peripheral artery disease, Circulation. 113: 1475-1547).

As used herein "cerebrovascular disease" or "CeVD" refers to a history of all disorders in which an area of the brain is temporarily or permanently affected by ischemia or bleeding and one or more of the cerebral blood vessels are involved in the pathological process. CeVD includes but is not limited to stroke, carotid stenosis, vertebral stenosis and intracranial stenosis, aneurysms, and vascular malformations (see Advisory Council for the National Institute of Neurological and Communicative Disorders and Stroke, 1975, A Classification and Outline of Cerebrovascular Diseases II, Stroke. 6: 564-616.).

As used herein "polyvascular disease", "PoVD", or "PVD" refers to pre-existent disease in multiple arterial territories, or two or more vascular territories (beds) with atherosclerotic involvement (Bhatt et al., 2009. Eur. Heart J. 30: 1195-1202). In certain embodiments, patients with polyvascular disease have coronary artery disease with concurrent peripheral artery disease (PAD), cerebrovascular disease (CeVD), or both.

As used herein, "high intensity statin therapy" and "high-dose atorvastatin/rosuvastatin" are used interchangeably to refer to administration of 40-80 mg of atorvastatin daily, or 20-40 mg of rosuvastatin daily.

As used herein, "maximally tolerated statin therapy" or "maximum tolerated dose of statin therapy" are used interchangeably to mean a therapeutic regimen comprising the administration of a daily dose of a statin that is the highest dose of statin that can be administered to a particular patient without causing unacceptable adverse side effects in the patient. Maximally tolerated statin therapy includes, but is not limited to, high intensity statin therapy.

As used herein, a patient is regarded as "statin intolerant" or "intolerant to statins" if the patient has a history of experiencing one or more adverse reactions that began or increased while on a daily statin therapeutic regimen and stopped when statin therapy was discontinued. In certain embodiments, the adverse reactions are musculoskeletal in nature, such as skeletal muscle pain, aches, weakness or cramping (e.g., myalgia, myopathy, rhabdomyolysis, etc.). Such adverse reactions are often intensified following exercise or exertion. Statin-related adverse reactions also include hepatic, gastrointestinal and psychiatric symptoms that correlate with statin administration. According to certain embodiments, a patient is deemed "statin intolerant" or "intolerant to statins" if, for example, any of the following applies to the patient: (1) has a history of skeletal muscle-related symptoms associated with at least two different and separate daily statin therapeutic regimens; (2) exhibits one or more statin-related adverse reaction(s) to the lowest approved daily doses of one or more statins; (3) unable to tolerate a cumulative weekly statin dose of seven times the lowest approved tablet size; (4) able to tolerate a low dose statin therapy but develops symptoms when the dose is increased (e.g., to achieve a targeted LDL-C level); or (5) statins are contraindicated for the patient.

As used herein, "not adequately controlled", in reference to hypercholesterolemia, means that the patient's serum low-density lipoprotein cholesterol (LDL-C) concentration, total cholesterol concentration, and/or triglyceride concentration is not reduced to a recognized, medically-acceptable level (taking into account the patient's relative risk of coronary heart disease) after at least 4 weeks on a therapeutic regimen comprising a stable daily dose of a statin. For example, a patient with hypercholesterolemia that is not adequately controlled by a statin includes a patient or patients with a serum LDL-C concentration of greater than or equal to about 70 mg/dL, 80 mg/dL, 90 mg/dL, 100 mg/dL, 110 mg/dL, 120 mg/dL, 130 mg/dL, 140 mg/dL, or more (depending on the patient's underlying risk of heart disease) after the patient has been on a stable daily statin regimen for at least 4 weeks.

As used herein, the expression "not adequately controlled", in reference to atherogenic lipoproteins, means that the patient's serum low-density lipoprotein cholesterol (LDL-C) concentration, non-high-density lipoprotein cholesterol, and/or apolipoprotein B concentration are not reduced to a recognized, medically-acceptable level (taking into account the patient's relative risk of coronary heart disease) after at least 4 weeks on a therapeutic regimen comprising a stable daily dose of a statin. For example, a patient with elevated levels of atherogenic lipoproteins that are not adequately controlled by a statin includes a patient or patients with a serum LDL-C concentration of greater than or equal to about 70 mg/dL, 80 mg/dL, 90 mg/dL, 100 mg/dL, 110 mg/dL, 120 mg/dL, 130 mg/dL, 140 mg/dL, or more (depending on the patient's underlying risk of heart disease); a non-high-density lipoprotein cholesterol concentration of greater than or equal to about 100 mg/dL; or an apolipoprotein B concentration of greater than or equal to about 80 mg/dL after the patient has been on a stable daily statin regimen for at least 4 weeks.

### Hypercholesterolemia And Other Atherogenic Lipoproteins Not Adequately Controlled by Maximum Tolerated Dose Statin Therapy

The present invention relates generally to the treatment of high cardiovascular risk patients who have hypercholesterolemia that is not adequately controlled by statins, i.e., hypercholesterolemia not adequately controlled by a therapeutic regimen comprising a daily maximum tolerated dose of a statin.

According to the invention, the high cardiovascular risk patient who receives the PCSK9 antibody or antigen-binding fragment thereof in accordance with the present invention has hypercholesterolemia that is inadequately controlled by a maximum tolerated dose statin therapy.

In certain embodiments, the high cardiovascular risk patient's elevated levels of atherogenic lipoproteins are inadequately controlled by a maximum tolerated dose of statin therapy.

Maximum tolerated dose of statin therapy includes daily administration of statins such as cerivastatin, pitavastatin, fluvastatin, lovastatin, and pravastatin.

### Patient Selection

The present invention provides a PCSK9 antibody or antigen-binding fragment thereof for use in a method for reducing the risk of major adverse cardiovascular events in accordance with claim 1.

In certain embodiments of the present invention, the antibody or antigen-binding fragment thereof is administered as an adjunct to diet.

In some embodiments, the antibody or antigen-binding fragment thereof is administered for the reduction of the risk of MACE in patients with recent acute coronary syndrome (ACS) (e.g., ACS event within the past 1, 2, 3, 4, 5, 6, 7, 8, 9, 10, 11, or 12 months). In certain embodiments, the ACS event is unstable angina.

In some embodiments, the antibody or antigen-binding fragment thereof is used either in combination with a statin, or as monotherapy, including in patients who are statin intolerant.

The high cardiovascular risk patients who are treatable by the methods of the present invention include those patients hospitalized for ACS.

In certain embodiments, the high cardiovascular risk patient may be selected on the basis of demonstrated inadequate control of atherogenic lipoproteins despite steady state (at least 2 weeks) treatment with atorvastatin 40-80 mg daily, or rosuvastatin 20-40 mg daily. In some embodiments, the patient has an LDL-C level greater than or equal to 110, 120, 130 or 140 mg/dL despite treatment with maximally tolerated statin therapy. In some embodiments, the patient has a non-HDL-C level greater than or equal to 100 mg/dL despite treatment with a high intensity or maximally tolerated statin therapy. In some embodiments, the patient has an apolipoprotein B level greater than or equal to 80 mg/dL despite treatment with a high intensity or maximally tolerated statin therapy. In some embodiments, the patient has an Lp(a) level greater than or equal to 15, 16, 17, 18, 19, 20, 21, 22, 23, 24, 25, 30, 35, 40, 45, 50, 55 or 60 mg/dL, as measured by an automated latex-enhanced nephelometric assay, despite treatment with a high intensity or maximally tolerated statin therapy. In one embodiment, the patient has an Lp(a) level greater than or equal to 21.2 mg/dL as measured by an automated latex-enhanced nephelometric assay despite treatment with a high intensity or maximally tolerated statin therapy.

According to certain embodiments, the high cardiovascular risk patient may be selected on the basis of having one or more additional risk factors selected from the group consisting of age (e.g., older than 40, 45, 50, 55, 60, 65, 70, 75, or 80 years), race, national origin, gender (male or female), exercise habits (e.g., regular exerciser, non-exerciser), other preexisting medical conditions (e.g., diabetes, high blood pressure, etc.), and current medication status (e.g., currently taking beta blockers, niacin, ezetimibe, fibrates, omega-3 fatty acids, bile acid resins, etc.). In certain embodiments, the high cardiovascular risk patient is selected based on preexisting diabetes (e.g., type I diabetes or type II diabetes), either alone or in combination with the foregoing selection criteria based on recent ACS and/or inadequate control of atherogenic lipoproteins.

In certain embodiments, the high cardiovascular risk patient has clinical atherosclerotic cardiovascular disease (ASCVD). Clinical ASCVD encompasses ACS, those with history of MI, stable or unstable angina or coronary or other arterial revascularization, stroke, TIA or PAD including aortic aneurysm, all of atherosclerotic origin.

According to the present invention, high cardiovascular risk patients may be selected on the basis of a combination of one or more of the foregoing selection criteria or therapeutic characteristics. The high cardiovascular risk or very high cardiovascular risk patient may also be selected on the basis of clinical guidelines, including for example the American Heart Association (AHA) and American College of Cardiology (ACC) Clinical Practice Guidelines. *See, e.g.,* Stone et al. 2013 ACC/AHA Guideline on the Treatment of Blood Cholesterol to Reduce Atherosclerotic Cardiovascular Risk in Adults: A Report of the American College of Cardiology/American Heart Association Task Force on Practice Guidelines. Circulation (2014) 129:S1-45. Grundy SM, et al. 2018 AHA/ACC/AACVPR/AAPA/ABC/ACPM/ADA/AGS/APhA/ASPC/NLA/PCNA Guideline on the Management of Blood Cholesterol, Journal of the American College of Cardiology (2018) (in press - available online November 10, 2018).

### Administration of a PCSK9 Inhibitor as Add-On Therapy to Maximum Tolerated Dose Statin Therapy

The present invention provides the PCSK9 antibody or antigen-binding fragment thereof for use in a method for reducing the risk of major adverse cardiovascular events in accordance with claim 1. In some embodiments, the PCSK9 antibody or antigen-binding fragment thereof can be administered as an add-on to the patient's pre-existing lipid modifying treatment (LMT), (if applicable), such as an add-on to the patient's pre-existing daily therapeutic statin regimen.

For example, the administration may include add-on therapeutic regimens wherein the PCSK9 inhibitor is administered as add-on therapy to the same stable daily therapeutic statin regimen (i.e., same dosing amount of statin) that the patient was on prior to receiving the PCSK9 inhibitor. In other embodiments, the PCSK9 antibody or antigen-binding fragment thereof is administered as add-on therapy to a therapeutic statin regimen comprising a statin in an amount that is more than or less than the dose of statin the patient was on prior to receiving the PCSK9 inhibitor. For example, after starting a therapeutic regimen comprising a PCSK9 antibody or antigen-binding fragment thereof administered at a particular dosing frequency and amount, the daily dose of statin administered or prescribed to the patient may (a) stay the same, (b) increase, or (c) decrease (e.g., up-titrate or down-titrate) in comparison to the daily statin dose the patient was taking before starting the PCSK9 antibody or antigen-binding fragment thereof therapeutic regimen, depending on the therapeutic needs of the patient.

### Therapeutic Efficacy

The present invention results in the reduction in serum levels of one or more lipid components selected from the group consisting of LDL-C, ApoB100, non-HDL-C, total cholesterol, VLDL-C, triglycerides, Lp(a), and remnant cholesterol. For example, according to certain embodiments of the present invention, administration of a pharmaceutical composition comprising the PCSK9 antibody or antigen-binding fragment thereof to a high cardiovascular risk patient with hypercholesterolemia or elevated levels of other atherogenic lipoproteins that are not adequately controlled by a stable daily maximum tolerated dose of statin therapy, (e.g., administration of the PCSK9 inhibitor on top of the high cardiovascular risk patient's maximum tolerated dose of statin therapy) will result in a mean percent reduction from baseline in serum low density lipoprotein cholesterol (LDL-C) of at least about 25%, about 30%, about 40%, about 50%, about 60%, or greater; a mean percent reduction from baseline in ApoB100 of at least about 25%, about 30%, about 40%, about 50%, about 60%, or greater; a mean percent reduction from baseline in non-HDL-C of at least about 25%, about 30%, about 40%, about 50%, about 60%, or greater; a mean percent reduction from baseline in total cholesterol of at least about 10%, about 15%, about 20%, about 25%, about 30%, about 35%, or greater; a mean percent reduction from baseline in VLDL-C of at least about 5%, about 10%, about 15%, about 20%, about 25%, about 30%, or greater; a mean percent reduction from baseline in triglycerides of at least about 5%, about 10%, about 15%, about 20%, about 25%, about 30%, about 35% or greater; and/or a mean percent reduction from baseline in Lp(a) of at least about 5%, about 10%, about 15%, about 20%, about 25%, or greater (e.g., as measured by an automated latex-enhanced nephelometric assay).

Exemplary types of cardiovascular risks include but are not limited to: the risk or rate of occurrence of coronary heart disease (CHD) death, non-fatal myocardial infarction, ischemic stroke (fatal or non-fatal), or unstable angina requiring hospitalization; the risk of any CHD event; the risk of major CHD event; the risk of any cardiovascular event; the risk of mortality (i.e., all-cause death), non-fatal myocardial infarction, or non-fatal ischemic stroke; the risk of CHD death; the risk of cardiovascular death; the risk of mortality (i.e., all-cause death); the risk of non-fatal myocardial infarction; the risk of fatal or non-fatal ischemic stroke; the risk of unstable angina requiring hospitalization; the risk of ischemia-driven coronary revascularization procedure; the risk of congestive heart failure requiring hospitalization; the risk of non-fatal cardiovascular event, and the risk of non-fatal myocardial infarction, stroke, or unstable angina.

Administration of the PCSK9 antibody or antigen-binding fragment thereof may reduce the risk of mortality of a high cardiovascular risk patient. In certain embodiments, the patient's risk of mortality is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In one embodiment, the patient's risk of mortality is reduced by about 29%. In certain embodiments, the survival time of the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75%, or about 80%.

Administration of the PCSK9 antibody or antigen-binding fragment thereof to a high cardiovascular risk patient who suffers from additional diseases may reduce the risk of mortality of the patient. In some embodiments, the patient has peripheral artery disease (PAD), and the patient's risk of mortality is reduced by about 29% in patients with PAD. In certain embodiments, the absolute risk of mortality is reduced by about 2.8% in patients with PAD. In other embodiments, the high cardiovascular risk patient has cerebrovascular disease (CeVD). In certain embodiments, the risk of mortality is reduced by about 42% in patients with CeVD. In certain embodiments, the absolute risk of mortality is reduced by about 4.9% in patients with CeVD. In certain embodiments, the risk of mortality is reduced by about 80% in patients with both PAD and CeVD who are administered the disclosed PCSK9 inhibitor. In certain embodiments, the absolute risk is reduced by about 16.2% in patients with both PAD and CeVD. In some embodiments, the patient has had a prior coronary artery bypass graft (CABG). In certain embodiments, the high cardiovascular risk patient has had an initial acute coronary syndrome (ACS) event prior to the CABG. In certain embodiments the risk of mortality is reduced by about 15% in patients having an initial ACS event prior to CABG. In certain embodiments, the absolute risk of mortality is reduced by about 0.5% in patients having an initial ACS event prior to CABG. In certain embodiments, the high cardiovascular risk patient has had an ACS event following the CABG. In certain embodiments, the risk of mortality is reduced by about 33% in patients having an ACS event following the CABG. In certain embodiments, the absolute risk of mortality is reduced by about 3.6% in patients having an ACS event following the CABG.

Administration of the PCSK9 antibody or antigen-binding fragment thereof to a high cardiovascular risk patient according to the present invention reduces the risk of MACE in the patient. In certain embodiments, ischemic stroke comprises fatal ischemic stroke and non-fatal ischemic stroke. In certain embodiments, the risk of MACE is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In one embodiment, the patient has an LDL-C level greater than or equal to 70 mg/dL despite treatment with a maximum tolerated dose of statin therapy, and the risk of MACE is reduced by about 15%. In another embodiment, the patient has an LDL-C level greater than or equal to 100 mg/dL despite treatment with a maximum tolerated dose of statin therapy, and the risk of MACE is reduced by about 24%. In another embodiment, the patient is not currently receiving statin therapy and the risk of MACE is reduced by about 35%. In another embodiment, the patient has diabetes, and the risk of MACE is reduced by about 16% and/or the absolute risk is reduced by about 2.3%. In one embodiment, the patient has an Lp(a) level greater than or equal to 21.2 mg/dL as measured by an automated latex-enhanced nephelometric assay despite treatment with a high intensity or a maximum tolerated dose of statin therapy. In certain embodiments, the time to first occurrence of CHD death, non-fatal myocardial infarction, unstable angina requiring hospitalization, or ischemic stroke in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

In some embodiments, the patient has peripheral artery disease (PAD), and the risk of MACE is reduced by about 17% in patients with PAD. In certain embodiments, the absolute risk of MACE is reduced by about 3.4% in patients with PAD. In some embodiments, the patient has cerebrovascular disease (CeVD), and the risk of MACE is reduced by about 19% in patients with CeVD. In certain embodiments, the absolute risk of MACE is reduced by about 4.5% in patients with CeVD. In certain embodiments, the risk of MACE is reduced by about 46% in patients with both PAD and CeVD. In certain embodiments, the absolute risk of MACE is reduced by about 13% in patients with both PAD and CeVD. In some embodiments, the patient has had a prior coronary artery bypass graft (CABG). In certain embodiments, the high cardiovascular risk patient has had an initial acute coronary syndrome (ACS) event prior to the CABG. In certain embodiments, the risk of MACE is reduced by about 15% in patients having an initial ACS event prior to CABG. In certain embodiments, the absolute risk of MACE is reduced by about 0.9% in patients having an initial ACS event prior to CABG. In certain embodiments, the high cardiovascular risk patient has had an ACS event following the CABG. In certain embodiments, the risk of MACE is reduced by about 23% in patients having an ACS event following the CABG. In certain embodiments, the absolute risk of MACE is reduced by about 6.4% in patients having an ACS event following the CABG.

Administration of the PCSK9 antibody or antigen-binding fragment thereof may reduce the risk of ischemic stroke (fatal or non-fatal) of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In one embodiment, the risk is reduced by about 40%. In certain embodiments, the time to first occurrence of ischemic stroke in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the ischemic stroke is fatal. In certain embodiments, the ischemic stroke is non-fatal.

Administration of the PCSK9 antibody or antigen-binding fragment thereof may reduce the risk of unstable angina requiring hospitalization of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In one embodiment, the risk is reduced by about 52%. In certain embodiments, the time to first occurrence of unstable angina requiring hospitalization in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of CHD death of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In another embodiment, the risk is reduced by about 28%. In certain embodiments, the time to first occurrence of CHD death in the patient is increased e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of cardiovascular death of a high cardiovascular risk patient. As used herein, "cardiovascular death," "CV death," and "cardiovascular mortality" are used interchangeably. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In another embodiment, risk is reduced by about 31%. In certain embodiments, the time to first occurrence of cardiovascular death in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of CHD event of a high cardiovascular risk patient. In certain embodiments, the CHD event is defined as CHD death, non-fatal myocardial infarction, unstable angina requiring hospitalization, or ischemia-driven coronary revascularization procedure. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the time to first occurrence of CHD event in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of major CHD event of a high cardiovascular risk patient. In certain embodiments, the major CHD event is defined as CHD death and non-fatal myocardial infarction. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the time to first occurrence of major CHD event in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of cardiovascular event of a high cardiovascular risk patient. In certain embodiments, the cardiovascular event is defined as non-fatal CHD event, cardiovascular death, and non-fatal ischemic stroke. In certain embodiments, the cardiovascular event is defined as non-fatal myocardial infarction, unstable angina requiring hospitalization, ischemia-driven coronary revascularization procedure, cardiovascular death, and non-fatal ischemic stroke. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the time to first occurrence of cardiovascular event in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of mortality, non-fatal myocardial infarction, or non-fatal ischemic stroke of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the time to first occurrence of all-cause death, non-fatal myocardial infarction, or non-fatal ischemic stroke in the patient is increased, e.g., about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of non-fatal myocardial infarction of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In one embodiment, the risk is reduced by about 21%. In certain embodiments, the patient has an Lp(a) level greater than or equal to 15, 16, 17, 18, 19, 20, 21, 22, 23, 24, 25, 30, 35, 40, 45, 50, 55, or 60 mg/dL, as measured by an automated latex-enhanced nephelometric assay, despite treatment with a high intensity or a maximally tolerated statin therapy. In one embodiment, the patient has an Lp(a) level greater than or equal to 21.2 mg/dL as measured by an automated latex-enhanced nephelometric assay despite treatment with a high intensity or a maximally tolerated statin therapy. In certain embodiments, the time to first occurrence of non-fatal myocardial infarction in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the rate of occurrence of ischemia-driven coronary revascularization procedure of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the time to first occurrence of ischemia-driven coronary revascularization procedure in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of congestive heart failure requiring hospitalization of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the time to first occurrence of congestive heart failure requiring hospitalization in the patient is increased, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of non-fatal cardiovascular event of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the rate of occurrence of total non-fatal cardiovascular events in the patient is reduced, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of non-fatal myocardial infarction, stroke, or unstable angina of a high cardiovascular risk patient. In certain embodiments, the risk is reduced by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the rate of occurrence of total non-fatal myocardial infarction, stroke, or unstable angina in the patient is reduced, e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%.

Administration of the PCSK9 antibody or antigen-binding fragment thereof may reduce the risk of type 1 or type 2 myocardial infarction in a high cardiovascular risk patient. In certain embodiments, the risk of type 1 myocardial infarction is reduced by e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the risk of type 1 myocardial infarction is reduced by about 13%. In certain embodiments, the risk of type 2 myocardial infarction is reduced by e.g., by about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 65%, about 70%, about 75% or about 80%. In certain embodiments, the risk of type 2 myocardial infarction is reduced by about 23%.

### PCSK9 Inhibitors

The invention comprises administering to the patient a therapeutic composition comprising the PCSK9 antibody or antigen-binding fragment thereof. As used herein, a "PCSK9 inhibitor" is any agent which binds to or interacts with human PCSK9 and inhibits the normal biological function of PCSK9 *in vitro* or *in vivo.* Non-limiting examples of categories of PCSK9 inhibitors include small molecule PCSK9 antagonists, nucleic acid-based inhibitors of PCSK9 expression or activity (e.g., siRNA or antisense), peptide-based molecules that specifically interact with PCSK9 (e.g., peptibodies), receptor molecules that specifically interact with PCSK9, proteins comprising a ligand-binding portion of an LDL receptor, PCSK9-binding scaffold molecules (e.g., DARPins, HEAT repeat proteins, ARM repeat proteins, tetratricopeptide repeat proteins, fibronectin-based scaffold constructs, and other scaffolds based on naturally occurring repeat proteins, etc., (see, e.g., Boersma and Pluckthun, 2011, Curr. Opin. Biotechnol. 22:849-857, and references cited therein), and anti-PCSK9 aptamers or portions thereof. According to certain embodiments, PCSK9 inhibitors that can be used in the context of the present methods are anti-PCSK9 antibodies or antigen-binding fragments of antibodies that specifically bind human PCSK9.

The term "human proprotein convertase subtilisin/kexin type 9" or "human PCSK9" or "hPCSK9," as used herein, refers to PCSK9 having the nucleic acid sequence shown in SEQ ID NO:197 and the amino acid sequence of SEQ ID NO:198, or a biologically active fragment thereof.

The term "antibody," as used herein, is intended to refer to immunoglobulin molecules comprising four polypeptide chains, two heavy (H) chains and two light (L) chains inter-connected by disulfide bonds, as well as multimers thereof (e.g., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody may comprise a homo-dimer or hetero-dimer (or other multimer) of any of the variable and constant domain configurations listed above in non-covalent association with one another and/or with one or more monomeric V_{H} or V_{L} domain (e.g., by disulfide bond(s)).

As with full antibody molecules, antigen-binding fragments may be monospecific or multispecific (e.g., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present methods using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody," as used herein, is intended to include antibodies having variable and constant regions derived from human germline immunoglobulin sequences. The human antibodies may nonetheless include amino acid residues not encoded by human germline immunoglobulin sequences (e.g., mutations introduced by random or site-specific mutagenesis *in vitro* or by somatic mutation *in vivo),* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody," as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant methods encompass antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present methods. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present methods, includes antibodies that bind PCSK9 or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9, however, has cross-reactivity to other antigens, such as PCSK9 molecules from other (non-human) species.

The term "surface plasmon resonance," as used herein, refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "K_{D}," as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain embodiments, the anti-PCSK9 antibody used in accordance with the present invention is an antibody with pH-dependent binding characteristics. As used herein, the expression "pH-dependent binding" means that the antibody or antigen-binding fragment thereof exhibits "reduced binding to PCSK9 at acidic pH as compared to neutral pH" (for purposes of the present disclosure, both expressions may be used interchangeably). For the example, "antibodies with pH-dependent binding characteristics" includes antibodies and antigen-binding fragments thereof that bind PCSK9 with higher affinity at neutral pH than at acidic pH. In certain embodiments, the antibodies and antigen-binding fragments bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

Anti-PCSK9 antibodies with pH-dependent binding characteristics may possess one or more amino acid variations relative to the parental anti-PCSK9 antibody. For example, an anti-PCSK9 antibody with pH-dependent binding characteristics may contain one or more histidine substitutions or insertions, e.g., in one or more CDRs of a parental anti-PCSK9 antibody. The anti-PCSK9 antibodies with pH-dependent binding may possess, e.g., 1, 2, 3, 4, 5, 6, 7, 8, 9, or more histidine substitutions, either within a single CDR of a parental antibody or distributed throughout multiple (e.g., 2, 3, 4, 5, or 6) CDRs of a parental anti-PCSK9 antibody.

As used herein, the expression "acidic pH" means a pH of 6.0 or less (e.g., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

Non-limiting examples of anti-PCSK9 antibodies that can be used in the context of the present methods include, e.g., alirocumab, or antigen-binding portions of any of the foregoing antibody.

### Preparation of Human Antibodies

Methods for generating human antibodies in transgenic mice are known in the art. Any such known methods can be used in the context of the present methods to make human antibodies that specifically bind to human PCSK9.

Using VELOCIMMUNE^{™} technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc., using standard procedures known to those skilled in the art. The mouse constant regions are replaced with a desired human constant region to generate the fully human antibody, for example wild-type or modified IgG1 or IgG4. While the constant region selected may vary according to specific use, high affinity antigen-binding and target specificity characteristics reside in the variable region. In general, the antibodies that can be used possess high affinities, as described above, when measured by binding to antigen either immobilized on solid phase or in solution phase.

Specific examples of human antibodies or antigen-binding fragments of antibodies that specifically bind PCSK9 include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs:1 and 11, or a substantially similar sequence thereof having at least 90%, at least 95%, at least 98% or at least 99% sequence identity. Alternatively, specific examples of human antibodies or antigen-binding fragments of antibodies that specifically bind PCSK9 which can be used in the context of the methods include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs 37, 45, 53, 61, 69, 77, 85, 93, 101, 109, 117, 125, 133, 141, 149, 157, 165, 173, 181, and 189, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs 6 and 15, or a substantially similar sequence thereof having at least 90%, at least 95%, at least 98% or at least 99% sequence identity. Alternatively, the antibody or antigen-binding fragment may comprise the three light chain CDRs (LCVR1, LCVR2, LCVR3) contained within a light chain variable region (LCVR) having an amino acid sequence selected from the group consisting of SEQ ID NOs 41, 49, 57, 65, 73, 81, 89, 97, 105, 113, 121, 129, 137, 145, 153, 161, 169, 177, 185, and 193, or a substantially similar sequence thereof having at least 90%, at least 95%, at least 98% or at least 99% sequence identity.

Sequence identity between two amino acids sequences is determined over the entire length of the reference amino acid sequence, i.e., the amino acid sequence identified with a SEQ ID NO, using the best sequence alignment and/or over the region of the best sequence alignment between the two amino acid sequences, wherein the best sequence alignment can be obtained with art known tools, e.g., Align, using standard settings, preferably EMBOSS::needle, Matrix: Blosum62, Gap Open 10.0, Gap Extend 0.5.

The anti-PCSK9 antibody, or antigen-binding protein, that is used in accordance with the present invention has HCDR1/HCDR2/HCDR3/LCDR1/LCDR2/LCDR3 amino acid sequences selected from SEQ ID NOs: 2/3/4/7/8/10 (mAb316P [also referred to as "REGN727," or "alirocumab"]).

### Pharmaceutical Compositions and Methods of Administration

The PCSK9 antibody or antigen-binding fragment thereof can be contained within a pharmaceutical composition. The pharmaceutical compositions are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Exemplary pharmaceutical formulations comprising anti-PCSK9 antibodies that can be used in the context of the present methods include any of the formulations as set forth in US 8,795,669 (describing, *inter alia,* exemplary formulations comprising alirocumab), or in WO2013/166448, or WO2012/168491.

Various delivery systems are known and can be used to administer the pharmaceutical composition, e.g., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition can be delivered subcutaneously or intravenously with a standard needle and syringe. In addition, with respect to subcutaneous delivery, a pen delivery device readily has applications in delivering a pharmaceutical composition. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany) and the like. Examples of disposable pen delivery devices having applications in subcutaneous delivery of a pharmaceutical composition of the present methods include, but are not limited to the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL) and the like.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, e.g., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

In some embodiments, the antibody or antigen-binding fragment thereof is administered as a subcutaneous injection into the thigh, abdomen, or upper arm using a single-use pre-filled pen or single-use pre-filled syringe. The injection site can be rotated with each injection. The antibody or antigen-binding fragment thereof should not be injected into areas of active skin disease or injury such as sunburns, skin rashes, inflammation, or skin infections.

### Dosage

The amount of the PCSK9 antibody or antigen-binding fragment thereof (e.g., anti-PCSK9 antibody) administered to a patient is, generally, a therapeutically effective amount. As used herein, the phrase "therapeutically effective amount" means a dose of PCSK9 antibody or antigen-binding fragment thereof that results in a detectable reduction (e.g., at least about 5%, about 10%, about 15%, about 20%, about 25%, about 30%, about 35%, about 40%, about 45%, about 50%, about 55%, about 60%, about 65%, about 70%, about 75% or more from baseline) in one or more parameters selected from the group consisting of LDL-C, ApoB, ApoB100, non-HDL-C, total cholesterol, VLDL-C, triglycerides, ApoC3, TRL particles, Lp(a) and remnant cholesterol).

In the case of an anti-PCSK9 antibody, a therapeutically effective amount can be from about 0.05 mg to about 600 mg, e.g., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody. According to certain exemplary embodiments, a therapeutically effective amount of an anti-PCSK9 antibody is 30 mg, 40 mg or 75 mg (e.g., in the case of alirocumab for patients with body weight less than 50 kg, and/or younger than or equal to 17 years old), 50 mg, 75 mg or 150 mg (e.g., in the case of alirocumab for patients with body weight greater than or equal to 50 kg, and/or younger than or equal to 17 years old), or 140 mg or 420 mg (e.g., in the case of evolocumab). Other dosing amounts of PCSK9 inhibitors will be apparent to persons of ordinary skill in the art.

The amount of anti-PCSK9 antibody contained within the individual doses may be expressed in terms of milligrams of antibody per kilogram of patient body weight (i.e., mg/kg). For example, the anti-PCSK9 antibody may be administered to a patient at a dose of about 0.0001 to about 10 mg/kg of body weight.

### Administration Regimens

According to certain embodiments, multiple doses of the PCSK9 antibody or antigen-binding fragment thereof (i.e., a pharmaceutical composition comprising the PCSK9 antibody or antigen-binding fragment thereof) may be administered to a patient over a defined time course (e.g., on top of a daily therapeutic statin regimen or other background LMT). This aspect comprises sequentially administering to a patient multiple doses of the PCSK9 antibody or antigen-binding fragment thereof. As used herein, "sequentially administering" means that each dose of the PCSK9 antibody or antigen-binding fragment thereof is administered to the patient at a different point in time, e.g., on different days separated by a predetermined interval (e.g., hours, days, weeks or months). The present invention includes sequentially administering to the patient a single initial dose of the PCSK9 antibody or antigen-binding fragment thereof, followed by one or more secondary doses of the PCSK9 antibody or antigen-binding fragment thereof, and optionally followed by one or more tertiary doses of the PCSK9 antibody or antigen-binding fragment thereof.

The terms "initial dose," "secondary doses," and "tertiary doses," refer to the temporal sequence of administration of the individual doses of a pharmaceutical composition comprising a PCSK9 antibody or antigen-binding fragment thereof. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the PCSK9 antibody or antigen-binding fragment thereof, but generally may differ from one another in terms of frequency of administration. In certain embodiments, however, the amount of PCSK9 inhibitor contained in the initial, secondary and/or tertiary doses varies from one another (e.g., adjusted up or down as appropriate) during the course of treatment. In certain embodiments, two or more (e.g., 2, 3, 4, or 5) doses are administered at the beginning of the treatment regimen as "loading doses" followed by subsequent doses that are administered on a less frequent basis (e.g., "maintenance doses").

According to exemplary embodiments, each secondary and/or tertiary dose is administered 1 to 26 (e.g., 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule which is administered to a patient prior to the administration of the very next dose in the sequence with no intervening doses.

This aspect may comprise administering to a patient any number of secondary and/or tertiary doses of the PCSK9 antibody or antigen-binding fragment thereof. For example, in certain embodiments, only a single secondary dose is administered to the patient. In other embodiments, two or more (e.g., 2, 3, 4, 5, 6, 7, 8, or more) secondary doses are administered to the patient. Likewise, in certain embodiments, only a single tertiary dose is administered to the patient. In other embodiments, two or more (e.g., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

The present invention includes administration regimens comprising an up-titration option (also referred to herein as "dose modification"). As used herein, an "up-titration option" means that, after receiving a particular number of doses of a PCSK9 antibody or antigen-binding fragment thereof, if a patient has not achieved a specified reduction in one or more defined therapeutic parameters, the dose of the PCSK9 inhibitor is thereafter increased. For example, in the case of a therapeutic regimen comprising administration of 75 mg doses of an anti-PCSK9 antibody to a patient at a frequency of once every two weeks, if after 4 weeks (i.e., 2 doses) or 8 weeks (i.e., 5 doses administered at week 0, week 2, week 4, week 6 and week 8), the patient has not achieved a serum LDL-C concentration of less than 50 mg/dL, then the dose of anti-PCSK9 antibody is increased to e.g., 150 mg administered once every two weeks thereafter (e.g., starting at week 10 or week 12, or later).

In certain embodiments, the antibody or antigen-binding fragment thereof that specifically binds PCSK9 is administered to the patient at a dose of about 75 mg at a frequency of once every two weeks. In certain embodiments, the about 75 mg dose is maintained if the patient's LDL-C measured after one or more, two or more, three or more, four or more, or five or more doses is <70 mg/dL. In certain embodiments, the about 75 mg dose is discontinued if the patient's LDL-C measured after one or more, two or more, three or more, four or more, or five or more doses remains ≥70 mg/dL, and the antibody or antigen-binding fragment thereof that specifically binds PCSK9 is subsequently administered to the patient at a dose of about 150 mg at a frequency of once every two weeks.

In certain embodiments, the antibody or antigen-binding fragment thereof that specifically binds PCSK9 is administered to the patient at a dose of about 150 mg at a frequency of once every two weeks. In certain embodiments, when the antibody or antigen-binding fragment thereof that specifically binds PCSK9 is administered to the patient at a dose of about 150 mg at a frequency of once every two weeks, the about 150 mg dose is discontinued if the patient's LDL-C measured after at least one dose or at least two, three, four, or five consecutive doses is < 10, 15, 20, or 25 mg/dL, and the antibody or antigen-binding fragment thereof that specifically binds PCSK9 is subsequently administered to the patient at a dose of about 75 mg at a frequency of once every two weeks. In certain embodiments, the about 150 mg dose is administered to the patient as a constant dose. In certain embodiments, the about 150 mg dose is administered to the patient after a dose adjustment as disclosed herein (e.g., from about 75 mg every two weeks, or from about 300 mg every four weeks).

In certain embodiments, the antibody or antigen-binding fragment thereof that specifically binds PCSK9 is administered to the patient at a dose of about 300 mg at a frequency of once every four weeks. In certain embodiments, the about 300 mg dose is maintained if the patient's LDL-C measured after one or more, two or more, three or more, four or more, or five or more doses is <70 mg/dL. In certain embodiments, the about 300 mg dose is discontinued if the patient's LDL-C measured after one or more, two or more, three or more, four or more, or five or more doses remains ≥70 mg/dL, and the antibody or antigen-binding fragment thereof that specifically binds PCSK9 is subsequently administered to the patient at a dose of about 150 mg at a frequency of once every two weeks.

In certain embodiments, the anti-PCSK9 antibody is administered to a patient at a dose of about 75 mg every two weeks, for example for at least two doses.

In certain embodiments, the anti-PCSK9 antibody is administered to a patient at a dose of about 150 mg every two weeks, for example for at least two doses.

In some embodiments, the antibody is administered to a patient at a dose of about 300 mg every two weeks or every 4 weeks.

In additional embodiments, if the patient's LDL-C is <25 mg/dL on any 2 consecutive measurements on a dose of 150 mg, the dose is thereafter reduced to 75 mg.

### Combination Therapies

As described elsewhere herein, the present invention may comprise administering a PCSK9 inhibitor to a patient in combination with ("on top of") the patient's previously prescribed lipid modifying therapy (LMT). LMTs include, but are not limited to statins, fibrates, niacin (e.g., nicotinic acid and its derivatives), bile acid sequestrants, ezetimibe, lomitapide, phytosterols, orlistat, etc. For example, the PCSK9 antibody or antigen-binding fragment thereof may be administered to a patient in combination with a stable daily therapeutic statin regimen. Exemplary daily therapeutic statin regimens that the PCSK9 antibody or antigen-binding fragment thereof may be administered in combination with in the context of the present invention include, e.g., atorvastatin (10, 20, 40 or 80 mg daily), (atorvastatin/ezetimibe 10/10 or 40/10 mg daily), rosuvastatin (5, 10 or 20 mg daily), cerivastatin (0.4 or 0.8 mg daily), pitavastatin (1, 2 or 4 mg daily), fluvastatin (20, 40 or 80 mg daily), simvastatin (5, 10, 20, 40 or 80 mg daily), simvastatin/ezetimibe (10/10, 20/10, 40/10 or 80/10 mg daily), lovastatin (10, 20, 40 or 80 mg daily), pravastatin (10, 20, 40 or 80 mg daily), and combinations thereof. In certain embodiments, the statin therapy is a maximally tolerated statin therapy for the patient. Other LMTs that a PCSK9 inhibitor may be administered in combination with in the context of the present methods include, e.g., (1) an agent which inhibits cholesterol uptake and or bile acid re-absorption (e.g., ezetimibe); (2) an agent which increase lipoprotein catabolism (such as niacin); and/or (3) activators of the LXR transcription factor that plays a role in cholesterol elimination such as 22-hydroxycholesterol.

In the context of the invention, additional therapeutically active component(s), e.g., any of the agents listed above or derivatives thereof, may be administered just prior to, concurrent with, or shortly after the administration of the PCSK9 antibody or antigen-binding fragment thereof; (for purposes of the present disclosure, such administration regimens are considered the administration of the PCSK9 antibody or antigen-binding fragment thereof "in combination with" an additional therapeutically active component). The present invention includes pharmaceutical compositions and uses thereof in which the PCSK9 antibody or antigen-binding fragment thereof is co-formulated with one or more of the additional therapeutically active component(s) as described elsewhere herein.

### EXAMPLES

The following examples are put forth so as to provide those of ordinary skill in the art with a complete disclosure and description of how to make and use the invention, and are not intended to limit the scope of what the inventors regard as their invention. Efforts have been made to ensure accuracy with respect to numbers used (e.g., amounts, temperature, etc.) but some experimental errors and deviations should be considered. Unless indicated otherwise, "parts" are parts by weight," molecular weight" is average molecular weight, temperature is in degrees Centigrade, and pressure is at or near atmospheric.

### Example 1. Generation of Human Antibodies to Human PCSK9

Human anti-PCSK9 antibodies were generated as described in US Patent No. 8,062,640. The exemplary PCSK9 inhibitor used in the following Examples is the human anti-PCSK9 antibody designated "mAb316P," also known as "REGN727," or "alirocumab." mAb316P has the following amino acid sequence characteristics: a heavy chain comprising SEQ ID NO:5 and a light chain comprising SEQ ID NO:9; a heavy chain variable region (HCVR) comprising SEQ ID NO:1 and a light chain variable domain (LCVR) comprising SEQ ID NO:6; a heavy chain complementarity determining region 1 (HCDR1) comprising SEQ ID NO:2, a HCDR2 comprising SEQ ID NO:3, a HCDR3 comprising SEQ ID NO:4, a light chain complementarity determining region 1 (LCDR1) comprising SEQ ID NO:7, a LCDR2 comprising SEQ ID NO:8 and a LCDR3 comprising SEQ ID NO:10.

### Example 2: Effect of Alirocumab, a Monoclonal Antibody to PCSK9, on Long-term Cardiovascular Outcomes Following Acute Coronary Syndrome: Protocol

### INTRODUCTION

Statins have been approved for clinical use since 1987. However, since that time, no non-statin lipid lowering therapy has been associated with reduced death from any cause. Inhibition of PCSK9 provides an opportunity to determine whether substantial further reduction of LDL-C and other atherogenic lipoproteins can provide further improvement in cardiovascular outcomes beyond those afforded by statins, including an impact on reduced mortality.

This study queried whether alirocumab, a fully human monoclonal antibody to PCSK9, could reduce cardiovascular risk, including mortality, when added to optimal statin therapy. Patients with recent acute coronary syndrome were chosen as the study population because they faced a higher risk of recurrent events than patients with stable cardiovascular disease, and therefore might derive a larger absolute benefit from an effective new treatment. The study population was further defined by minimum qualifying levels of atherogenic lipoproteins in order to target those whose residual cardiovascular risk was likely to be modified by further reduction of these lipoproteins. This study also provided substantial information regarding the safety of PCSK9 inhibition.

### STUDY OBJECTIVE

The present study was an investigator-initiated, international, multicenter, randomized, double-blind, placebo-controlled study in approximately 18,000 patients with recent acute coronary syndrome (ACS). The primary objective was to evaluate whether alirocumab (75-150 mg by subcutaneous injection every 2 weeks), initiated 1-12 months after an index ACS event, reduced the incidence of the composite outcome of coronary heart disease death, major non-fatal coronary events (acute myocardial infarction or hospitalization for unstable angina), or ischemic stroke.

### STUDY POPULATION

Principal inclusion criteria were: 1) hospitalization for ACS, defined by symptoms of myocardial ischemia with an unstable pattern, occurring at rest or with minimal exertion, within 72 hours of an unscheduled hospital admission, due to presumed or proven obstructive coronary disease and at least one of the following: a) elevated cardiac biomarkers, or b) resting ECG changes consistent with ischemia or infarction, plus additional evidence of obstructive coronary disease from regional wall motion or perfusion abnormality, ≥70% epicardial coronary stenosis by angiography, or need for coronary revascularization procedure; and 2) lipid levels inadequately controlled by atorvastatin 40-80 mg or rosuvastatin 20-40 mg daily or maximum tolerated dose of one of these agents, defined by at least one of the following: a) LDL-C ≥70 mg/dl, b) non-HDL-C ≥100 mg/dl, or c) apolipoprotein B ≥80 mg/dl.

Principal exclusion criteria were: 1) age <40 years; 2) qualifying index ACS event <4 or >52 weeks before randomization; 3) not on stable lipid-modifying therapy for at least 2 weeks before randomization; 4) uncontrolled hypertension (>180 mm Hg systolic and/or >110 mm Hg diastolic at randomization visit); 5) New York Heart Association Class III or IV congestive heart failure persisting despite treatment or LVEF <25% if measured; 6) history of hemorrhagic stroke; 7) fasting triglycerides >400 mg/dl (4.52 mmol/L) at qualifying laboratory visit; 8) recurrent ACS event within 2 weeks prior to randomization visit; 9) coronary revascularization procedure performed within 2 weeks prior to randomization visit or planned after randomization; 10) liver transaminases >3 times upper limit of normal; laboratory evidence of current hepatitis B or C infection; creatine kinase >3 times upper limit of normal; estimated glomerular filtration rate <30 ml/min/1.73m2; positive urine or serum pregnancy test; 11) recent diagnosis of hypothyroidism with treatment initiated within 1 month of qualifying laboratory visit; 12) cancer with past 5 years except for adequately treated basal or squamous cell skin cancer or in situ cervical cancer; 13) previous treatment with any PCSK9 antibody; 14) use of fibrates, other than fenofibrate or fenofibric acid, during the run-in period; and 15) inability to provide informed consent or comply with study requirements; pregnancy, lactation, or childbearing potential without use of effective contraception.

The trial was enrolling male and female patients at least 40 years of age who had been hospitalized for ACS defined by unstable symptoms of myocardial ischemia occurring at rest or minimal exertion within 72 hours of an unscheduled hospital admission, due to presumed or proven obstructive coronary disease. In addition, a qualifying ACS event required at least one of the following criteria to be fulfilled: elevated cardiac biomarkers consistent with acute myocardial infarction, or resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.

Qualifying patients must have had demonstrated inadequate control of atherogenic lipoproteins despite steady state (at least 2 weeks') treatment with atorvastatin 40-80 mg daily, rosuvastatin 20-40 mg daily, or the maximum tolerated dose of one of these agents. Thus, the background statin treatment in the trial was concordant with secondary prevention guidelines of the American Heart Association and American College of Cardiology for reduction of blood cholesterol levels (see Stone et al., Circulation (2014) 129:S1-45.). Inadequate control of atherogenic lipoproteins was defined by at least one of the following: LDL-C ≥70 mg/dL (1.81 mmol/L), non-high density lipoprotein cholesterol (non-HDL-C) ≥100 mg/dL (2.59 mmol/L), or apolipoprotein B ≥80 mg/dL (0.8 mmol/L).

### STUDY PROCEDURES

Patients entered a run-in period of duration 2-16 weeks. During this period, patients were instructed in the technique of self-injection using the study auto-injector device. Atorvastatin (40-80 mg daily) or rosuvastatin (20-40 mg daily) was initiated and/or adjusted as necessary to determine the maximum tolerated dose. Other non-excluded lipid-modifying therapies may also have been initiated during the run-in period, at the investigator's discretion. After at least two weeks of steady-state lipid modifying therapy, a fasting blood sample was obtained to determine if at least one of the qualifying lipoprotein criteria was met.

Patients who met all inclusion and no exclusion criteria at the end of the run-in period were randomly assigned to initial treatment with alirocumab 75 mg (1 ml injection volume) subcutaneously every 2 weeks, or matching placebo. Follow-up visits occurred 1, 2, 4, 8, 12, 16, 20, and 24 months after randomization, then at 6 month intervals until the common study end date. At randomization and at multiple time points after randomization, patients were assessed for study end points and adverse events, and blood and urine samples were collected for measurements including lipoproteins and apolipoproteins; hematology and chemistry studies including liver, muscle, and kidney function tests; hemoglobin A1c; high sensitivity C-reactive protein (hsCRP), anti-alirocumab antibodies; and pregnancy testing in women of child-bearing potential. LDL-C was calculated using the Friedewald formula, except that values <15 mg/dl were confirmed by direct measurement (as well as when the TG values exceeded 400 mg/dL (4.52 mmol/L)). Samples were also collected for measurement of PCSK9 levels, lipoprotein sub-fractions, and other mediators of inflammation and cardiovascular risk. An electrocardiogram was recorded at randomization and at study completion. During the randomized treatment period, lipoprotein levels remained blinded to patients and investigators, and treating physicians were instructed to refrain from usual clinical lipoprotein testing.

This study was seeking to determine whether achieving LDL-C levels in the lower portion of the physiologic range improves clinical outcomes; the trial was not designed to explore the safety of sustained, sub-physiologic LDL-C levels. Accordingly, blinded dose adjustment and monitoring procedures were incorporated in the protocol, as follows. Among patients assigned to treatment with alirocumab, if LDL-C measured 1 month after randomization (i.e., after 2 doses of alirocumab 75 mg every 2 weeks) remained ≥50 mg/dl, the dose of alirocumab was increased in a blinded fashion to 150 mg every 2 weeks. If LDL-C measured 1 month after randomization was <50 mg/dl, the dose of alirocumab was maintained at 75 mg. If LDL-C was <25 mg/dl on any 2 consecutive measurements on alirocumab 150 mg, the dose was reduced to 75 mg. If LDL-C was <25 mg/dl but ≥15 mg/dl on 2 consecutive measurements on alirocumab 75 mg, that dose was continued but the patient was monitored for potentially related adverse events by an independent safety physician who reports individual and aggregate findings to the Data Safety Monitoring Board (DSMB) and recommends blinded discontinuation of treatment if data suggest that an adverse event is causally related to treatment. If LDL-C was <15 mg/dl on 2 consecutive measurements during treatment with alirocumab 75 mg, active treatment was discontinued at the next study visit and placebo injections were substituted in a blinded manner for the remaining duration of the study. In composite, these blinded dose adjustments were intended to maximize the number of patients in the alirocumab group with LDL-C<50 mg/dl, while minimizing the number of patients with sustained levels of LDL-C<15 mg/dl.

### STUDY OUTCOMES

The primary efficacy measure was the time to first occurrence of coronary heart disease death, major non-fatal coronary event (myocardial infarction or hospitalization for unstable angina), or ischemic stroke.

Secondary end points included ischemia-driven coronary revascularization procedures, hospitalization for congestive heart failure, and all-cause mortality.

The primary efficacy measures were: time to first occurrence of coronary heart disease death, non-fatal acute myocardial infarction, fatal or non-fatal ischemic stroke, or unstable angina requiring hospitalization.

The main secondary efficacy measures were (in hierarchical order): 1) time from randomization to first occurrence of major coronary heart disease event (coronary heart disease death or non-fatal myocardial infarction), unstable angina requiring hospitalization, or ischemia-driven coronary revascularization procedure; 2) time from randomization to first occurrence of a major coronary heart disease event; 3) time from randomization to first occurrence of any cardiovascular event (any cardiovascular death, any non-fatal coronary heart disease event, or non-fatal ischemic stroke); 4) time from randomization to first occurrence of all-cause mortality, non-fatal myocardial infarction, or non-fatal ischemic stroke; and 5) time from randomization to death (all-cause mortality).

Other secondary efficacy measures were: 1) time from randomization to coronary heart disease death; 2) time from randomization to first occurrence of non-fatal myocardial infarction; 3) time from randomization to first occurrence of ischemic stroke; 4) time from randomization to first occurrence of unstable angina requiring hospitalization; 5) time from randomization to first occurrence of ischemia-driven coronary revascularization procedure; and 6) time from randomization to first occurrence of congestive heart failure requiring hospitalization.

Safety measures were: all adverse events; heart rate and blood pressure; hematology; and biochemistry assessments.

Other measures were: 1) anti-alirocumab antibodies assessed throughout the study; and 2) percent change in LDL cholesterol, apolipoprotein B, non-HDL cholesterol, and high sensitivity C-reactive protein.

Laboratory efficacy end points included change from baseline in calculated LDL-C, apolipoprotein B, non-HDL-C, and hsCRP. Safety of alirocumab treatment was assessed by reporting of adverse events and laboratory tests. Adverse events of special interest in this trial included allergic events, local injection site reactions, liver enzyme increase, and hemolytic anemia.

### STATISTICAL CONSIDERATIONS

The projected Kaplan-Meier incidence of a primary end point event in the placebo group was 3.8% at 12 months, 6.4% at 24 months, 9.0% at 36 months, and 11.4% at 48 months. Other assumptions included 1% of patients lost to follow-up through 24 months, a median LDL-C at baseline of 90 mg/dl, and a 50% reduction of LDL-C from baseline with alirocumab treatment, resulting in a 15% hazard reduction. Based on these assumptions and specifying a log-rank test at a 1-sided 2.49% significance level to account for two interim analyses, the trial had 90% power with 1613 primary end point events, corresponding to a sample size of 18,000 patients randomized over 40 months. To allow sufficient duration of exposure to alirocumab for thorough assessments of safety and efficacy, the trial continued until 1613 primary end point events have occurred and all evaluable surviving patients have been followed for at least 2 years. For the primary outcome, treatment effects were examined across subgroups categorized according to gender, age, race, geographical region, and time from ACS event to randomization. Time-to-event secondary outcomes were analyzed using the same methodology as for the primary end point. For main secondary outcomes, the overall type 1 error was controlled by use of a sequential inferential approach. Proportional hazards regression models were constructed to include changes in or absolute values of LDL-C and other lipid parameters. Analysis of subpopulations defined by categorical and continuous variables were performed according to a pre-specified statistical analysis plan. Safety results were presented by treatment group without formal inferential testing.

The independent DSMB, composed of 3 cardiologists, 1 lipidologist, and 1 statistician, reviewed interim data at regular intervals to assess safety and efficacy. When approximately 50% of events had occurred, the DSMB conducted an interim analysis for futility (non-binding boundary corresponding to hazard ratio >1.008). When approximately 75% of events had occurred, the DSMB conducted a second interim analysis for futility (non-binding boundary corresponding to hazard ratio >0.951) and overwhelming efficacy (hazard ratio <0.802 corresponding to p<0.0001 for the primary end point with consistency across subgroups and regions, positive trends for secondary end points including all-cause mortality, and no excess non-cardiovascular mortality).

### Example 3: Effect of Alirocumab, a Monoclonal Antibody to PCSK9, on Long-term Cardiovascular Outcomes Following Acute Coronary Syndrome: Results

This study showed for the first and only time that a non-statin lipid lowering therapy was associated with reduced death from any cause.

### STUDY PATIENTS

A total of 18,924 patients were enrolled at 1,315 sites in 57 countries in this study. The qualifying acute coronary syndrome (ACS) was myocardial infarction in 83.2% of patients and unstable angina in 16.8% of patients. Most patients (92.1%) qualified with an LDL cholesterol of 70 mg per deciliter or higher. An additional 4.2% fulfilled only the non-HDL-cholesterol criterion and 0.3% fulfilled only the apolipoprotein B criterion. Median time from qualifying acute coronary syndrome to randomization was 2.6 months (interquartile range, 1.7 to 4.3) for the entire population, 2.6 months (interquartile range, 1.7 to 4.4) for alirocumab treatment group, and 2.6 months (interquartile range, 1.7 to 4.3) for the placebo treatment group.

Among these patients, 9,462 of them were in the alirocumab treatment group, and 9,462 of them were in the placebo treatment group. At randomization, baseline characteristics of the two treatment groups were well balanced **(Table 1).** Most patients were treated with guideline-recommended secondary prevention medications and underwent coronary revascularization for the index event. At randomization, 88.8% of patients were treated with atorvastatin 40 to 80 mg daily or rosuvastatin 20 to 40 mg daily. After 1 and 3 years of follow-up, the corresponding percentages were 84.7% and 82.8% in the alirocumab group and 86.2% and 86.6% in the placebo group. There were no nominally significant differences between the two groups in baseline characteristics.

**Table 1. Baseline Characteristics of the Patients***

| **Characteristic** | | | **Alirocumab (N=9462)** | **Placebo (N=9462)** |
|---|---|---|---|---|
| Age - yr | | | 58.5 ± 9.3 | 58.6 ± 9.4 |
| Female sex - no. (%) | | | 2390 (25.3) | 2372 (25.1) |

| Race† - no. (%) | | | | |
|---|---|---|---|---|
| | White | | 7500 (79.3) | 7524 (79.5) |
| | Asian | | 1251 (13.2) | 1247 (13.2) |
| | Black or African American | | 235 (2.5) | 238 (2.5) |
| | Other | | 475 (5.0) | 451 (4.8) |

| Region of enrollment - no. (%) | | | | |
|---|---|---|---|---|
| | Central and Eastern Europe | | 2719 (28.7) | 2718 (28.7) |
| | Western Europe | | 2084 (22.0) | 2091 (22.1) |
| | Canada/USA | | 1435 (15.2) | 1436 (15.2) |
| | Latin America | | 1293 (13.7) | 1295 (13.7) |
| | Asia | | 1150 (12.2) | 1143 (12.1) |
| Rest of the world | | | 781 (8.3) | 779 (8.2) |

| Medical history prior to index acute coronary syndrome - no. (%) | | | | |
|---|---|---|---|---|
| Hypertension | | | 6205 (65.6) | 6044 (63.9) |
| Diabetes mellitus | | | 2693 (28.5) | 2751 (29.1) |
| Current tobacco smoker | | | 2282 (24.1) | 2278 (24.1) |
| Family history of premature coronary heart disease | | | 3408 (36.0) | 3365 (35.6) |
| Myocardial infarction | | | 1790 (18.9) | 1843 (19.5) |
| Percutaneous coronary intervention | | | 1626 (17.2) | 1615 (17.1) |
| Coronary artery bypass graft | | | 521 (5.5) | 526 (5.6) |
| Stroke | | | 306 (3.2) | 305 (3.2) |
| Peripheral arterial disease | | | 373 (3.9) | 386 (4.1) |
| Conqestive heart failure | | | 1365 (14.4) | 1449 (15.3) |

| Index acute coronary syndrome event - no. (%) | | | | |
|---|---|---|---|---|
| ST-segment elevation myocardial infarction | | | 3301 (35.0) | 3235 (34.2) |
| Non-ST-segment elevation myocardial infarction | | | 4574 (48.4) | 4601 (48.7) |
| Unstable angina | | | 1568 (16.6) | 1614 (17.1) |
| Percutaneous coronary intervention or coronary artery bypass graft for index acute coronary syndrome - no. (%) | | | 6798 (71.8) | 6878 (72.7) |
| Time from index acute coronary syndrome to randomization - weeks | | | 11.3 (7.6-19.0) | 11.4 (7.6-18.7) |
| Body mass index‡ | | | 28.5 ± 4.9 | 28.5 ± 4.8 |

| Medications at randomization - no. (%) | | | | |
|---|---|---|---|---|
| Lipid-modifying agents | | | | |
| High-dose atorvastatin/rosuvastatin | | | 8380 (88.6) | 8431 (89.1) |
| Atorvastatin 80 mg or rosuvastatin 40 mg | | | 2607 (27.6) | 2572 (27.2) |
| Atorvastatin 40 mg or rosuvastatin 20 mg | | | 5773 (61.0) | 5859 (61.9) |
| Low-/moderate-dose atorvastatin/rosuvastatin | | | 830 (8.8) | 777 (8.2) |
| Other statin | | | 19 (0.2) | 27 (0.3) |
| No statin | | | 227 (2.4) | 233 (2.5) |
| Ezetimibe | | | 269 (2.8) | 285 (3.0) |

| Other | | | | |
|---|---|---|---|---|
| Antiplatelet agents | | | 9350 (98.8) | 9354 (98.9) |
| Aspirin | | | 9050 (95.6) | 9036 (95.5) |
| Adenosine diphosphate receptor antagonist | | | 8296 (87.7) | 8245 (87.1) |
| Vitamin K antagonist or other oral anticoagulant | | | 378 (4.1) | 403 (4.3) |
| Beta-blocker | | | 7998 (84.5) | 7992 (84.5) |
| Angiotensin converting enzyme inhibitor or angiotensin II receptor blocker | | | 7356 (77.7) | 7360 (77.8) |
| Use of a statin during 3 months immediately prior to index acute coronary syndrome - no. (%) | | | 3090 (32.7) | 3074 (32.5) |

| Lipid levels at randomization* | | | | |
|---|---|---|---|---|
| LDL cholesterol§ | | | | |
| Mean | | | 92 ± 31 | 92 ± 31 |
| Median | | | 87 (73-104) | 87 (73-104) |

| Non-HDL cholesterol | | | | |
|---|---|---|---|---|
| | | Mean | 122 ± 35 | 123 ± 36 |
| | | Median | 115 (99-136) | 115 (99-137) |

| | Apolipoprotein B | | | |
|---|---|---|---|---|
| | | Mean | 83 ± 21 | 83 ± 22 |
| | | Median | 79 (69-93) | 80 (69-93) |
| | HDL cholesterol | | 43 (37-50) | 42 (36-50) |
| | Apolipoprotein A1 | | 131 (118-148) | 132 (117-147) |
| | Triglycerides | | 129 (94-181) | 129 (95-183) |
| | Lipoprotein(a) | | 21 (7-59) | 22 (7-60) |
| Hemoglobin A_{1c} - % | | | 6.21 ± 1.23 | 6.21 ± 1.23 |
| Creatinine clearance - ml/min per 1.73 m² | | | 79.5±19.4 | 79.8±19.2 |
| High-sensitivity C-reactive protein - mg/l | | | 1.61 (0.78-3.79) | 1.67 (0.79-3.94) |

| | | | | |
|---|---|---|---|---|
| * Lipid levels are reported in units of mg per deciliter. To convert the values for cholesterol to millimoles per liter, multiply by 0.02586. To convert the values for triglycerides to millimoles per liter, multiply by 0.01129. HDL denotes high-density lipoprotein, and LDL low-density lipoprotein. Data are median (interquartile range) except mean (standard deviation) as noted. † Race was self-reported. ‡ Body-mass index is the weight in kilograms divided by the square of the height in meters. § LDL cholesterol was calculated with the Friedewald formula; calculated values <15 mg per deciliter were confirmed by ultracentrifugation/beta quantification. | | | | |

### STUDY TREATMENT

At first assessment following randomization, 2615 (27.6%) patients treated with alirocumab had an LDL-cholesterol level of 50 mg per deciliter or higher and subsequently underwent blinded up titration of alirocumab from 75 mg to 150 mg. Of these 2615 patients, 805 later underwent blinded down titration to 75 mg after two consecutive LDL cholesterol measurements below 25 mg per deciliter. Of all patients treated with alirocumab, 730 (7.7%) had two consecutive LDL-cholesterol measurements below 15 mg per deciliter on the 75 mg dose, leading to blinded switch to placebo injections a median 8.3 months from randomization. Among patients who were blindly switched to placebo, median baseline LDL cholesterol was 71 (interquartile range, 58 to 83) mg per deciliter. Of the total time on treatment with alirocumab, 78% and 22% were at the 75 mg and 150 mg doses, respectively.

Patients were followed for a median of 2.8 (interquartile range, 2.3 to 3.4) years. Premature treatment discontinuation for reasons other than death occurred in 1,343 (14.2%) patients in the alirocumab treatment group and in 1,496 (15.8%) patients in the placebo treatment group. Additionally, 14 and 9 patients in the alirocumab and placebo treatment groups, respectively, were lost to follow-up (vital status). Due to having two consecutive LDL values below 15 mg/dL, 730 (7.7%) of the patients in the alirocumab treatment group were switched to the placebo treatment group in a blinded manner. Exposure to intended treatment (including blinded switch from alirocumab to placebo) as a percentage of total follow-up time was 86% and 91% in the alirocumab and placebo groups, respectively. Ascertainment was complete for 99.1% and 99.8% of potential patient-years of follow-up for the primary endpoint and death, respectively.

The patients were treated for an average (median) of 33 months.

### EFFECT OF TREATMENT ON LIPOPROTEINS

At baseline, LDL cholesterol was 92 ± 31 mg per deciliter (2.39 ± 0.80 mmol per liter), (mean ± standard deviation). On an intention-to-treat basis in the alirocumab group, mean LDL cholesterol reached a nadir of 40 mg per deciliter (1.03 mmol per liter) at 4 months, rising to 48 mg per deciliter (1.24 mmol per liter) at 12 months, and 66 mg per deciliter (1.72 mmol per liter) at 48 months (**Figure 1A****).** Excluding values collected after discontinuation of alirocumab, mean LDL-cholesterol levels at 4, 12, and 48 months were 38, 42, and 53 mg per deciliter (0.97, 1.09, and 1.38 mmol per liter), respectively, corresponding to 62.7%, 61.0%, and 54.7% average placebo-corrected reductions. The absolute reductions of mean LDL-cholesterol levels in the alirocumab group relative to the placebo group were 55.7 mg/dL, 54.1 mg/dL, and 48.1 mg/dL at 4, 12, and 48 months, respectively. In the placebo group, mean LDL-cholesterol levels (intention to treat) at 4, 12, and 48 months were 93, 96, and 103 mg per deciliter (2.42, 2.50, and 2.67 mmol per liter), respectively. Other lipid measurements are provided in **Figures 1B-1G****.**

### EFFICACY END POINTS

The primary end point occurred in 903 (9.5%) patients in the alirocumab group and 1052 (11.1%) in the placebo group **(Table 2);** 4-year Kaplan-Meier rate estimates were 12.5% and 14.5% in the alirocumab and placebo groups, respectively (hazard ratio 0.85; 95% confidence interval, 0.78 to 0.93; P<0.001) **(****Figure 2****).** Thus, on this endpoint, alirocumab reduced the risk of MACE by 15%. To prevent one primary end point event would require 49 patients to be treated for 4 years. MACE was a composite endpoint which included patients who experienced (1) death from coronary heart disease (CHD), (2) non-fatal myocardial infarction, (3) fatal or non-fatal ischemic stroke, or (4) unstable angina requiring hospitalization. As shown in **Table 2,** each component of the MACE endpoint was lower in the alirocumab-treated patients, showing a statistically significant difference compared to placebo for non-fatal myocardial infarction, fatal or non-fatal ischemic stroke, and unstable angina requiring hospitalization.

**Table 2. Primary End Point and Secondary End Points (Intention-to-Treat Population).***

| **Outcome** | **Alirocumab (N=9462)** | **Placebo (N=9462)** | **Hazard Ratio (95% confidence interval)** | **P Value** |
|---|---|---|---|---|
| | ***no. of patients (%)*** | | | |
| Primary end point: coronary heart disease death, non-fatal myocardial infarction, fatal or non-fatal ischemic stroke, or hospitalization for unstable angina | 903 (9.5) | 1052 (11.1) | 0.85 (0.78, 0.93) | <0.001 |

| Secondary end points (in order of hierarchical testing) | | | | |
|---|---|---|---|---|
| Any coronary heart disease event* | 1199 (12.7) | 1349 (14.3) | 0.88 (0.81, 0.95) | 0.001 |
| Major coronary heart disease event† | 793 (8.4) | 899 (9.5) | 0.88 (0.80, 0.96) | 0.006 |
| Any cardiovascular event‡ | 1301 (13.7) | 1474 (15.6) | 0.87 (0.81, 0.94) | <0.001 |
| Death from any cause, non-fatal myocardial infarction, non-fatal ischemic stroke | 973 (10.3) | 1126 (11.9) | 0.86 (0.79, 0.93) | <0.001 |
| Coronary heart disease death | 205 (2.2) | 222 (2.3) | 0.92 (0.76, 1.11) | 0.38 |
| Cardiovascular death | 240 (2.5) | 271 (2.9) | 0.88 (0.74, 1.05) | 0.15 |
| All-cause death | 334 (3.5) | 392 (4.1) | 0.85 (0.73, 0.98) | 0.026 |

| Other end points | | | | |
|---|---|---|---|---|
| Non-fatal myocardial infarction | 626 (6.6) | 722 (7.6) | 0.86 (0.77, 0.96) | 0.006 |
| Fatal or non-fatal ischemic stroke | 111 (1.2) | 152 (1.6) | 0.73 (0.57, 0.93) | 0.01 |
| Unstable angina requiring hospitalization | 37 (0.4) | 60 (0.6) | 0.61 (0.41, 0.92) | 0.02 |
| Ischemia-driven coronary revascularization procedure | 731 (7.7) | 828 (8.8) | 0.88 (0.79, 0.97) | 0.009 |
| Congestive heart failure requiring hospitalization | 176 (1.9) | 179 (1.9) | 0.98 (0.79, 1.20) | 0.84 |

| | | | | |
|---|---|---|---|---|
| * Coronary heart disease death, non-fatal myocardial infarction, unstable angina requiring hospitalization, or ischemia-driven coronary revascularization procedure. † Coronary heart disease death or non-fatal myocardial infarction. ‡ Any non-fatal coronary heart disease event, any cardiovascular death, or non-fatal ischemic stroke. | | | | |

Alirocumab reduced the risk of any coronary heart disease event, major coronary heart disease event, any cardiovascular event, and the composite of death, non-fatal myocardial infarction and non-fatal stroke (**Table 2).** Although the reductions in coronary heart disease death or in cardiovascular death were not statistically significant, all-cause death occurred less frequently among patients in the alirocumab group (334 vs. 392 patients; estimated 4-year event rates, 5.3% vs. 6.4%; hazard ratio, 0.85; 95% confidence interval, 0.73 to 0.98; P=0.026; **Figure 3****).** As all-cause death followed coronary heart disease and cardiovascular death in the hierarchy of main secondary end points, the P-value for all-cause death is considered nominally significant. To prevent one death would require 87 patients to be treated for 4 years. Alirocumab also reduced the risk of non-fatal myocardial infarction, non-fatal ischemic stroke, hospitalization for unstable angina, and ischemia-driven coronary revascularization, but not hospitalization for congestive heart failure.

The effect of alirocumab on the primary endpoint was consistent across predefined subgroups **(****Figure 4****),** including three predefined subgroups of baseline LDL cholesterol (less than 80 mg per deciliter, 80 up to 100 mg per deciliter, and 100 mg per deciliter or greater; P-value for interaction, 0.09) (see **Figures 5A, 5B and 5C**). However, the cumulative incidence of the primary end point among patients assigned to placebo was 9.5%, 9.5%, and 14.9% in these three subgroups, respectively. Accordingly, in the subgroup with the highest baseline LDL cholesterol and the greatest absolute risk, alirocumab produced the greatest absolute risk reduction compared with placebo **(****Figure 5C****).**

Of particular interest, MACE was significantly reduced by 24% in these patients with baseline LDL cholesterol level over 100 mg/dL (HR=0.76 (95% confidence interval 0.65-0.87)), which was significantly different (p=0.05) from patients with a baseline LDL cholesterol level below 100 mg/dL **(****Figure 6****).** To prevent one primary endpoint in this subgroup would require 16 patients to be treated for 4 years. Corresponding levels of LDL-C are shown for each of the three predefined subgroups on an intention-to-treat (ITT) basis in **Figures 7A, 7B and 7C****.**

In a post hoc analysis across the three predefined subgroups of baseline LDL cholesterol, there was no significant heterogeneity of effect of alirocumab on death (P-value for ARR interaction, 0.12) (see **Figures 8A, 8B and 8C**). Similar to the primary end point, the absolute risk of death in the placebo group and the absolute difference in death between alirocumab and placebo groups were greatest in the subgroup with baseline LDL cholesterol of at least 100 mg per deciliter. The risk of death was significantly reduced by 29% in patients with a baseline LDL-C level at or above 100 mg/dL (HR=0.71 (95% confidence interval 0.56-0.90)). **(Table 3A).**

No significant difference in risk reduction was seen in patients analyzed by age (<65 v. ≥ 65), sex (male v. female), region (Eastern Europe, Western Europe, North America, South America, Asia, and Rest of World), or time from randomization.

**Tables 3A and 3B** focus on patients with a baseline LDL-C ≥ 100 mg/dL. **Table 3A** shows further analyses in the subpopulation of patients who had baseline LDL-C levels at or above 100 mg/dL. In this subgroup of patients, alirocumab significantly reduced the risk of major cardiovascular events by 24%, and additional post hoc analyses also found alirocumab reduced death from coronary heart disease (CHD) by 28% and death from any cause by 29%. Risk reduction was also significant in the secondary endpoint of CHD event, major CHD event, death, non-fatal myocardial infarction, or non-fatal ischemic stroke, and cardiovascular death. Similar results were observed in a pre-specified analysis of subgroups according to baseline LDL cholesterol levels of <80 mg/d, 80 to <100 mg/dL or ≥100 mg/dL (median baseline LDL-C: 118 mg/dL) **(Table 3B).**

**Table 3A. Efficacy: Subgroup with baseline LDL-C ≥ 100 mg/dL (median baseline LDL-C: 118 mg/dL)**

| **Endpoint, n (%)** | **Alirocumab (N=2814)** | **Placebo (N=2815)** | **Absolute Risk Reduction (%)** | **HR (95% CI)** |
|---|---|---|---|---|
| MACE | 324 (11.5) | 420 (14.9) | 3.4 | 0.76 (0.65-0.87) |
| CHD death | 69 (2.5) | 96 (3.4) | 1.0 | 0.72 (0.53-0.98) |
| CV death | 81 (2.9) | 117 (4.2) | 1.3 | 0.69 (0.52-0.92) |
| All-cause death | 114 (4.1) | 161 (5.7) | 1.7 | 0.71 (0.56-0.90) |

**Table 3B. Efficacy: Subgroups according to baseline LDL cholesterol levels of <80 mg/dL, 80 to <100 mg/dL or ≥100 mg/dL (median baseline LDL-C: 118 mg/dL)**

| **Outcome by Baseline LDL Cholesterol Category** | | **Alirocumab** | **Placebo** | **Hazard Ratio (95% CI)** | **Interaction P Value for Relative Risk Reduction** | **Absolute Risk Reductio n % (95% CI)** | **Interaction P Value for Absolute Risk Reduction** |
|---|---|---|---|---|---|---|---|
| | | *no. of patients (%)* | | | | | |
| Primary end point | | | | | 0.09 | | <0.001 |
| | <80 mg/dl | 296 (8.3) | 341 (9.5) | 0.86 (0.74 to 1.01) | | 1.3 (-0.1 to 2.6) | |
| | 80 to <100 mg/dl | 283 (9.2) | 291 (9.5) | 0.96 (0.82 to 1.14) | | 0.3 (-1.2 to 1.8) | |
| | ≥100 mg/dl | 324 (11.5) | 420 (14.9) | 0.76 (0.65 to 0.87) | | 3.4 (1.6 to 5.2) | |
| Any coronary heart disease event | | | | | 0.09 | | <0.001 |
| | <80 mg/dl | 421 (11.8) | 454 (12.7) | 0.93 (0.81 to 1.06) | | 0.9 (-0.6 to 2.4) | |
| | 80 to <100 mg/dl | 374 (12.2) | 387 (12.6) | 0.95 (0.83 to 1.10) | | 0.4 (-1.2 to 2.1) | |
| | ≥100 mg/dl | 404 (14.4) | 508 (18.0) | 0.78 (0.69 to 0.89) | | 3.7 (1.8 to 5.6) | |
| Major coronary heart disease event | | | | | 0.09 | | 0.002 |
| | <80 mg/dl | 257 (7.2) | 286 (8.0) | 0.90 (0.76 to 1.06) | | 0.8 (-0.4 to 2.0) | |
| | 80 to <100 mg/dl | 251 (8.2) | 249 (8.1) | 1.00 (0.84 to 1.19) | | -0.1 (-1.4 to 1.3) | |
| | ≥100 mg/dl | 285 (10.1) | 364 (12.9) | 0.77 (0.66 to 0.90) | | 2.8 (1.1 to 4.5) | |
| Any cardiovascular event | | | | | 0.11 | | <0.001 |
| | <80 mg/dl | 454 (12.7) | 493 (13.8) | 0.92 (0.81 to 1.04) | | 1.1 (-0.5 to 2.7) | |
| | 80 to <100 mg/dl | 406 (13.2) | 429 (14.0) | 0.93 (0.82 to 1.07) | | 0.8 (-1.0 to 2.5) | |
| | ≥100 mg/dl | 441 (15.7) | 552 (19.6) | 0.78 (0.69 to 0.89) | | 3.9 (2.0 to 5.9) | |
| Death, non-fatal myocardial infarction, or non-fatal ischemic stroke | | | | | 0.11 | | <0.001 |
| | <80 mg/dl | 318 (8.9) | 364 (10.2) | 0.87 (0.75 to 1.01) | | 1.3 (-0.1 to 2.6) | |
| | 80 to <100 mg/dl | 306 (10.0) | 316 (10.3) | 0.96 (0.82 to 1.12) | | 0.3 (-1.2 to 1.9) | |
| | ≥100 mg/dl | 349 (12.4) | 446 (15.8) | 0.77 (0.67 to 0.88) | | 3.4 (1.6 to 5.3) | |

| Coronary heart disease death | | | | | | | |
|---|---|---|---|---|---|---|---|
| | <80 mg/dl | 74 (2.1) | 73 (2.0) | 1.00 (0.73 to 1.39) | | 0 (-0.7 to 0.6) | |
| | 80 to <100 mg/dl | 62 (2.0) | 53 (1.7) | 1.17 (0.81 to 1.68) | | -0.3 (-1.0 to 0.4) | |
| | ≥100 mg/dl | 69 (2.5) | 96 (3.4) | 0.72 (0.53 to 0.98) | | 1.0 (0.1 to 1.8) | |

| Cardiovascular death | | | | | | | |
|---|---|---|---|---|---|---|---|
| | <80 mg/dl | 85 (2.4) | 88 (2.5) | 0.96 (0.71 to 1.29) | | 0.1 (-0.6 to 0.8) | |
| | 80 to <100 mg/dl | 74 (2.4) | 66 (2.2) | 1.12 (0.80 to 1.56) | | -0.3 (-1.0 to 0.5) | |
| | ≥100 mg/dl | 81 (2.9) | 117 (4.2) | 0.69 (0.52 to 0.92) | | 1.3 (0.3 to 2.2) | |

| All-cause death | | | | | | | |
|---|---|---|---|---|---|---|---|
| | <80 mg/dl | 118 (3.3) | 132 (3.7) | 0.89 (0.69 to 1.14) | | 0.4 (-0.5 to 1.2) | |
| | 80 to <100 mg/dl | 102 (3.3) | 99 (3.2) | 1.03 (0.78 to 1.36) | | -0.1 (-1.0 to 0.9) | |
| | ≥100 mg/dl | 114 (4.1) | 161 (5.7) | 0.71 (0.56 to 0.90) | | 1.7 (0.5 to 2.8) | |

### SAFETY

### a. Adverse Events

Among patients treated with alirocumab, compared to those treated with placebo, there was no excess of adverse events or laboratory abnormalities **(Table 4),** except for injection site reactions (3.8% vs. 2.1%, respectively). Neurocognitive events occurred in 1.5% of patients on alirocumab and 1.8% on placebo, new-onset diabetes in 9.6% versus 10.1%, respectively, and hemorrhagic stroke (confirmed by adjudication) in <0.1% versus 0.2%. Neutralizing antidrug antibodies were detected in 0.5% of patients on alirocumab and in <0.1% on placebo.

### b. Very Low LDL-C

The safety and efficacy of alirocumab was analyzed in patients who reached very low LDL-C, compared with matched patients from the placebo group.

Of 9462 patients randomized to receive alirocumab, 730 (7.7%) reached very low LDL-C (<15 mg/dL or 0.39 mmol/L) at two consecutive measurements and had substitution of placebo at median 8.3 months after randomization. Using propensity score matching, they were compared (3:1) with 2152 patients initially assigned to placebo. Propensity score matching was also used to compare new onset diabetes in 525 patients without diabetes at baseline who had two consecutive very low LDL-C levels and substitution of placebo with 1675 matched patients in the placebo group. Neurocognitive events and hemorrhagic stroke were also evaluated in relation to very low LDL-C.

Characteristics associated with very low LDL-C and used in propensity score matching included male sex, diabetes, lower baseline LDL-C and lipoprotein(a), and enrollment in Asia. Despite being switched to placebo, patients with very low LDL-C on alirocumab had lower incidence of MACE than matched patients from the placebo group (6.4% vs 8.5%, HR 0.71, 95% CI 0.52-0.98, P=0.039). Very low LDL-C on alirocumab was not associated with a higher risk of new onset diabetes, compared with matched patients from the placebo group (15.1% vs 13.0%, HR 1.10, 95% CI 0.85-1.43, P=0.46). There was also no identified association of very low LDL-C with neurocognitive events or hemorrhagic stroke.

**Table 4. Adverse Events and Laboratory Test Results**

| **Outcome** | **Alirocumab (N=9451)** | **Placebo (N=9443)** |
|---|---|---|
| Adverse events - no. of patients (%) | | |
| Any | 7165 (75.8) | 7282 (77.1) |
| Serious | 2202 (23.3) | 2350 (24.9) |
| Leading to death | 181 (1.9) | 222 (2.4) |
| Leading to treatment discontinuation | 343 (3.6) | 324 (3.4) |
| Local injection site reaction | 360 (3.8) | 203 (2.1) |
| General allergic reaction | 748 (7.9) | 736 (7.8) |
| Diabetes worsening or diabetic complication (in patients with diabetes at baseline) - no./total no. of patients (%) | 506/2688 (18.8) | 583/2747 (21.2) |
| New-onset diabetes* (in patients without diabetes at baseline) - no./total no. of patients (%) | 648/6763 (9.6) | 676/6696 (10.1) |
| Neurocognitive disorder | 143 (1.5) | 167 (1.8) |
| Hepatic disorder | 500 (5.3) | 534 (5.7) |
| Cataracts | 120 (1.3) | 134 (1.4) |
| Hemorrhagic stroke (adjudicated) | 9 (<0.1) | 16 (0.2) |

| Laboratory abnormalities at any time - no./total no. of patients (%) | | |
|---|---|---|
| Alanine transaminase >3 times upper limit of normal range | 212/9369 (2.3) | 228/9341 (2.4) |
| Aspartate aminotransferase > 3 times upper limit of normal | 160/9367 (1.7) | 166/9338 (1.8) |
| Total bilirubin > 2 times upper limit of normal | 61/9368 (0.7) | 78/9341 (0.8) |
| Creatine kinase >10 times upper limit of normal | 46/9369 (0.5) | 48/9338 (0.5) |
| Antidrug antibodies | 67/9091 (0.7) | 32/9097 (0.4) |
| Neutralizing antidrug antibodies | 43/9091 (0.5) | 6/9097 (<0.1) |

| | | |
|---|---|---|
| * Defined by one or more of the following, with confirmation of the diagnosis by blinded external review by experts in diabetology: adverse event report, new prescription of diabetes medication, hemoglobin A1c at least 6.5% on two occasions (and baseline level <6.5%), fasting blood glucose at least 126 mg per deciliter on two occasions (and baseline level <126 mg per deciliter). | | |

### CONCLUSIONS

In summary, ODYSSEY OUTCOMES met its primary endpoint as powered (15% relative risk reduction of major CV events / MACE). A consistent benefit was achieved in all individual endpoints (significant for MI, ischemic stroke, unstable angina requiring hospitalization, and ischemia-driven coronary revascularization, and with a positive trend for CHD and CV death). This was the first non-statin trial to demonstrate a nominally significant benefit on all-cause mortality. For patients with LDL> 100 mg/dL, all MACE endpoints were improved significantly including for CHD death, CV death and all-cause mortality. With up to 5 years of double-blinded follow-up period, no imbalance was observed in overall safety and safety events of interest between the alirocumab and the placebo groups (including new onset diabetes, cataract, neurocognitive events, and hemorrhagic stroke).

Compared with placebo in post-ACS patients, alirocumab 75 or 150 mg Q2W targeting LDL-C levels 25-50 mg/dL, and allowing levels as low as 15 mg/dL: (1) reduced MACE, MI and ischemic stroke; (2) was associated with a lower rate of all-cause death; and (3) was safe and well-tolerated over the duration of the trial. Specifically, in this nearly 19,000-patient placebo-controlled trial, including more than 6000 patients treated for ≥ 3 years, there was no safety signal with alirocumab.

Among patients with ACS and baseline LDL-C ≥ 100 mg/dL, alirocumab reduced MACE by 24% (ARR: 3.4%) and all-cause death by 29% (ARR: 1.7%) compared with placebo. Without intending to be bound by scientific theory, these are the patients who may benefit most from treatment.

### Example 4: Relationship of Baseline Lp(a) to Risk of MACE and Death

An elevated lipoprotein(a) (Lp(a)) level is an independent risk factor for cardiovascular disease. Lp(a) is a pro-atherosclerotic and pro-thrombotic/anti-fibrinolytic lipid, and is not reduced by statins or ezetimibe. Efforts to show the potential benefits of lowering Lp(a) have been unsuccessful in the past 30 years. This example demonstrates the specific benefit of alirocumab in lowering of Lp(a) in the ODYSSEY OUTCOMES clinical study.

### a. Methods

Lp(a) was measured at baseline, 4 months from the initial alirocumab or placebo administration, 12 months from the initial alirocumab or placebo administration, and at the end of study by automated latex nephelometry (Siemens Healthcare Diagnostics, Deerfield, IL). The size heterogeneity of Apolipoprotein(a) had only a moderate effect on Lp(a) recovery in the assay. The inter-assay coefficient of variation was 3.1% to 4.8% depending on Lp(a) concentration. Distributions of Lp(a) and LDL-C levels were assessed for the overall population and separately in the alirocumab and placebo groups at baseline and at Months 4 and 12 (±4 weeks) after randomization. If a patient had multiple values within each of these visit windows, the last value within the window was analyzed.

Calculated or directly measured LDL-C levels include cholesterol contained in Lp(a), which corresponds to approximately 30% of Lp(a) mass. To account for this and derive an estimate of cholesterol contained in LDL particles, LDL-C_{corrected} was calculated as LDL-C - 0.3×[Lp(a)]. Similarly, to derive an estimate of cholesterol carried in all apo-B containing particles other than Lp(a), corrected non-HDL cholesterol was calculated using the relationship, non-HDL C_{corrected} = (total cholesterol - HDL-C) - 0.3x[Lp(a)].

It was determined whether there was heterogeneity in the relative and absolute effects of alirocumab treatment on MACE according to quartile of baseline Lp(a). To assess the former, a Cox proportional hazard model stratified by geographical region with terms for baseline Lp(a) quartile, treatment, and their interaction was constructed. To assess the latter, absolute risk reductions with alirocumab treatment, quantified as differences in observed incidences in the two treatment groups, were compared across quartiles of baseline Lp(a) using a Gail-Simon test. Influence of treatment on absolute reduction of the risk of MACE was also assessed according to categories of Lp(a) (<median, ≥median) and baseline LDL-C (<100 mg/dL, ≥100 mg/dL [<2.59 mmolL, ≥2.59 mm/L]).

### b. Baseline Characteristics

Among the 18,924 patients, the mean (SD) of baseline Lp(a) level was 39.0 (43.2) mg/dL. The distribution of baseline Lp(a) was highly skewed with a median of 21.2 mg/dL (interquartile range 6.7 to 59.6 mg/dL) **(****Figure 9****).** The minimum level was 1 mg/dL, the 90th percentile level was 101 mg/dL, and the maximum level was 361 mg/dL. Baseline characteristics according to Lp(a) quartile were compared by chi-square tests (categorical variables) or rank-based tests (continuous variables). The demographics and clinical characteristics by baseline Lp(a) are shown in **Tables 5 and 6,** respectively. Participants in the upper Lp(a) quartiles were more likely to be female, black, and from North America, but less likely to smoke or have diabetes. LDL-C concentration and the percentage of patients treated with high-intensity statin were greatest in the highest quartile of Lp(a). Conversely, LDL-C_{corrected} and non-HDL-C_{corrected} decreased across increasing quartiles of Lp(a). Participants in the higher Lp(a) quartiles were more likely to have had blinded up-titration of alirocumab and less likely to have had blinded substitution of placebo for alirocumab.

**Table 5. Demographics by Baseline Lp(a) Quartile**

| **Variable** | **Q1: <6.7 mg/dL (n=4730)** | **Q2: 6.7 to <21.2 mg/dL (n=4731)** | **Q3: 21.2 to <59.6 mg/dL (n=4729)** | **Q4: ≥59.6 mg/dL (n=4734)** | **P-value*** |
|---|---|---|---|---|---|
| Age, years | 58 (52-65) | 58 (52-65) | 58 (52-65) | 58 (52-65) | 0.14 |
| Female | 20.2% | 24.2% | 24.5% | 31.8% | <0.0001 |
| Race | | | | | <0.0001 |
| White | 84.3% | 79.8% | 75.3% | 78.2% | |
| Black or African American | 0.6% | 1.0% | 3.1% | 5.2% | |
| Asian | 10.3% | 14.3% | 16.7% | 11.5% | |
| Other | 4.9% | 4.8% | 4.9% | 5.1% | |
| Region | | | | | <0.0001 |
| Eastern Europe | 35.9% | 30.7% | 25.3% | 23.0% | |
| Western Europe | 21.2% | 20.9% | 22.9% | 23.3% | |
| North America | 12.5% | 13.1% | 14.5% | 20.6% | |
| South America | 14.3% | 13.5% | 12.4% | 14.5% | |
| Asia | 9.7% | 13.3% | 15.3% | 10.3% | |
| Rest of world | 6.6% | 8.5% | 9.6% | 8.3% | |
| * The p-value indicates whether there is a significant difference in the values of the 4 quartiles. | | | | | |

**Table 6. Clinical Characteristics by Baseline Lp(a) Quartile**

| ***Variable*** | ***Q1: <6.7 mg*/*dL (n=4730)*** | ***Q2:* 6.7 to *<21.2 mg*/*dL (n=4731)*** | ***Q3: 21.2 to <59.6 mg*/*dL (n=4729)*** | ***Q4: ≥59.6 mg*/*dL (n=4734)*** | ***P-value**** |
|---|---|---|---|---|---|
| Time from ACS to randomization, months | 2.6 (1.7-4.2) | 2.6 (1.7-4.4) | 2.6 (1.7-4.3) | 2.6 (1.7-4.4) | 0.41 |
| ACS Type | | | | | 0.0079 |
| NSTEMI | 47.8% | 47.4% | 48.4% | 50.8% | |
| STEMI | 35.2% | 36.2% | 34.3% | 32.7% | |
| Unstable angina | 17.1% | 16.4% | 17.4% | 16.5% | |
| Revasc. for index event | 70.7% | 72.4% | 72.6% | 73.4% | 0.02 |
| Medications | | | | | |
| High-intensity statin | 88.3% | 87.4% | 88.7% | 91.0% | <0.0001 |
| Aspirin | 95.3% | 95.5% | 95.7% | 95.8% | 0.56 |
| P2Y12 antagonist | 86.3% | 87.6% | 87.6% | 88.2% | 0.04 |
| ACE inhibitor/ARB | 79.0% | 78.2% | 77.0% | 76.9% | 0.04 |
| Beta-blocker | 85.2% | 84.6% | 83.6% | 84.6% | 0.18 |
| Lp(a), mg/dL | 2.0 (2.0-4.8) | 12.2 (9.3-15.9) | 37.6 (28.3-47.7) | 92.2 (73.2-119.0) | |
| Total cholesterol, mg/dL | 159 (141-183) | 158 (141-180) | 158 (140-181) | 164 (146-186) | <0.001 |
| HDL-C, mg/dL | 42 (36-50) | 42 (36-50) | 42 (36-50) | 43 (37-51) | <0.001 |
| LDL-C, mg/dL | 83 (69-101) | 85 (72-102) | 86 (73-104) | 92 (78-109) | <0.001 |
| LDL-C_{corrected}, mg/dL | 82 (69-100) | 81 (68-98) | 75 (61-92) | 62 (48-79) | <0.001 |
| Non-HDL-C, mg/dL | 115 (99-138) | 113 (98-135) | 113 (98-135) | 119 (103-139) | <0.001 |
| Triglycerides, mg/dL | 144 (103-205) | 129 (95-183) | 123 (90-171) | 123 (91-169) | <0.001 |
| ApoB, mg/dL | 79 (68-93) | 78 (68-92) | 78 (68-92) | 82 (71-95) | <0.001 |
| BMI (kg/m²) | 28.5 (25.7-31.6) | 27.9 (25.2-30.9) | 27.7 (24.9-30.8) | 27.7 (25.0-31.0) | <0.001 |
| Diabetes | 30.7% | 29.0% | 29.0% | 26.5% | 0.001 |
| Current smoking | 26.0% | 25.2% | 23.7% | 21.5% | <0.001 |
| Hypertension | 66.2% | 64.2% | 63.2% | 65.2% | 0.0184 |
| History of MI | 18.7% | 17.8% | 18.8% | 21.5% | <0.0001 |
| History of stroke | 3.2% | 3.1% | 3.4% | 3.3% | 0.92 |
| Family History of premature CHD | 34.9% | 34.0% | 34.7% | 39.6% | <0.0001 |
| **Treatment Variables Among Alirocumab Treated Patients** | (n=2327) | (n=2438) | (n=2356) | (n=2341) | |
| % time treated with each dose | | | | | <0.001 |
| 75 mg | 81.4% | 82.3% | 79.3% | 68.6% | |
| 150 mg | 18.6% | 17.7% | 20.7% | 31.4% | |
| % switched to placebo | 12.2% | 9.7% | 7.0% | 1.9% | <0.001 |
| ** The p-value indicates whether there is a significant difference in the values of the 4 quartiles.* | | | | | |

### c. Baseline Lp(a), Cardiovascular Events, and Mortality in the Placebo Group

Incidences of MACE, CHD death, and non-fatal myocardial infarction (MI) were compared across four baseline Lp(a) categories divided according to quartiles using a Cox regression model with baseline Lp(a). Adjustment for age, sex, race, geographic region, time since index event, BMI, smoking history, diabetes, baseline LDL-C, and/or treatment assignment was performed. The relationship between baseline Lp(a) quartile and incidence of events in the placebo group is shown in **Figure 10****;** and modeling of this relationship is shown in **Table 7** below.

**Table 7. Baseline Lp(a) Quartile and Cardiovascular Events and Mortality in Placebo Group**

| | **Unadjusted Model** | | | | **Adjusted Model** | | | |
|---|---|---|---|---|---|---|---|---|
| **Endpoint** | **Q2 vs. Q1 HR (95% CI)** | **Q3 vs. Q1 HR (95% CI)** | **Q4 vs. Q1 HR (95% CI)** | **Linear Trend p-value** | **Q2 vs. Q1 HR (95% CI)** | **Q3 vs. Q1 HR (95% CI)** | **Q4 vs. Q1 HR (95% CI)** | **Linear Trend p-value** |
| MACE | 1.12 (0.93, 1.34) | 1.22 (1.03, 1.46) | 1.46 (1.23, 1.73) | <0.0001 | 1.13 (0.94, 1.36) | 1.29 (1.08, 1.54) | 1.68 (1.41, 2.00) | <0.0001 |
| Non-fatal MI | 1.11 (0.89, 1.38) | 1.25 (1.01, 1.55) | 1.58 (1.29, 1.94) | <0.0001 | 1.12 (0.90, 1.40) | 1.31 (1.05, 1.62) | 1.80 (1.46, 2.22) | <0.0001 |
| Ischemic Stroke | 0.93 (0.58, 1.47) | 1.06 (0.68, 1.64) | 1.03 (0.66, 1.61) | 0.7506 | 0.94 (0.60,1.50) | 1.14 (0.73, 1.77) | 1.26 (0.80, 1.99) | 0.2333 |
| CHD Death | 1.13 0.77, 1.65) | 1.04 (0.71, 1.52) | 1.27 (0.88, 1.83) | 0.2917 | 1.15 (0.79, 1.68) | 1.10 (0.75, 1.62) | 1.50 (1.03, 2.18) | 0.0571 |
| CV Death | 1.04 (0.73, 1.47) | 1.20 (0.86, 1.68) | 1.17 (0.83, 1.64) | 0.2659 | 1.06 (0.74, 1.50) | 1.27 (0.91, 1.78) | 1.37 (0.97, 1.94) | 0.0418 |
| All-Cause Death | 0.88 (0.67, 1.17) | 1.02 (0.78, 1.33) | 0.90 (0.68, 1.20) | 0.7261 | 0.89 (0.67, 1.18) | 1.06 (0.81, 1.39) | 1.02 (0.77, 1.36) | 0.6079 |

| | | | | | | | | |
|---|---|---|---|---|---|---|---|---|
| *Baseline Lp(a) quartile is the referent quartile. The adjusted model is adjusted for baseline corrected LDL-cholesterol.* | | | | | | | | |

As shown in **Table 8,** significantly higher incidences of MACE, CHD death, and non-fatal MI were observed with increasing baseline Lp(a) level. In unadjusted Cox models, individuals in the highest, compared to the lowest Lp(a) baseline quartile were 46-58% more likely to experience MACE or non-fatal myocardial infarction. The relationship between baseline quartile of Lp(a) and MACE, non-fatal myocardial infarction, and cardiovascular death was strengthened after adjustment for baseline LDL-C_{corrected}. Spline analysis of continuous baseline Lp(a) and the hazard ratio for MACE (Figure 11) indicated a relatively linear relationship between baseline Lp(a) and MACE. The interaction p-values for MACE, non-fatal MI, and CHD death with alirocumab treatment in the adjusted model were 0.44, 0.19, and 0.52, respectively. The interaction p-values for MACE, non-fatal MI, and CHD death with baseline LDL-C level in the adjusted model were 0.67, 0.25, and 0.92, respectively. No relationship was observed between baseline Lp(a) quartile and ischemic stroke, CHD death, or all-cause death.

**Table 8. Cardiovascular Events by Baseline Lp(a) Quartile**

| ***Cardiovascular Event*** | ***Q1:* <6.7 *mg*/*dL (n=4730)*** | ***Q2: 6.7* to *<21.2 mg*/*dL (n=4731)*** | ***Q3: 21.2 to <59.6 mg*/*dL (n=4729)*** | ***Q4: ≥59.6 mg*/*dL (n=4734)*** |
|---|---|---|---|---|
| MACE | | | | |
| Events n/N (%) | 439/4730 (9.3) | 458/4731 (9.7) | 481/4729 (10.2) | 577/4734 (12.2) |
| HR (95% CI) unadjusted | reference | 1.06 (0.93-1.21) | 1.12 (0.99-1.28) | 1.37 (1.21-1.55) |
| p-value for trend across quartiles, unadjusted | | p<0.0001 | | |
| HR (95% CI) adjusted* | | 1.05 (0.92-1.20) | 1.08 (0.95-1.23) | 1.28 (1.13-1.46) |
| p-value for trend across quartiles, adjusted* | | p=0.0005 | | |
| Non-fatal MI | | | | |
| Events n/N (%) | 305/4730 (6.5) | 306/4731 (6.5) | 326/4729 (6.9) | 411/4734 (8.7) |
| HR (95% CI) unadjusted | reference | 1.02 (0.87-1.19) | 1.09 (0.94-1.28) | 1.39 (1.20-1.62) |
| p-value for trend across quartiles, unadjusted | | p<0.0001 | | |
| HR (95% CI) adjusted* | | 1.00(0.85-1.17) | 1.04 (0.89-1.22) | 1.26 (1.08-1.46) |
| p-value for trend across quartiles, adjusted* | | p=0.0052 | | |
| CHD death | | | | |
| Events n/N (%) | 90/4730 (1.9) | 104/4731 (2.2) | 109/4729 (2.3) | 124/4734 (2.6) |
| HR (95% CI) unadjusted | reference | 1.18 (0.89-1.56) | 1.24 (0.94-1.65) | 1.43 (1.09-1.88) |
| p-value for trend across quartiles, unadjusted | | p=0.07 | | |
| HR (95% Cl) adjusted* | | 1.18 (0.89-1.57) | 1.23 (0.93-1.64) | 1.46 (1.10-1.93) |
| p-value for trend across quartiles, adjusted* | | p=0.07 | | |
| *Adjusted for age, sex, race, geographic region, time since index event, BMI, smoking history, diabetes, baseline LDL-C, and treatment assignment. | | | | |

### d. Effect of alirocumab on MACE stratified by baseline Lp(a) quartile

The relative incidences of MACE, non-fatal MI, CHD death, and all-cause death in the alirocumab-treated group relative to the placebo-treated group were compared across the four baseline Lp(a) quartiles. Adjustment for age, sex, race, geographic region, time since index event, BMI, smoking history, diabetes, and baseline LDL-C was performed. Relative and absolute treatment effects on MACE stratified by baseline Lp(a) quartile are shown in Figure 12. As shown in Table 9, greater reductions in incidences of MACE and non-fatal MI by alirocumab treatment were observed with increasing baseline Lp(a) level. No relationship was observed between baseline Lp(a) quartile and the effect of alirocumab in reducing the incidences of CHD death or all-cause death.

Overall, the hazard ratio for MACE (alirocumab/placebo) was 0.85 (95% confidence interval [CI] 0.78 to 0.93, P<0.001), with an absolute risk reduction of 1.6%. There was no statistically significant interaction of treatment and baseline Lp(a) quartile on the relative risk of MACE. In contrast, absolute risk reduction with alirocumab was several-fold greater in the upper quartiles (2.3% and 2.1%) than in the lower quartiles of baseline Lp(a) (0.4% and 1.4%, interaction P-value 0.0011). The numbers of patients needed to treat with alirocumab for a median of 2.8 years to prevent one MACE were 250, 71, 43, and 47 in quartiles 1, 2, 3, and 4 of baseline Lp(a), respectively.

Patients with baseline LDL-C >100 mg/dL (>2.59 mmol/l), baseline Lp(a) > median (21.2 mg/dl), or both, were identified as patients who received benefit from alirocumab treatment **(****Figure 13****).**

**Table 9. Cardiovascular Events by Baseline Lp(a) Quartile**

| **Cardiovascular Event** | **Q1: <6.7 mg/dL (n=4730)** | **Q2: 6.7 to <21.2 mg/dL (n=4731)** | **Q3: 21.2 to <59.6 mg/dL (n=4729)** | **Q4: ≥59.6 mg/dL (n=4734)** |
|---|---|---|---|---|
| MACE | | | | |
| Treatment HR (95% CI) unadjusted | 0.95 (0.79-1.14) | 0.85 (0.71-1.03) | 0.79 (0.66-0.95) | 0.83 (0.70-0.98) |
| Treatment HR (95% CI) adjusted* | 0.97 (0.80-1.17) | 0.87 (0.72-1.04) | 0.79 (0.66-0.96) | 0.82 (0.70-0.97) |

| Non-fatal MI | | | | |
|---|---|---|---|---|
| Treatment HR (95% CI) unadjusted | 1.04 (0.83-1.30) | 0.88 (0.70-1.10) | 0.79 (0.63-0.98) | 0.80 (0.66-0.97) |
| Treatment HR (95% CI) adjusted* | 1.07 (0.85-1.34) | 0.89 (0.71-1.01) | 0.80 (0.64-0.99) | 0.78 (0.64-0.95) |

| CHD death | | | | |
|---|---|---|---|---|
| Treatment HR (95% CI) unadjusted | 0.75 (0.50-1.14) | 0.83 (0.57-1.23) | 1.11 (0.76-1.61) | 0.99 (0.69-1.40) |
| Treatment HR (95% CI) adjusted* | 0.76 (0.50-1.16) | 0.85 (0.58-1.26) | 1.13 (0.77-1.65) | 1.05 (0.77-1.50) |

| All-cause death | | | | |
|---|---|---|---|---|
| Treatment HR (95% CI) unadjusted | 0.70 (0.52-0.94) | 0.94 (0.70-1.27) | 0.85 (0.64-1.13) | 0.93 (0.69-1.25) |
| Treatment HR (95% CI) adjusted* | 0.73 (0.54-0.98) | 0.97 (0.73-1.31) | 0.87 (0.65-1.15) | 0.95 (0.70-1.28) |

| | | | | |
|---|---|---|---|---|
| *Adjusted for age, sex, race, geographic region, time since index event, BMI, smoking history, diabetes, and baseline LDL-C. | | | | |

### e. Effect of alirocumab on Lp(a) levels

The Lp(a) levels in the patients over the course of the study were also analyzed. **Figure 14** shows median Lp(a) concentration by baseline quartile of Lp(a) and treatment group. Baseline distributions of Lp(a) were similar in both treatment groups. As shown in **Figures 18A and 18B****,** alirocumab significantly reduced the Lp(a) levels in the patients 4 months from the initial treatment, and the reduction was maintained until the end of the study. Further, absolute changes at Month 4 from baseline in LDL-C and Lp(a) were compared within the alirocumab group in the four baseline Lp(a) categories divided according to quartiles. **Figure 15** shows the absolute change from baseline to Month 4 in Lp(a), LDL-C and LDL-C_{corrected} in the alirocumab group **(****Figure 15A****)** and the placebo group **(****Figure 15B****).** The absolute Lp(a) change increased in magnitude across Lp(a) quartiles, whereas the absolute LDL-C change was constant across Lp(a) quartiles **(Table 10).** Therefore, no correlation was observed between LDL-C lowering and Lp(a) lowering in these patients.

Overall, the median relative and absolute change from baseline to Month 4 in the alirocumab group was -23% (IQR -47%, 0%) and -5.0 mg/dl (IQR -13.5, 0), respectively. Although the relative change in Lp(a) with alirocumab treatment was similar across baseline Lp(a) quartiles, there was a substantial gradient of median absolute change, ranging from 0 mg/dl in quartile 1 to -20.2 mg/dl in quartile 4. Change in LDL-C was similar in all Lp(a) quartiles; in contrast the change in LDL-C_{corrected} was smaller in the upper Lp(a) quartiles, with an overall median change of -51.1 mg/dl (IQR -67.2, - 33.7) **(****Figure 15A****).** Baseline Lp(a) was strongly correlated with the change from baseline to Month 4 in Lp(a) and weakly correlated with the change in corrected LDL-C and corrected non-HDL-C **(****Figure 16A, 16B****,** and **16C).** There were no systematic changes in Lp(a) levels over time in the placebo group **(****Figure 15B****).**

**Table 10. Changes of LDL-C and Lp(a) Levels Across Lp(a) Quartiles**

| **Alirocumab group** | **Q1: <6.7 mg/dL (n=4730)** | **Q2: 6.7 to <21.2 mg/dL (n=4731)** | **Q3: 21.2 to <59.6 mg/dL (n=4729)** | **Q4: ≥59.6 mg/dL (n=4734)** |
|---|---|---|---|---|
| Absolute change* | | | | |
| LDL-C (mg/dL) | -53.7 (-35.5, -69.0) | -54.1 (-37.1, -71.0) | -53.3 (-36.3, -70.3) | -54.1 (-36.7, -71.0) |
| Lp(a) (mg/dL) | 0 (0, -1.42) | -5.12 (-2.25, -7.86) | -9.8 (-3.18, -16.2) | -20.2 (-8.0, -34.3) |

| | | | | |
|---|---|---|---|---|
| *Values in table are median (interquartile range); change between baseline and Month 4. | | | | |

### f. Effect of Lp(a) change on outcomes

To determine the association between modification of Lp(a) levels by alirocumab treatment and endpoint events, the relationships between change in Lp(a) from baseline to Month 4 and risk of MACE and secondary endpoints after Month 4 were described with Cox proportional hazard regression models among patients in the alirocumab group. Four models were developed for each outcome of interest: a model without covariates (unadjusted model; Model 1), a model adjusted for baseline Lp(a) (Model 2); a model additionally adjusted for either baseline LDL C_{corrected} and change from baseline to Month 4 in LDL C_{corrected} (Model 3A) or baseline non-HDL C_{corrected} and change from baseline to Month 4 in non-HDL C_{corrected} (Model 3B); and a model adjusted for all variables in Model 3 as well as the demographic and clinical variables listed above with either LDL C_{corrected} (Model 4A) or non-HDL C_{corrected} (Model 4B). This fourth model thus shows the effect of change in Lp(a) on the outcome of interest by treatment with alirocumab that is independent of the effect of alirocumab on change in LDL-C_{corrected} and non-HDL C_{corrected}.

A comparison of Models 2 and 3A indicates whether the relationship between the change in Lp(a) and MACE is modified by adjustment for the simultaneous change in LDL C_{corrected}. Similarly, a comparison of Models 2 and 3B indicates whether the relationship between the change in Lp(a) and MACE is modified by adjustment for the simultaneous change in all other apoB-containing lipoproteins. Effects were summarized by hazard ratios per 1 mg/dl reduction in Lp(a) (all models) and in LDL C_{corrected} or non-HDL C_{corrected} (Models 3A, 3B, 4A, and 4B) at Month 4. The estimated reduction in risk of MACE with alirocumab attributable to lowering of Lp(a) and to simultaneous lowering of LDL C_{corrected} (Model 3A) or non-HDL C_{corrected} (Model 3B) was calculated across the distribution of baseline Lp(a). **Table 11** shows the results of sequential Cox proportional hazards models relating the change in Lp(a) under alirocumab treatment to the risk of MACE, with concurrent adjustment for corrected LDL -C or corrected non-HDL-C.

**Table 11. Relationship of Lp(a) under alirocumab treatment to the risk of MACE**

| **Model** | **Model Adjustments** | **Change Parameter** | **HR (95% CI) per 1 mg/dl decrease** | **p-value** |
|---|---|---|---|---|
| 1 | None | Lp(a) | 0.998 (0.993, 1.002) | 0.2730 |
| 2 | Baseline Lp(a) | Lp(a) | 0.993 (0.989, 0.998) | 0.0027 |
| 3A | Baseline Lp(a), baseline LDL-C_{corrected}, change from baseline to Month 4 in LDL-C_{corrected} | Lp(a) | 0.994 (0.990, 0.999) | 0.0081 |
| | | LDL-Ccorrected | 0.996 (0.994, 0.998) | 0.0002 |
| 3B | Baseline Lp(a), baseline non-HDL-C_{corrected}, change from baseline to Month 4 in non-HDL-C_{corrected} | Lp(a) | 0.994 (0.990, 0.998) | 0.0078 |
| | | Non-HDL-C_{corrected} | 0.997 (0.995, 0.998) | 0.0004 |
| 4A | Baseline Lp(a), baseline LDL-C_{corrected}, change from baseline to Month 4 in LDL-C_{corrected}, demographic and clinical characteristics | Lp(a) | 0.994 (0.990, 0.998) | 0.0071 |
| | | LDL-C_{corrected} | 0.995 (0.993, 0.997) | <0.0001 |
| 4B | Baseline Lp(a), baseline non-HDL-C_{corrected}, change from baseline to Month 4 in non-HDL-C_{corrected}, demographic and clinical characteristics | Lp(a) | 0.994 (0.990, 0.998) | 0.0064 |
| | | Non-HDL-C_{corrected} | 0.996 (0.994, 0.998) | 0.0001 |

**Table 12** shows the modeling for CHD death or nonfatal myocardial infarction, CV death and all cause death. In unadjusted models, no significant relationship was found between the change in Lp(a) and the risk of MACE (Model 1). After adjustment for baseline Lp(a), a significant relationship of the change in Lp(a) with MACE was apparent (Model 2). This is because higher baseline Lp(a) is associated with greater cardiovascular risk and greater reduction in Lp(a) on alirocumab treatment, and, therefore, accounting for the former exposes the relationship of the latter to the risk of MACE. Importantly, further adjustment for baseline and change from baseline to Month 4 in LDL-C_{corrected} or baseline and change from baseline to Month 4 in non-HDL-C_{corrected} did not attenuate the relationships (comparison of Models 2 and 3A and 3B, respectively). In models adjusted for LDL-C_{corrected} or non-HDL-C_{corrected} (Models 3A and 3B), a 1 mg/dl decrease in Lp(a) was associated with hazard ratios for MACE of 0.994 (95% CI 0.990-0.998) and 0.994 (95% CI 0.990-0.998), respectively. This indicates that reduction in Lp(a) under alirocumab treatment was associated with reduced risk of MACE, independent of the concurrent reduction of LDL-C_{corrected} or non-HDL-C_{corrected}. Further adjustment for demographic and clinical variables had minimal effects on the relationships (Models 4A and 4B).

**Table 12. Relationship of Changes in Lp(a) and LDL-C from Baseline to Month 4 to Outcomes After Month 4 in the alirocumab Group.**

| **Endpoint** | **Model** | **Model Adjustments** | **Change Parameter** | **HR (95% CI) per 1 mg/dl decrease** | **p-value** |
|---|---|---|---|---|---|
| CHD Death + non-fatal MI | | | | | |
| | 1 | None | Lp(a) | 0.998 (0.993, 1.002) | 0.3517 |
| | 2 | Baseline Lp(a) | Lp(a) | 0.993 (0.989, 0.998) | 0.0029 |
| | 3A | Baseline Lp(a), baseline LDL-C_{corrected}, change from baseline to Month 4 in LDL-C_{corrected} | Lp(a) | 0.994 (0.990, 0.998) | 0.0086 |
| | | | LDL-C_{corrected} | 0.996 (0.993, 0.998) | 0.0004 |
| | 3B | Baseline Lp(a), baseline non-HDL-C_{corrected}, change from baseline to Month 4 in non-HDL-C_{corrected} | Lp(a) | 0.994 (0.990, 0.999) | 0.0087 |
| | | | Non-HDL-C_{corrected} | 0.996 (0.994, 0.998) | 0.0004 |
| | 4A | Baseline Lp(a), baseline LDL-C_{corrected}, change from baseline to Month 4 in LDL-C_{corrected}, demographic and clinical characteristics | Lp(a) | 0.994 (0.990, 0.999) | 0.0090 |
| | | | LDL-C_{corrected} | 0.995 (0.993, 0.997) | <0.0001 |
| | 4B | Baseline Lp(a), baseline non-HDL-C_{corrected}, change from baseline to Month 4 in non-HDL-C_{corrected}, demographic and clinical characteristics | Lp(a) | 0.994 (0.990, 0.999) | 0.0084 |
| | | | Non-HDL-C_{corrected} | 0.996 (0.994, 0.998) | 0.0001 |

| Ischemic Stroke | | | | | |
|---|---|---|---|---|---|
| | 1 | None | Lp(a) | 0.990 (0.979, 1.002) | 0.0905 |
| | 2 | Baseline Lp(a) | Lp(a) | 0.993 (0.979, 1.006) | 0.2833 |
| | 3A | Baseline Lp(a), baseline LDL-C_{corrected}, change from baseline to Month 4 in LDL-C_{corrected} | Lp(a) | 0.993 (0.980, 1.007) | 0.3455 |
| | | | LDL-C_{corrected} | 0.996 (0.990, 1.003) | 0.2536 |
| | 3B | Baseline Lp(a), baseline non-HDL-C_{corrected}, change from baseline to Month 4 in non-HDL-C_{corrected} | Lp(a) | 0.994 (0.980, 1.007) | 0.3556 |
| | | | Non-HDL-C_{corrected} | 0.996 (0.991, 1.002) | 0.1638 |
| | 4A | Baseline Lp(a), baseline LDL-C_{corrected}, change from baseline to Month 4 in LDL-C_{corrected}, demographic and clinical characteristics | Lp(a) | 0.993 (0.980, 1.005) | 0.2530 |
| | | | LDL-C_{corrected} | 0.996 (0.990, 1.002) | 0.2282 |
| | 4B | Baseline Lp(a), baseline non-HDL-C_{corrected}, change from baseline to Month 4 in non-HDL-C_{corrected}, demographic and clinical characteristics | Lp(a) | 0.993 (0.981, 1.005) | 0.2637 |
| | | | Non-HDL-C_{corrected} | 0.996 (0.991, 1.001) | 0.1463 |

| CV Death | | | | | |
|---|---|---|---|---|---|
| | 1 | None | Lp(a) | 1.000 (0.992, 1.008) | 0.9649 |
| | 2 | Baseline Lp(a) | Lp(a) | 0.997 (0.989, 1.005) | 0.4667 |
| | 3A | Baseline Lp(a), baseline LDL-C_{corrected}, change from baseline to Month 4 in LDL-C_{corrected} | Lp(a) | 0.998 (0.989, 1.006) | 0.5953 |
| | | | LDL-C_{corrected} | 0.995 (0.991, 0.999) | 0.0155 |
| | 3B | Baseline Lp(a), baseline non-HDL-C_{corrected}, change from baseline to Month 4 in non-HDL-C_{corrected} | Lp(a) | 0.998 (0.990, 1.006) | 0.6055 |
| | | | Non-HDL-C_{corrected} | 0.995 (0.992, 0.999) | 0.0062 |
| | 4A | Baseline Lp(a), baseline LDL-C_{corrected}, change from baseline to Month 4 in LDL-C_{corrected}, demographic and clinical characteristics | Lp(a) | 0.996 (0.988, 1.004) | 0.3316 |
| | | | LDL-C_{corrected} | 0.994 (0.990, 0.998) | 0.0038 |
| | 4B | Baseline Lp(a), baseline non-HDL-C_{corrected}, change from baseline to Month 4 in non-HDL-C_{corrected}, demographic and clinical characteristics | Lp(a) | 0.996 (0.989, 1.004) | 0.3508 |
| | | | Non-HDL-C_{corrected} | 0.994 (0.991, 0.998) | 0.0014 |

| All-Cause Death | | | | | |
|---|---|---|---|---|---|
| | 1 | None | Lp(a) | 0.998 (0.992, 1.005) | 0.6046 |
| | 2 | Baseline Lp(a) | Lp(a) | 0.997 (0.990, 1.005) | 0.4986 |
| | 3A | Baseline Lp(a), baseline LDL-C_{corrected}, change from baseline to Month 4 in LDL-C_{corrected} | Lp(a) | 0.998 (0.991, 1.006) | 0.6671 |
| | | | LDL-C_{corrected} | 0.995 (0.991, 0.998) | 0.0019 |
| | 3B | Baseline Lp(a), baseline non-HDL-C_{corrected}, change from baseline to Month 4 in non-HDL-C_{corrected} | Lp(a) | 0.998 (0.991, 1.006) | 0.6375 |
| | | | Non-HDL-C_{corrected} | 0.996 (0.993, 0.999) | 0.0056 |
| | 4A | Baseline Lp(a), baseline LDL-C_{corrected}, change from baseline to Month 4 in LDL-C_{corrected}, demographic and clinical characteristics | Lp(a) | 0.998 (0.991, 1.004) | 0.4959 |
| | | | LDL-C_{corrected} | 0.994 (0.990, 0.997) | 0.0002 |
| | 4B | Baseline Lp(a), baseline non-HDL-C_{corrected}, change from baseline to Month 4 in non-HDL-C_{corrected}, demographic and clinical characteristics | Lp(a) | 0.998 (0.991, 1.004) | 0.4771 |
| | | | Non-HDL-C_{corrected} | 0.995 (0.992, 0.998) | 0.0007 |

The magnitude of Lp(a) change under alirocumab treatment increased with baseline Lp(a) concentration. For example, patients at the 25^{th}, 50^{th}, and 75^{th} percentiles of the baseline Lp(a) distribution had expected changes in Lp(a) under alirocumab treatment of -1.6, -4.8, and -13.4 mg/dl. **Figure 17A** and **B** shows the relationship between absolute change in Lp(a) at month 4, adjusted for baseline Lp(a) and baseline and absolute change in LDL-C_{corrected} **(****Figure 17A****)** or baseline and absolute change in non-HDL-C_{corrected} **(****Figure 17B****).** In these models, the proportion of the combined relative risk reduction by change in Lp(a) and LDL-C_{corrected} that can be attributed to Lp(a), based on log hazard ratios, is 4%, 11%, and 27% at the 25th, 50th, and 75th percentile of baseline Lp(a), respectively. The corresponding proportion of combined relative risk reduction by change in Lp(a) in the non-HDL-C_{corrected} model is 4%, 12%, and 29% at the 25th, 50th, and 75th percentile of baseline Lp(a), respectively. Thus, among patients with low baseline Lp(a), reduction of Lp(a) with alirocumab contributes minimally to the reduction in MACE. In contrast, among patients with high baseline Lp(a), reduction of Lp(a) with alirocumab contributes substantially to the reduction of MACE, although the effect of reducing corrected LDL (or non-HDL)-C remains primary.

Accordingly, the magnitude of Lp(a) change under alirocumab treatment and its contribution to cardiovascular risk reduction increased with baseline Lp(a) concentration. For example, at the 25th, 50th, and 75th percentiles of baseline Lp(a), the hazard ratios for MACE associated with Lp(a) changes of -1.6, -4.8, and -13.4 mg/dl were 0.991, 0.973, and 0.928, respectively, and the proportion of the reduction in risk of MACE attributable to the change in Lp(a) were 4%, 10%, and 26%. Thus, among patients with higher baseline Lp(a), Lp(a) reduction with alirocumab becomes a prominent contributor to the reduction of MACE. These results suggest that the relationship between Lp(a) reduction and MACE or non-fatal MI was independent of LDL-C level. There was no relationship between time-weighted moving average of absolute Lp(a) change and ischemic stroke, CHD death, CV death, or all-cause death.

### g. Effect of Lp(a) change on total events

To investigate the contribution of Lp(a) reduction to the decrease in total events (non-fatal CV events and death) observed with alirocumab treatment, patients with ACS and LDL-C ≥70 mg/dL, non-HDL-C ≥100 mg/dL, or apoB ≥80 mg/dL on intensive or maximum tolerated statin therapy were randomized 1:1 to alirocumab treatment or placebo. The relationship between Lp(a) change from baseline to Month 4 and subsequent events was described in the alirocumab group by frailty models adjusted for demographic and clinical variables, baseline Lp(a) and LDL-C, and LDL-C change from baseline to Month 4. Effects were summarized by hazard ratios at baseline Lp(a) percentiles (see **Table 13).**

In the alirocumab group, there were 1,636 nonfatal events and 299 deaths. Of the combined effects of Lp(a) and LDL-C lowering, the proportions of risk reduction attributable to Lp(a) change were 5%, 13%, and 30% at the 25th, 50th, and 75th percentiles of baseline Lp(a), respectively. Thus, Lp(a) lowering by alirocumab contributed to a reduction in total events, especially among patients with higher baseline Lp(a) levels. Even after adjustment for LDL-C change, the Lp(a) decrease induced by alirocumab was associated with lower risk of total events.

**Table 13. Relationship of absolute change in Lp(a) at Month 4 to risk of Total Events**

| **Model Adjustments** | **Parameter** | **Baseline Lp(a) Percentile²** | **Parameter Change³** | **HR (95% CI)** | **p-value** |
|---|---|---|---|---|---|
| Baseline Lp(a) | Lp(a) | | | | 0.0109 |
| | | 25^{th} | 1.6 mg/dL decrease | 0.991 (0.984, 0.998) | |
| | | 50^{th} | 4.8 mg/dL decrease | 0.973 (0.952, 0.994) | |
| | | 75^{th} | 13.4 mg/dL decrease | 0.926 (0.872, 0.982) | |
| Baseline Lp(a), Demographic and Clinical Characteristics,¹ Baseline LDL-C | Lp(a) | | | | 0.0013 |
| | | 25^{th} | 1.6 mg/dL decrease | 0.988 (0.981, 0.995) | |
| | | 50^{th} | 4.8 mg/dL decrease | 0.966 (0.946, 0.986) | |
| | | 75^{th} | 13.4 mg/dL decrease | 0.907 (0.855, 0.963) | |
| Baseline Lp(a), Demographic and Clinical Chracteristics,¹ Baseline LDL-C, Change LDL-C | Lp(a) | | | | 0.0056 |
| | | 25^{th} | 1.6 mg/dL decrease | 0.990 (0.983, 0.997) | |
| | | 50^{th} | 4.8 mg/dL decrease | 0.970 (0.950, 0.991) | |
| | | 75^{th} | 13.4 mg/dL decrease | 0.920 (0.867, 0.976) | |
| | LDL-C | | | | 0.0005 |
| | | 25^{th} | 51.1 mg/dl decrease | 0.816 (0.728, 0.915) | |
| | | 50^{th} | 50.5 mg/dL decrease | 0.818 (0.731, 0.916) | |
| | | 75^{th} | 48.9 mg/dL decrease | 0.824 (0.738, 0.919) | |

| | | | | | |
|---|---|---|---|---|---|
| ¹ Age, sex, race, geographic region, body mass index, smoking history, diabetes, time from index ACS to randomization ² 25^{th} percentile = 6.7 mg/dL; 50^{th} percentile = 21.2 mg/dL; 75^{th} percentile = 59.6 mg/dL ³ Expected change in indicated parameter for a patient at the specified quartile of baseline Lp(a) | | | | | |

### CONCLUSION

Baseline Lp(a) level predicted MACE, CHD death, and non-fatal MI in both the alirocumab and the placebo groups, independent of baseline LDL-C level and demographic and clinical variables. Baseline Lp(a) did not predict death or ischemic stroke. Baseline Lp(a) was also associated with the benefit of alirocumab treatment on MACE and non-fatal MI. Therefore, baseline Lp(a) level was an independent predictor of MACE and non-fatal MI among patients with recent ACS, independent of treatment with alirocumab and baseline LDL-C.

Additionally, the median change from baseline in Lp(a) level was approximately -5 mg/dL with alirocumab and 0 mg/dL with placebo. In the alirocumab group, reduction in Lp(a) predicted reduction in MACE and non-fatal MI in models accounting for demographic and clinical variables, baseline LDL-C, and LDL-C change from baseline. No relationship was observed between Lp(a) reduction and stroke or mortality. In the placebo group, where there was no systematic change from baseline, change in Lp(a) did not predict any outcome event. In this study, patients with levels of Lp(a) at or above the cohort median (21 mg/dL) or with levels of LDL-C at or above 100 mg/dL derived a benefit from alirocumab treatment. Therefore, Lp(a) lowering by alirocumab was associated with a reduced risk of MACE, non-fatal MI, and a reduction in total events, independent of baseline LDL-C and concurrent LDL-C lowering.

### Example 5: Secondary Analysis: Effect of Alirocumab on Mortality and MACE after Acute Coronary Syndrome in Patients Treated for More than One Year

A group of patients of particular interest, according to a secondary (post hoc) analysis, are those who have been treated for longer than 12 months. As shown in **Table 14,** patients with a baseline LDL-C level at or above 100 mg/dL who received treatment of alirocumab for less than twelve months showed a reduction in MACE (HR = 0.81 (confidence interval 0.66-1.01)). When the same population was treated with alirocumab for more than twelve months, the reduction of MACE events was increased and significant (HR = 0.71 (confidence interval 0.58-0.87)). A similar trend was observed for all-cause death. Patients with a baseline LDL-C above 100 mg/dL who received treatment of alirocumab for less than twelve months showed a reduction in all cause death (HR = 0.79 (confidence interval 0.51-1.22)). When the same population was treated with alirocumab for more than twelve months, all-cause death was decreased and significant (HR = 0.67 (confidence interval 0.50-0.89)). An additional pre-specified analysis of MACE and all-cause death in 8242 patients who were eligible for at least three years of follow-up is shown in **Figure 20****.** More than 44% of the intent-to-treat (ITT) population was treated for over three years. As shown here, lower rates of MACE events **(****Figure 20B****;** HR 0.83) and all-cause death **(****Figure 20D****;** HR 0.78) were observed in the population treated for at least three years as compared to MACE events **(****Figure 20A****;** HR 0.85) and all-cause death **(****Figure 20C****;** HR 0.85) in the total study population.

These results indicate that the benefits of alirocumab treatment continue to accrue, with greater risk reductions beyond the first year in the overall population, as well as in patients with baseline LDL-C levels at or above 100 mg/dL.

**Table 14. Post Hoc Landmark Analysis**

| | **All patients** | | | **Patients with ≥100 mg/dL LDL-C at baseline** | | |
|---|---|---|---|---|---|---|
| | **Overall HR** | **0-12 Months** | **Beyond 12 months** | **Overall HR** | **0-12 Months** | **Beyond 12 months** |
| **Time to first MACE event** | 0.85 (0.78-0.93) | 0.94 (0.83-1.08) | 0.77 (0.69-0.87) | 0.76 (0.65-0.87) | 0.81 (0.66-1.01) | 0.71 (0.58-0.87) |
| **Time to first all-cause death** | 0.85 (0.73-0.98) | 1.01 (0.77-1.32) | 0.79 (0.66-0.94) | 0.71 (0.56-0.90) | 0.79 (0.51-1.22) | 0.67 (0.50-0.89) |

### Example 6: Secondary Analysis - Effect of Alirocumab on All-Cause Death, Cardiovascular Death, and Non-Cardiovascular Death in Patients Treated for More than One Year

### a. Effect on All Cause Death

A total of 726 (3.8%) patients died during the trial. The baseline characteristics of the total patient cohort, survivors, and patients who died are summarized in **Table 16.** There were fewer deaths in the alirocumab group than in the placebo group (334 [3.5%] vs 392 [4.1%], respectively; HR 0.85, 95% CI 0.73 to 0.98; p=0.026; **Table 15**)**.** Based on 4-year Kaplan-Meier estimates of death at 4 years of 5.3% and 6.4%, respectively, in the alirocumab and placebo groups, the absolute risk reduction was 1.1% and the number of patients needed to treat for 4 years to prevent 1 death was 87.

**Table 15. Summary of all-cause death, cardiovascular death and noncardiovascular death endpoints**

| **Endpoint** | **Alirocumab (n=9462)** | **Placebo (n=9462)** | **HR (95% CI)*** | **Log-rank p-value*** |
|---|---|---|---|---|
| All-cause death | 334 (3.5%) | 392 (4.1%) | 0.85 (0.73, 0.98) | 0.03 |
| Cardiovascular death | 240 (2.5%) | 271 (2.9%) | 0.88 (0.74, 1.05) | 0.15 |
| Non-cardiovascular death | 94 (1.0%) | 121 (1.3%) | 0.77 (0.59, 1.01) | 0.06 |
| *Stratified by region. | | | | |

Similar to the primary endpoint, allowing a non-constant treatment hazard rate on all-cause death suggested a lag in the treatment effect of alirocumab; treatment benefit was absent during the first year (HR 1.01, 95% CI 0.77 to 1.32; p=0.95), but was evident after the first year (HR 0.79, 95% CI 0.66 to 0.94; p=0.0073; p=0.13 for improvement in model fit vs the constant hazard model [with an HR of 0.85]).

Given prior evidence for a delayed benefit of lipid lowering in many statin trials, a pre-specified analysis examined whether the reduction in death with alirocumab was related to the potential duration of treatment. Death was examined among 8242 patients (44% of the full study cohort) eligible for ≥3 years of follow-up. The baseline characteristics of this subset and the remaining subset of patients enrolled for <3 years before the common study end date are described in **Table 17.** Among patients eligible for ≥3 years of follow-up, the benefit of alirocumab on all-cause death was more pronounced than in the overall trial population, i.e. alirocumab reduced death by 22% (HR 0.78, 95% CI 0.65 to 0.94; p=0.01; **Figure 20D****).** For patients eligible for <3 years of follow-up, the treatment hazard ratio for all-cause death was 0.96 (95% CI 0.76 to 1.21; p=0.71; interaction p=0.19).

**Table 16. Baseline characteristics**

| **Characteristic** | | **All patients (N=18 924)** | **Died during follow-up (N=726)** | **Alive at end of follow-up (N=18 198)** | |
|---|---|---|---|---|---|
| Age, yr | | 59 (52, 65) | 64 (57, 71) | 58 (52, 65) | |
| Female sex | | 4762 (25.2%) | 202 (27.8%) | 4560 (25.1%) | |

| Race† | | | | | |
|---|---|---|---|---|---|
| | White | 15 024 (79.4%) | 551 (75.9%) | 14 443 (79.5%) | |
| | Asian | | 2498 (13.2%) | 93 (12.8%) | 2405 (13.2%) |
| | Black or African American | | 473 (2.5%) | 26 (3.6%) | 447 (2.5%) |
| | Other | | 929 (4.9%) | 56 (7.7%) | 873 (4.8%) |

| Region of enrollment | | | | | |
|---|---|---|---|---|---|
| | Central and Eastern Europe | | 5437 (28.7%) | 234 (32.2%) | 5203 (28.6%) |
| | Western Europe | | 4175 (22.1%) | 84 (11.6%) | 4091 (22.5%) |
| | Canada/USA | | 2871 (15.2%) | 136 (18.7%) | 2735 (15.0%) |
| | Latin America | | 2588 (13.7%) | 133 (18.3%) | 2455 (13.5%) |
| | Asia | | 2293 (12.1%) | 75 (10.3%) | 2218 (12.2%) |
| | Rest of the world | | 1560 (8.2%) | 64 (8.8%) | 1496 (8.2%) |

| Medical history before index ACS | | | | | |
|---|---|---|---|---|---|
| | Hypertension | | 12 249 (64.7%) | 583 (80.3%) | 11 666 (64.1%) |
| | Family history of premature CHD | | 6773 (35.8%) | 272 (37.5%) | 6501 (35.7%) |
| | Myocardial infarction | | 3639 (19.2%) | 245 (33.7%) | 3394 (18.7%) |
| | PCI | | 3241 (17.1%) | 188 (25.9%) | 3053 (16.8%) |
| | CABG | | 1047 (5.5%) | 98 (13.5%) | 949 (5.2%) |
| | Stroke | | 611 (3.2%) | 68 (9.4%) | 543 (3.0%) |
| | Peripheral artery disease | | 759 (4.0%) | 76 (10.5%) | 683 (3.8%) |
| | Congestive heart failure | | 241 (8.6%) | 2574 (14.1%) | 2815 (14.9%) |
| | COPD | | 746 (3.9%) | 102 (14.0%) | 644 (3.5%) |
| | Malignant disease | | 532 (2.8%) | 35 (4.8%) | 497 (2.7%) |

| Diabetes status | | | | | |
|---|---|---|---|---|---|
| | Diabetes mellitus | | 5444 (28.8%) | 346 (47.7%) | 5098 (28.0%) |
| | Prediabetes | | 8246 (43.6%) | 253 (34.8%) | 7993 (43.9%) |
| | Normal | | 5234 (27.7%) | 127 (17.5%) | 5107 (28.1%) |

| Tobacco smoking status | | | | | |
|---|---|---|---|---|---|
| | Current | | 4560 (24.1%) | 164 (22.6%) | 4396 (24.2%) |
| | Former | | 7811 (41.3%) | 311 (42.8%) | 7500 (41.2%) |
| | Never | | 6552 (34.6%) | 251 (34.6%) | 6301 (34.6%) |

| Index ACS event | | | | | |
|---|---|---|---|---|---|
| | STEMI | | 6536 (34.6%) | 181 (25.1%) | 6355 (35.0%) |
| | Non-STEMI | | 9175 (48.6%) | 407 (56.5%) | 8768 (48.2%) |
| | Unstable angina | | 3182 (16.8%) | 132 (18.3%) | 3050 (16.8%) |
| PCI or CABG for index ACS | | | 13677 (72.3) | 421 (58.0) | 13256 (72.8) |
| Time from index ACS to randomization, months | | | 2.6 (1.7, 4.3) | 2.5 (1.7, 3.7) | 2.6 (1.7, 4.4) |
| Body mass index‡ | | | 27.9 (25.2, 31.1) | 27.7 (24.5, 31.5) | 27.9 (25.2, 31.1) |

| Medications at randomization | | | | | |
|---|---|---|---|---|---|
| | Lipid-modifying agents | | | | |
| | | High-intensity atorvastatin/rosuvastatin | 16 811 (88.8%) | 639 (88.0%) | 16 172 (88.9%) |
| | | Low-/moderate-intensity atorvastatin/rosuvastatin | 1607 (8.5%) | 61 (8.4%) | 1546 (8.5%) |
| | | Other statin | 46 (0.2%) | 1 (0.1%) | 45 (0.2%) |
| | | Ezetimibe | 554 (2.9) | 18 (2.5) | 536 (2.9) |
| | Aspirin | 18 086 (95.6%) | 667 (91.9%) | 17 419 (95.7%) | |
| | P2Y₁₂ antagonist | 16 541 (87.4%) | 597 (82.2%) | 15 944 (87.6%) | |
| | Beta-blocker | 15 990 (84.5%) | 603 (83.1%) | 15 387 (84.6%) | |
| | ACE inhibitor or ARB | 14 716 (77.8%) | 589 (81.1%) | 14 127 (77.6%) | |
| Use of a statin during the 3 months immediately before index ACS | | 6164 (32.6%) | 286 (39.6%) | 5878 (32.3%) | |

| Lipid levels at randomization (mg/dL)* | | | | | |
|---|---|---|---|---|---|
| | LDL-C§ | 86 (73, 104) | 91 (74, 110) | 86 (73, 104) | |
| | Non-HDL-C | 115 (99, 137) | 120 (100, 143) | 115 (99, 136) | |
| | Apolipoprotein B | 79 (69, 93) | 82 (71, 98) | 79 (69, 93) | |
| | HDL cholesterol | 43 (36, 50) | 42 (36, 51) | 43 (36, 50) | |
| | Triglycerides | 129 (94, 182) | 132 (92, 187) | 129 (94, 182) | |
| | Lipoprotein(a) | 21 (7, 60) | 22 (7, 57) | 21 (7, 60) | |
| Hemoglobin A_{1c}, % | | 5.8 (5.5, 6.3) | 6.1 (5.7, 7.3) | 5.8 (5.5, 6.3) | |
| GFR, ml/min per 1.73 m² | | 78.2 (67.4, 90.1) | 70.2 (54.9, 85.2) | 78.5 (67.8, 90.4) | |
| High-sensitivity C-reactive protein, mg/L | | 1.6 (0.8, 3.9) | 2.8 (1.1, 6.6) | 1.6 (0.8, 3.8) | |

| | | | | | |
|---|---|---|---|---|---|
| ACE = angiotensin converting enzyme; ACS = acute coronary syndrome; ARB = angiotensin II receptor blocker; CABG = coronary artery bypass graft; CHD = coronary heart disease; GFR = glomerular filtration rate; HDL = high-density lipoprotein cholesterol; LDL-C = low-density lipoprotein cholesterol; PCI = percutaneous coronary intervention; STEMI = ST-segment elevation myocardial infarction. *To convert the values for cholesterol to mmol/L, multiply by 0.02586. To convert the values for triglycerides to mmol/L, multiply by 0.01129. Data are median (interquartile range) except mean (standard deviation) as noted. † Race was self-reported. ‡ Body-mass index is the weight in kilograms divided by the square of the height in meters. § LDL-C was calculated with the Friedewald formula; calculated values <15 mg/dL were confirmed by ultracentrifugation/beta quantification. | | | | | |

**Table 17: Baseline Characteristics According to Duration of Potential Follow-Up**

| **Characteristic** | | **Eligible for ≥3 years of follow-up (n=8242)** | **Not eligible for 3 years of follow-up (n=10 682)** | |
|---|---|---|---|---|
| Age, yr | | 58 (52, 65) | 58 (52, 65) | |
| Female sex | | 2069 (25.1%) | 2693 (25.2%) | |

| Race† | | | | |
|---|---|---|---|---|
| | White | 7055 (85.6%) | 7969 (74.6%) | |
| | Asian | 585 (7.1%) | 1913 (17.9%) | |
| | Black or African American | 205 (2.5%) | 268 (2.5%) | |
| | Other | 397 (4.8%) | 532 (5.0%) | |

| Region of enrolment | | | | |
|---|---|---|---|---|
| | Central and Eastern Europe | 2428 (29.5%) | 3009 (28.2%) | |
| | Western Europe | 1957 (23.7%) | 2218 (20.8%) | |
| | Canada/USA | 1474 (17.9%) | 1397 (13.1%) | |
| | Latin America | 1041 (12.6%) | 1547 (14.5%) | |
| | Asia | 459 (5.6%) | 1834 (17.2%) | |
| | Rest of the world | | 883 (10.7%) | 677 (6.3%) |

| Medical history before index ACS | | | | |
|---|---|---|---|---|
| | Hypertension | | 5373 (65.2%) | 6876 (64.4%) |
| | Family history of premature CHD | | 3114 (37.8%) | 3659 (34.3%) |
| | Myocardial infarction | | 1753 (21.3%) | 1886 (17.7%) |
| | PCI | | 1567 (19.0%) | 1674 (15.7%) |
| | CABG | | 515 (6.3%) | 532 (5.0%) |
| | Stroke | | 269 (3.3%) | 342 (3.2%) |
| | Peripheral artery disease | | 380 (4.6%) | 379 (3.6%) |
| | Congestive heart failure | | 1242 (15.1%) | 1573 (14.7%) |
| | COPD | | 357 (4.3%) | 389 (3.6%) |
| | Malignant disease | | 250 (3.0%) | 282 (2.6%) |

| Diabetes status | | | | |
|---|---|---|---|---|
| | Diabetes mellitus | | 2287 (27.7%) | 3157 (29.6%) |
| | Prediabetes | | 3550 (41.1%) | 4696 (44.0%) |
| | Normal | | 2405 (29.2%) | 2829 (26.5%) |

| Tobacco smoking status | | | | |
|---|---|---|---|---|
| | Current | | 2058 (25.0%) | 2502 (23.4%) |
| | Former | | 3318 (40.3%) | 4493 (42.1%) |
| | Never | | 2865 (34.8%) | 3687 (34.5%) |

| Index ACS event | | | | |
|---|---|---|---|---|
| | STEMI | | 2752 (33.5%) | 3784 (35.5%) |
| | Non-STEMI | | 4286 (52.1%) | 4889 (45.8%) |
| | Unstable angina | | 1183 (14.4%) | 1999 (18.7%) |
| PCI or CABG for index ACS | | | 5815 (70.6) | 7862 (73.6) |
| Time from index ACS to randomization, months | | | 2.3 (1.6, 3.3) | 3.1 (1.9, 5.9) |
| Body mass index‡ | | | 28.2 (25.5, 31.4) | 27.7 (25.0, 30.9) |

| Medications at randomization | | | | |
|---|---|---|---|---|
| | Lipid-modifying agents | | | |
| | | High-intensity atorvastatin/rosuvastatin | 7487 (90.8%) | 9324 (87.3%) |
| | | Low-/moderate-intensity atorvastatin/rosuvastatin | 535 (6.5%) | 1072 (10.0%) |
| | | Other statin | 28 (0.3%) | 18 (0.2%) |
| | | Ezetimibe | 233 (2.8) | 321 (3.0) |
| | Aspirin | | 7866 (95.4%) | 10220 (95.7%) |
| | P2Y₁₂ antagonist | | 7194 (87.3%) | 9347 (87.5%) |
| | Beta-blocker | | 7019 (85.2%) | 8971 (84.0%) |
| | ACE inhibitor or ARB | | 6458 (78.4%) | 8258 (77.3%) |
| Use of a statin during the 3 months immediately before index ACS | | | 2754 (33.4) | 3410 (31.9) |

| Lipid levels at randomization* | | | | |
|---|---|---|---|---|
| | LDL cholesterol§ | | 87 (74, 104) | 86 (73, 104) |
| | Non-HDL cholesterol | | 115 (100, 137) | 115 (99, 137) |
| | Apolipoprotein B | | 80 (69, 93) | 79 (69, 93) |
| | HDL cholesterol | 42 (37, 50) | 42 (36, 50) | |
| | Triglycerides | 127 (92, 181) | 131 (96, 182) | |
| | Lipoprotein(a) | 19 (6, 56) | 22 (7, 62) | |
| Hemoglobin A_{1c}, % | | 5.8 (5.5, 6.3) | 5.9 (5.6, 6.4) | |

| GFR, ml/min per 1.73 m² | | | | |
|---|---|---|---|---|
| High-sensitivity C-reactive protein, mg/L | | 1.7 (0.8, 4.1) | 1.6 (0.7, 3.7) | |

| | | | | |
|---|---|---|---|---|
| ACE = angiotensin converting enzyme; ACS = acute coronary syndrome; ARB = angiotensin II receptor blocker; CABG = coronary artery bypass graft; CHD = coronary heart disease; GFR = glomerular filtration rate; HDL = high-density lipoprotein; LDL = low-density lipoprotein; PCI = percutaneous coronary intervention; STEMI = ST-segment elevation myocardial infarction. * Lipid levels are reported in units of mg/dL. To convert the values for cholesterol to mmol/L, multiply by 0.02586. To convert the values for triglycerides to mmol/L, multiply by 0.01129. Data are median (interquartile range) except mean (standard deviation) as noted. † Race was self-reported. ‡ Body-mass index is the weight in kilograms divided by the square of the height in meters. § LDL cholesterol was calculated with the Friedewald formula; calculated values <15 mg/dL were confirmed by ultracentrifugation/beta quantification. | | | | |

### b. Effect on Cardiovascular and Non-Cardiovascular Death

CHD death occurred in 205 (2.2%) patients in the alirocumab group and 222 (2.3%) patients in the placebo group (HR 0.92, 95% CI 0.76 to 1.11; p=0.38), and represented 427 (58.8%) of 726 total deaths. Cardiovascular death occurred in 240 (2.5%) patients in the alirocumab group and 271 (2.9%) patients in the placebo group (HR 0.88, 95% CI 0.74 to 1.05; p=0.15), and represented 511 (70.4%) deaths of all-cause mortality. Non-CV death was numerically but non-significantly lower with alirocumab (94 [1.0%] vs 121 [1.3%]; HR 0.77, 95% CI 0.59 to 1.01; p=0.06) compared with placebo (Table 15). Adjudicated CV and non-CV causes of death are summarized in Tables 18 and 19. There were a low proportion of undetermined causes of death. Among 31 fewer CV deaths in the alirocumab group than in the placebo group, the largest differences between groups were for fatal myocardial infarction (n=10) and sudden cardiac death (n=8). Among 27 fewer non-CV causes of death in the alirocumab group versus the placebo group, the largest difference was for death due to pulmonary (n=14) causes.

**Table 18. Adjudicated causes of cardiovascular death**

| **Cause of death, n** | **Alirocumab (n=240)** | **Placebo (n=271)** | **Delta** | |
|---|---|---|---|---|
| Acute myocardial infarction | 41 | 51 | -10 | |
| Cardiovascular hemorrhage | 0 | 2 | -2 | |
| Cardiovascular procedure | 5 | 4 | +1 | |
| Heart failure or cardiogenic shock | 29 | 33 | -4 | |
| Other cardiovascular causes Stroke | 16 | 14 | +2 | |
| | Hemorrhagic | 9 | 8 | +1 |
| | Ischemic | 10 | 14 | -4 |
| | Undetermined | 0 | 2 | -2 |
| Sudden cardiac | | 109 | 117 | -8 |
| Undetermined | | 21 | 26 | -5 |

**Table 19. Adjudicated causes of non-cardiovascular death**

| **Cause of death, n** | | **Alirocumab (n=94)** | **Placebo (n=121)** | **Delta** |
|---|---|---|---|---|
| Pulmonary | | **33** | 47 | -14 |
| | Primary cause of death = infection | **12** | 19 | |
| | Primary cause of death = malignancy | **18** | 22 | |
| Other non-cardiovascular | | **22** | 28 | -6 |
| | Primary cause of death = infection | **6** | 11 | |
| | Primary cause of death = malignancy | **14** | 14 | |
| Gastrointestinal/hepatobiliary/Pancreatic | | **15** | 19 | -4 |
| | Primary cause of death = infection | **4** | 3 | |
| | Primary cause of death = malignancy | **9** | 14 | |
| Hemorrhage | | **3** | 6 | -3 |
| | Primary cause of death = malignancy | **0** | 1 | |
| Neurological process not a stroke/hemorrhage | | **1** | 4 | -3 |
| | Primary cause of death = malignancy | **1** | 2 | |
| Suicide | | **2** | 5 | -3 |
| Non-cardiovascular procedure or surgery | | **3** | 2 | +1 |
| | Primary cause of death = infection | **1** | 0 | |
| | Primary cause of death = malignancy | **0** | 1 | |
| Accident or trauma | | **10** | 8 | +2 |
| Renal | | **5** | 2 | +3 |
| | Primary cause of death = infection | **2** | 0 | |
| | Primary cause of death = malignancy | **1** | 2 | |

### c. Relationship between Non-fatal Cardiovascular Events and Non-Cardiovascular Death

Overall, there were 206 fewer non-fatal CV events in the alirocumab group than in the placebo group (HR 0.83, 95% CI 0.76 to 0.91; p<0.0001). When non-fatal CV events and non-CV death are described as ordinal functions **(Table 20),** there were numerically fewer of both types of events with alirocumab than with placebo as first events, second events, and third or more events, suggesting a linkage between the two types of events.

Furthermore, conditional on having a non-fatal CV event, the risk of subsequent non-CV death was higher. Among patients at risk for a first event in the alirocumab and placebo groups, non-CV death occurred as a first event in 0.8% and 0.9%, respectively. After a first non-fatal CV event occurring an overall median of 1.1 (0.4, 1.9) years after randomization, non-CV death occurred as a second event in 1.8% and 2.0%, respectively, of the patients in the alirocumab and placebo groups. Similarly, after a second non-fatal CV event occurring an overall median of 1.5 (0.9, 2.3) years after randomization, non-CV death occurred as a third event in 3.7% and 6.2%, respectively, of the patients in the alirocumab and placebo groups. Qualitatively, these data indicate that each successive prior non-fatal CV event is associated with an increased subsequent risk for non-CV death.

**Table 20: Number and timing of non-cardiovascular deaths and nonfatal myocardial infarction, stroke, or unstable angina requiring hospitalization according to ordinal event number**

| | | **Alirocumab** | **Placebo** | | |
|---|---|---|---|---|---|
| | | **n (%)** | **Median (Q1, Q3) event time** | **n (%)** | **Median (Q1, Q3) event time** |
| First event | | | | | |
| | Non-fatal myocardial infarction, stroke, or unstable angina | 761 (8.0%) | 1.0 (0.4, 1.9) | 917 (9.7%) | 1.1 (0.5, 1.9) |
| | Non-cardiovascular death | 74 (0.8%) | 1.9 (1.2, 2.5) | 90 (0.9%) | 1.8 (1.2, 2.4) |

| Second event | | | | | |
|---|---|---|---|---|---|
| | Non-fatal myocardial infarction, stroke, or unstable angina | 162 (21.3%) | 1.4 (0.9, 2.2) | 210 (22.9%) | 1.6 (0.9, 2.4) |
| | Non-cardiovascular death | 14 (1.8%) | 2.7 (1.8, 3.3) | 18 (2.0%) | 1.6 (1.1, 2.4) |

| Third or more event(s) | | | | | |
|---|---|---|---|---|---|
| | Non-fatal myocardial infarction, stroke, or unstable angina | 111 | | 113 | |
| | Non-cardiovascular death | 6 (3.7%) | | 13 (6.2%) | |
| Total non-cardiovascular deaths | | 94 (1.0%) | 2.0 (1.4, 2.7) | 121 (1.3%) | 1.8 (1.2, 2.4) |

| | | | | | |
|---|---|---|---|---|---|
| Event time is expressed as years since randomization | | | | | |

To explore this possibility quantitatively, a joint semiparametric model was constructed with treatment assignment and region of enrolment in the hazard functions **(Table 21).** The model indicated that alirocumab was a predictor of reduced CV mortality (p=0.043), and provided an association parameter of 2.15 (95% CI 1.61 to 2.69), indicating that patients at higher risk for non-fatal CV events were also at higher risk for CV death. Thus, a dependency between antecedent non-fatal CV events and subsequent fatal CV events, coupled with a reduction in the former with alirocumab, indicates that prevention of non-fatal CV events may account for the lower number of CV deaths with alirocumab.

**Table 21: Joint semiparametric model results: total non-fatal myocardial infarction, stroke, or unstable angina and non-cardiovascular deaths, Treatment and Geographic Region* in Model**

| | | **HR (95% CI)** | **P-value** |
|---|---|---|---|
| Non-fatal myocardial infarction, stroke, or unstable angina (1034 events for alirocumab vs 1240 for placebo) | | | |
| | Alirocumab treatment | 0.83 (0.76, 0.91) | <0.0001 |
| | Eastern Europe | 0.90 (0.75, 1.09) | <0.0001 |
| | North America | 2.20 (1.83, 2.64) | |
| | Rest of the world | 1.63 (1.32, 2.00) | |
| | South America | 0.93 (0.75, 1.15) | |
| | Western Europe | 1.28 (1.06, 1.54) | |

| Non-cardiovascular death (94 events for alirocumab vs 121 for placebo) | | | |
|---|---|---|---|
| | Alirocumab treatment | 0.75 (0.57, 0.99) | 0.0426 |
| | Eastern Europe | 1.58 (0.92, 2.72) | 0.0273 |
| | North America | 1.87 (1.05, 3.34) | |
| | Rest of the world | 1.20 (0.60, 2.41) | |
| | South America | 2.09 (1.17, 3.74) | |
| | Western Europe | 1.13 (0.63, 2.04) | |
| Association between non-fatal and fatal events: *α̂* (95% CI) = 2.15 (1.61 to 2.69) | | - | «0.0001 |
| CI, confidence interval; HR, hazard ratio. | | | |

| | | | |
|---|---|---|---|
| * Asia is used as reference for geographic region analyses. | | | |

To explore whether the strength of the relationship between non-fatal CV events and non-CV death was affected by adjustment for factors that were prognostic for non-fatal CV events and/or non-CV death, a series of univariate joint semiparametric models were fit with predictors expected to be predictive of these events. After including all factors significantly related to non-fatal CV events or non-CV death in univariate models-that allowed convergence of the adjusted model-the estimated association parameter remained significant (1.82, 95%CI: 1.08 to 2.55; p<0.0001), indicating a persistent, strong relationship between non-fatal CV events and non-CV death.

### d. Relationship of Death to Baseline and Achieved LDL-C Levels

In a post-hoc analysis, death was examined in three predefined subgroups of baseline LDL-C level (<80 mg/dL [2.07 mmol/L], 80 to <100 mg/dL [2.07 to <2.59 mmol/L], and ≥100 mg/dL [2.59 mmol/L]; **Figures 8A** - **8C**). The HR for death was numerically lowest in the subgroup with baseline LDL-C ≥100 mg/dL (HR 0.71, 95% CI 0.56 to 0.90), but there was no significant heterogeneity of the effect of alirocumab on relative risk of death across categories of baseline LDL-C (p interaction=0.12). In contrast, there were significant gradients of the absolute risk of death in the placebo group and the absolute difference in death between alirocumab and placebo groups across baseline LDL-C subgroups, with greatest risk in the placebo group, and greatest risk reduction with alirocumab observed in the subgroup with baseline LDL-C ≥100 mg/dL (P interaction<0.005). Using the Kaplan-Meier estimates of all-cause death at 4 years for patients with a baseline LDL-C value ≥100 mg/dL (5.8% vs 9.6%), the absolute risk reduction was 3.8% and the number needed to treat for 4 years to avoid 1 death in that patient subset was 26.

Additional analyses explored the relation between LDL-C levels achieved 4 months after randomization and death occurring after that time. In linear models according to quartile of achieved LDL-C, there was lower risk of all-cause death (p=0.005) in patients with lower achieved LDL-C values at 4 months **(Table 22).** When the trial population was stratified by treatment assignment, there was no relation between achieved LDL-C value at 4 months and subsequent death in the placebo group, wherein the median LDL-C value at 4 months was 87 mg/dL (2.25 mmol/L) **(Table 23).** In contrast, a strong relationship was evident in the alirocumab group (Table 4) wherein the median achieved LDL-C value at 4 months was 31 mg/dL (0.80 mmol/L). A spline analysis relating continuous values of achieved LDL-C to the risk of all-cause death yielded similar findings: the relation between achieved LDL-C at 4 months and subsequent death in the entire trial cohort was a nearly monotonic function (p-value for model = 0.007) **(****Figure 19A****)** that was more pronounced in the alirocumab group **(****Figure 19C****).** In contrast, the relation in the placebo group was U-shaped, with higher mortality in patients with either lowest or highest achieved LDL-C at 4 months **(****Figure 19B****).**

**Table 22: All-cause, cardiovascular, and non-cardiovascular death by ITT month 4 LDL-C quartile (all patients)**

| **Endpoint** | | **Incidence n/N (%)** | **HR (95% CI)*** | **P-value for linear trend*** |
|---|---|---|---|---|
| All-cause death | | | | |
| | Q1 | 135/4682 (2.9) | 0.73 (0.58, 0.91) | 0.005 |
| | Q2 | 164/4733 (3.5) | 0.86 (0.69, 1.05) | |
| | Q3 | 167/4721 (3.5) | 0.90 (0.73, 1.10) | |
| | Q4 | 192/4712 (4.1) | Reference | |

| Cardiovascular death | | | | |
|---|---|---|---|---|
| | Q1 | 92/4682 (2.0) | 0.73 (0.56, 0.95) | 0.028 |
| | Q2 | 116/4733 (2.5) | 0.89 (0.69, 1.14) | |
| | Q3 | 112/4721 (2.4) | 0.88 (0.69, 1.14) | |
| | Q4 | 130/4712 (2.8) | Reference | |

| Noncardiovascular death | | | | |
|---|---|---|---|---|
| | Q1 | 43/4682 (0.9) | 0.73 (0.50, 1.08) | 0.08 |
| | Q2 | 48/4733 (1.0) | 0.78 (0.54, 1.14) | |
| | Q3 | 55/4721 (1.2) | 0.92 (0.64, 1.32) | |
| | Q4 | 62/4712 (1.3) | Reference | |

| | | | | |
|---|---|---|---|---|
| *Stratified by region; ITT=intention to treat; Month-4 LDL-C quartiles: Q1 = ≤0.80 mmol/L (≤31 mg/dL); Q2 = >0.80 to 1.68 mmol/L (>31 to ≤65 mg/dL); Q3 = >1.68 to 2.38 mmol/L (>65 to ≤92 mg/dL); Q4 = >2.38 mmol/L (>92 mg/dL) | | | | |

**Table 23. All-cause death by ITT month-4 LDL-C quartile in the placebo and alirocumab groups**

| **LDL-C quartile** | | **Incidence n/N (%)** | **HR (95% CI)*** | **P-value for linear trend*** |
|---|---|---|---|---|
| Placebo group | | | | 0.96 |
| | Q1: ≤1.86 mmol/L (≤72 mg/dL) | 104/2373 (4.4) | 0.99 (0.75, 1.29) | |
| | Q2: >1.86 to ≤2.25 mmol/L (>72 to ≤87 mg/dL) | 74/2354 (3.1) | 0.73 (0.54, 0.97) | |
| | Q3: >2.25 to ≤2.77 mmol/L (>87 to ≤107 mg/dL) | 71/2363 (3.0) | 0.68 (0.50, 0.91) | |
| | Q4: >2.77 mmol/L (>107 mg/dL) | 110/2336 (4.7) | Reference | |

| Alirocumab group | | | | 0.027 |
|---|---|---|---|---|
| | Q1: ≤0.52 mmol/L (≤20 mg/dL) | 70/2319 (3.0) | 0.71 (0.52, 0.96) | |
| | Q2: >0.52 to ≤0.80 mmol/L (>20 to ≤31 mg/dL) | 62/2369 (2.6) | 0.63 (0.46, 0.87) | |
| | Q3: >0.80 to ≤1.27 mmol/L (>31 to ≤49 mg/dL) | 69/2389 (2.9) | 0.68 (0.50, 0.93) | |
| | Q4: >1.27 mmol/L (>49 mg/dL) | 98/2345 (4.2) | Reference | |

| | | | | |
|---|---|---|---|---|
| *Stratified by region; Cl=confidence interval; HR=hazard ratio; ITT=intention to treat; LDL-C=low-density lipoprotein cholesterol. | | | | |

### CONCLUSION

Alirocumab reduced death after ACS in patients with elevated atherogenic lipoproteins on intensive statin treatment, with concordant effects on CV and non-CV death. Patients with higher LDL-C at baseline or followed for ≥3 years had particularly prominent reductions in death with alirocumab. Lower achieved LDL-C was associated with fewer deaths, particularly patients achieving lower LDL-C levels at 4 months of treatment with alirocumab experienced a reduced risk of death. Joint semiparametric model analysis demonstrated that the risk of non-CV death was linked to the risk of non-fatal CV events (e.g. non-fatal MI, unstable angina requiring hospitalization and non-fatal ischemic stroke), and alirocumab had a strong effect on non-fatal CV events. Accordingly, the prevention of non-fatal CV events with alirocumab was associated with a reduction in non-CV death.

### Example 7: Secondary Analysis - Effect of Alirocumab on Total Number of Fatal and Non-Fatal Cardiovascular Events

Risk of non-CV death is routinely assumed to be independent of risk of non-fatal CV events and unmodifiable by LDL-C lowering. Moreover, prior trials (FOURIER, SPIRE) have failed to demonstrate an effect on non-CV death. Therefore, in a further secondary analysis, the effect of alirocumab on total (first and subsequent) non-fatal cardiovascular (CV) events and all-cause death was analyzed using a joint frailty model that accounts for the potential relationship between non-fatal and fatal events. This analysis included all-cause death and total non-fatal CV events (MI, stroke, hospitalization for UA or heart failure, or ischemia-driven coronary revascularization). A sensitivity analysis restricted non-fatal events to MI, stroke, or UA. Total non-fatal and fatal event hazard functions were separately estimated by the joint model, linked by a shared frailty that accounted for risk heterogeneity across patients and correlated within-patient non-fatal events. The model also determined if non-fatal events were associated with an increased risk for death. The model provides accurate relative estimates of non-fatal event risk if non-fatal events are associated with increased risk for death. Treatment effects were summarized by HRs and compared against the customary analysis of first non-fatal CV event or death.

There were 5425 total deaths or non-fatal CV events, 77% greater than first events (3064). Alirocumab, compared with placebo, produced similar relative reductions in first and total events. Importantly, there were 385 fewer total non-fatal cardiovascular or death events with alirocumab (2,905 events for placebo, 2,520 events for alirocumab), versus 190 fewer first non-fatal cardiovascular or death events (1,627 events for placebo, 1,437 events for alirocumab) **(****Figure 23****).** Thus, analysis of first events reflects only about half of the total event reduction associated with alirocumab treatment over a median of 2.8 years.

At 4 years, the estimates for the expected total number of non-fatal cardiovascular events for a given patient in the placebo and alirocumab groups at a given time after randomization are 0.357 and 0.301, respectively. In contrast, the expected proportions of patients with a first non-fatal cardiovascular event in the placebo and alirocumab groups were 0.183 and 0.160, respectively. The number needed to treat (95% CI) for 4 years to prevent one non-fatal event is 18 (11 to 53) based on total events and 44 (26 to 129) based on first events.

Non-fatal CV events were associated with a markedly higher risk of death, and the frailty variance indicated substantial inter-patient heterogeneity in risk **(Table 24).**

**Table 24. Analysis of the effect of alirocumab on total non-fatal CV events and all-cause death**

| | | **HR (95% CI)** | **P-value** |
|---|---|---|---|
| **Death and total non-fatal CV events (n=5425)** | | | |
| | Alirocumab : placebo HR for non-fatal events (n=2186 vs. n=2513) | 0.85 (0.78-0.93) | 0.0002 |
| Alirocumab : placebo HR for fatal events (n=334 vs. n=392) | | 0.82 (0.70-0.96) | 0.0158 |
| | Association between non-fatal and fatal events | 2.77 (2.48-3.10) | <0.0001 |
| | Frailty variance 2.42 (95% CI 2.23-2.61) | - | <0.0001 |

| **Death and total non-fatal CV events restricted to MI, stroke, or UA (n=2999)** | | | |
|---|---|---|---|
| | Alirocumab : placebo HR for non-fatal events (n=1034 vs. n=1239) | 0.81 (0.73-0.91) | 0.0002 |
| | Alirocumab : placebo HR for fatal events (n=334 vs. n=392) | 0.81 (0.68-0.96) | 0.0161 |
| | Association between non-fatal and fatal events | 3.22 (2.77-3.78) | <0.0001 |
| | Frailty variance 2.47 (95% CI 2.24-2.70) | - | <0.0001 |
| **Analysis of first event: death or non-fatal CV event (n=3064)** | | | |
| | Alirocumab : placebo HR for first event (n=1437 vs. 1627) | 0.88 (0.82-0.94) | 0.0002 |

| | | | |
|---|---|---|---|
| CI, confidence interval; HR, hazard ratio. | | | |

Based on the estimated proportions at 4 years, the number needed to treat (95% CI) to prevent 1 non-fatal event is 44 (26 to 129) based on first events and 18 (11 to 53) based on total events. Accounting for total events therefore illustrates the high burden of ongoing disease in the study population and the diminution of that burden by alirocumab **(****Figure 22****).** Corresponding (post hoc) plots by baseline LDL-C subgroups are shown in **Figure 23A and 23B****.** In **Figure 23A****,** the expected number of non-fatal cardiovascular events for a given patient with baseline LDL-C ≥100 mg/dL in the placebo and alirocumab groups at 4 years were 0.489 and 0.380, respectively, while the expected proportion of patients with a first non-fatal cardiovascular event in the placebo and alirocumab groups were 0.237 and 0.183, respectively. The number needed to treat (95% CI) for 4 years to prevent one non-fatal event is 19 (12 to 45) based on first events and 9 (5 to 46) based on total events. The number needed to treat to prevent one non-fatal event in the LDL-C ≥100 mg/dL subgroup based on total events is 9 (5 to 46). In **Figure 23B****,** the expected number of non-fatal cardiovascular events for a given patient with baseline LDL-C <100 mg/dL in the placebo and alirocumab groups at 4 years were 0.160 and 0.151, respectively, while the expected proportion of patients with a first non-fatal cardiovascular event in the placebo and alirocumab groups were 0.302 and 0.267, respectively. The number needed to treat (95% CI) for 4 years to prevent one non-fatal event is 103 (38 to -140) based on first events and 29 (14 to -217) based on total events (negative values in CI reflect harm).

The types and counts of adjudicated non-fatal cardiovascular events after randomization are summarized below in **Table 25.** Myocardial infarction and coronary revascularization were the most common types of events, and the proportions of each event type within the treatment groups were similar. Patients randomized to alirocumab had numerically fewer non-fatal cardiovascular events of every type, except for heart failure requiring hospitalization.

**Table 25. Categories of non-fatal cardiovascular events**

| **Event, n (% of total)** | **Alirocumab (n=9,462)** | **Placebo (n=9,462)** | **Total (n=18,924)** |
|---|---|---|---|
| Myocardial infarction | 866 (39.6) | 994 (39.6) | 1860 (39.6) |
| Stroke | 131 (6.0) | 181 (7.2) | 312 (6.6) |
| Unstable angina requiring hospitalization | 37 (1.7) | 64 (2.5) | 101 (2.1) |
| Heart failure requiring hospitalization | 283 (12.9) | 276 (11.0) | 559 (11.9) |
| Ischemia-driven coronary revascularization procedure | 869 (39.8) | 998 (39.7) | 1867 (39.7) |
| **Total** | **2,186** | **2,513** | **4,699** |

The total non-fatal cardiovascular and death joint frailty model results for the overall study population and for LDL-C subgroups stratified at a baseline level of 100 mg/dL are shown in **Figure 24****.** Based on the interaction p values (p=0.02 for non-fatal events, p=0.03 for death), there was evidence of significant heterogeneity in the relative alirocumab treatment effects on both total non-fatal cardiovascular events and death, with the subgroup ≥100 mg/dL at randomization deriving greater relative benefit. This result is also consistent with observations in **Figures 23A and 23B****.**

**Table 26** summarizes the distributions of deaths and non-fatal cardiovascular events by ordinal event. There were 5,425 total deaths or non-fatal cardiovascular events, 77% greater than first events (n=3,064). While a majority of patients did not experience an event during the study, a sizable subset of patients experienced more than 1 event (1,261 patients). Among patients at risk for a first event in the alirocumab and placebo groups, death occurred as a first event in 2.2% and 2.5%, respectively. Notably, conditional on having a first non-fatal cardiovascular event, the risk of subsequent death was greater. After a first non-fatal cardiovascular event occurring an overall median of 1.0 (0.4, 1.7) year after randomization, death occurred as a second event in 5.7% and 5.0%, respectively, of the patients in the alirocumab and placebo groups. Similarly, after a second non-fatal cardiovascular event occurring an overall median of 1.2 (0.6, 2.0) years after randomization, death occurred as a third event in 6.2% and 6.6%, respectively, of the patients in the alirocumab and placebo groups. Qualitatively, these data indicate that each successive prior non-fatal cardiovascular event is associated with an increased subsequent risk for death. The joint frailty model confirms this observation with an association parameter (95% CI) of 2.04 (1.78 to 2.29), linking the risks of non-fatal cardiovascular events and death.

**Table 26: Distributions of death and adjudicated non-fatal cardiovascular events by event number**

| | | **Alirocumab** | | **Placebo** | |
|---|---|---|---|---|---|
| **First event** | | **n/N (%)** | **Median (Q1, Q3) event time** | **n/N (%)** | **Median (Q1, Q3) event time** |
| | Non-fatal cardiovascular | 1,230/9,462 (13.0) | 0.9 (0.4, 1.7) | 1,392/9,462 (14.7) | 1.0 (0.4, 1.8) |
| | Death | 207/9,462 (2.2) | 1.5 (0.7, 2.4) | 235/9,462 (2.5) | 1.5 (0.8, 2.3) |
| **Second event** | | | | | |
| | Non-fatal cardiovascular | 513/1,230 (41.7) | 1.2 (0.6, 2.0) | 608/1,392 (43.7) | 1.3 (0.6, 2.0) |
| | Death | 70/1,230 (5.7) | 1.4 (0.7, 2.4) | 70/1,392 (5.0) | 1.6 (1.0, 2.4) |

| **Third event** | | | | | |
|---|---|---|---|---|---|
| | Non-fatal cardiovascular | 188/513 (36.7) | 1.6 (1.0, 2.3) | 245/608 (40.3) | 1.5 (0.9, 2.4) |
| | Death | 32/513 (6.2) | 1.4 (1.0, 2.7) | 40/608 (6.6) | 1.4 (0.7, 2.4) |

| **Fourth and additional event(s)** | | | | | |
|---|---|---|---|---|---|
| | Non-fatal cardiovascular | 255 | | 268 | |
| | Death | 25 | | 47 | |

| **Total** | | | | | |
|---|---|---|---|---|---|
| | Non-fatal cardiovascular | 2,186 | | 2,513 | |
| | Death | 334 | | 392 | |

Baseline characteristics by groups defined by event frequency categories are summarized in **Table 27** below. Patients with at least one event were older, had higher baseline LDL-C, and were more likely to have comorbidities than patients without an event during the study, including diabetes, hypertension, and MI prior to the ACS index event. Comparing groups with at least one event, patients with multiple events or an only event of death had higher baseline LDL-C relative to patients with a single non-fatal event, and there were several differences in terms of comorbidities.

**Table 27: Baseline Characteristics of Patients by Categories of Number of Non-fatal Cardiovascular and Fatal Events**

| | **(A) No Events (n=15,860)** | **(B) Only Event = Death (n =442)** | **(C) Only Event = Non-fatal CV (n=1,361)** | **(D) Multiple Events (n =1,261)** | **p Value** | | | |
|---|---|---|---|---|---|---|---|---|
| | | | | | **(A) vs. (B) +(C)+(D)** | **(B) vs. (C)** | **(C) vs. (D)** | **(B) vs. (D)** |
| Age, years | 58 (51-65) | 63 (56-70) | 59 (52-66) | 61 (54-68) | <0.0001 | <0.0001 | 0.0004 | <0.0001 |
| Age category | | | | | <0.0001 | <0.0001 | 0.0003 | 0.01 |
| <65 years | 74.5 | 56.3 | 71.1 | 64.2 | | | | |
| 65 to <75 years | 20.8 | 31.5 | 22.5 | 26.6 | | | | |
| ≥75 years | 4.7 | 12.2 | 6.4 | 9.2 | | | | |
| Female sex | 24.9 | 25.1 | 27.9 | 26.1 | 0.03 | n.s. | n.s. | n.s. |
| Region | | | | | <0.0001 | <0.0001 | 0.0001 | <0.0001 |
| Western Europe | 22.3 | 11.3 | 22.6 | 22.1 | | | | |
| Eastern Europe | 29.3 | 36.0 | 25.2 | 22.5 | | | | |
| North America | 13.8 | 12.9 | 20.9 | 27.4 | | | | |
| South America | 13.9 | 20.6 | 12.3 | 10.6 | | | | |
| Asia | 12.8 | 10.4 | 9.6 | 6.3 | | | | |
| Rest of world | 7.9 | 8.8 | 9.4 | 11.1 | | | | |
| Index event | | | | | <0.0001 | 0.005 | 0.02 | 0.01 |
| NSTEMI | 47.4 | 52.6 | 52.8 | 57.8 | | | | |
| STEMI | 35.6 | 26.5 | 32.3 | 27.2 | | | | |
| Unstable angina | 17.0 | 20.8 | 14.9 | 15.0 | | | | |
| Time from index event to randomization , months | 2.7 (1.7-4.4) | 2.5 (1.7-3.6) | 2.6 (1.7-4.2) | 2.4 (1.6-3.9) | <0.0001 | n.s. | 0.03 | n.s. |
| Lipid-lowering therapy at Randomizatio n | | | | | <0.0001 | n.s. | 0.003 | 0.004 |
| High dose atorvastatin/ rosuvastatin | 89.3 | 88.7 | 86.5 | 85.9 | | | | |
| Other LLT | 10.0 | 9.9 | 12.1 | 11.2 | | | | |
| No LLT | 0.7 | 1.4 | 1.4 | 2.9 | | | | |
| LDL-C, mg/dL | 86 (73-103) | 91 (74-109) | 88 (73-107) | 92 (76-113) | <0.0001 | n.s. | 0.0007 | n.s. |
| LDL-C ≥100 mg/dL | 28.6 | 37.3 | 32.0 | 39.3 | <0.0001 | 0.04 | <0.0001 | n.s. |
| Diabetes status | | | | | <0.0001 | <0.0001 | n.s. | <0.0001 |
| Diabetes | 26.8 | 44.8 | 33.4 | 43.4 | | | | |
| Pre-diabetes | 44.6 | 34.4 | 42.0 | 36.0 | | | | |
| Normal | 28.7 | 20.8 | 24.5 | 20.6 | | | | |
| Smoking status | | | | | 0.02 | n.s. | n.s. | n.s. |
| Current | 24.0 | 22.9 | 24.5 | 25.5 | | | | |
| Former | 41.0 | 41.6 | 42.1 | 44.2 | | | | |
| Never | 35.0 | 35.5 | 33.4 | 30.4 | | | | |
| Medical history prior to index event | | | | | | | | |
| Hypertension | 62.4 | 77.2 | 73.2 | 80.7 | <0.0001 | n.s. | <0.0001 | n.s. |
| Myocardial infarction | 16.8 | 28.5 | 25.8 | 39.7 | <0.0001 | n.s. | <0.0001 | <0.0001 |
| Stroke | 2.6 | 7.5 | 5.7 | 6.8 | <0.0001 | n.s. | n.s. | n.s. |
| Malignant disease | 2.6 | 3.2 | 3.7 | 4.9 | <0.0001 | n.s. | n.s. | n.s. |
| COPD | 3.1 | 11.3 | 5.5 | 10.6 | <0.0001 | <0.0001 | <0.0001 | n.s. |
| GFR, mL/min per 1.73 m² | 78.8 (68.3-90.6) | 73.2 (59.9-87.3) | 76.6 (64.9-88.6) | 74.5 (60.0-87.3) | <0.0001 | n.s. | <0.0001 | n.s. |
| GFR <60 mL/min per 1.73 m² | 11.9 | 25.3 | 17.0 | 24.6 | <0.0001 | 0.0002 | <0.0001 | n.s. |

The Joint Frailty Model was also used to analyze different baseline LDL-C subgroups (see **Figure 25**). The interaction p values indicate significant heterogeneity in the relative treatment effects on total non-fatal cardiovascular events and death by baseline LDL-C subgroups. Among 5,629 patients with baseline LDL-C) levels ≥100 mg/dL, alirocumab reduced total non-fatal cardiovascular events and death by 255. In contrast, among 13,295 patients with baseline LDL-C <100 mg/dL, alirocumab reduced total non-fatal cardiovascular events and death by 130.

### CONCLUSION

In conclusion, in patients with ACS followed for a median of 28 months, the total number of deaths and non-fatal CV events prevented with alirocumab was twice the number of first events prevented. Alirocumab reduced non-fatal CV events and death in the presence of a strong association between non-fatal and fatal event risk. Lower achieved LDL-C was associated with fewer deaths.

### Example 8: Secondary Analysis - Effects of Alirocumab in Diabetic and Pre-Diabetic ACS Patients

An additional group of ACS patients of particular interest is those with diabetes and pre-diabetes, because a majority of patients with ACS have a glucometabolic abnormality. ACS patients with diabetes are at higher risk for recurrent ischemic cardiovascular events than ACS patients without diabetes, and derive greater absolute benefit from high-intensity statin therapy or ezetimibe plus statin treatment. In this pre-specified analysis, the CV efficacy and glucometabolic safety of alirocumab or placebo was compared among people with diabetes, pre-diabetes, or normoglycemia.

### a. Baseline Characteristics

At randomization, 5444 (28.8%) patients had diabetes, 8246 (43.6%) patients had prediabetes, and 5234 (27.7%) patients had normoglycemia. In each glycometabolic category, baseline characteristics were well-balanced between the alirocumab and placebo groups. The baseline characteristics of the normoglycemic, pre-diabetic, and diabetic patients are provided in **Table 28** and the baseline characteristics across glycemic strata by treatment group are provided in **Table 29.** Most patients underwent coronary revascularization for the index ACS event and received evidence-based treatment with dual antiplatelet agents, beta-blockers, and inhibitors of the renin-angiotensin system. Higher baseline values of body mass index, non-HDL cholesterol and triglycerides, and lower levels of HDL cholesterol were observed across glycometabolic strata. There was no gradient of LDL cholesterol level across glycometabolic strata.

**Table 28. Baseline Characteristics of Normoglycemic, Pre-Diabetic, and Diabetic Patients**

| | | ***Normoglycemia (n=5234)*** | ***Prediabetes (n=8246)*** | ***Diabetes (n=5444)*** |
|---|---|---|---|---|
| Age (years) | | 56 (50, 63) | 59 (52, 65) | 59 (53, 66) |
| Female sex | | 20.6% | 23.6% | 31.9% |
| BMI, kg/m², median (Q1, Q3)* | | 27 (25, 30) | 28 (25, 31) | 29 (26, 33) |
| Systolic BP - mmHg | | 125 (115, 135) | 126 (117, 137) | 130 (120, 140) |
| Diastolic BP - mmHg | | 78 (70, 83) | 79 (70, 83) | 79 (70, 84) |

| Index acute coronary syndrome event - no. (%) | | | | |
|---|---|---|---|---|
| | Non-ST-segment elevation myocardial infarction | 2478 (47.4) | 3921 (47.6) | 2776 (51.1) |
| | ST-segment elevation myocardial infarction | 1922 (36.8) | 2971(36.1) | 1643 (30.3) |
| | Unstable angina | 826 (15.8) | 1344 (16.3) | 1012 (18.6) |
| NSTEMI/STEMI/UA | | 47.4/36.8/15.8% | 47.6/36.1/16.3% | 51.1/30.3/18.6% |
| Laboratory values, median (Q1, Q3) | | | | |
| LDL-C, mg/dL | | 86 (74, 104) | 88 (74, 104) | 85 (71, 104) |
| Non-HDL-C, mg/dL | | 112 (97, 134) | 115 (100, 136) | 117 (101, 140) |
| HDL-C, mg/dL | | 44 (38, 52) | 43 (37, 51) | 41 (35, 48) |
| Triglycerides, mg/dL | | 117 (87, 164) | 127 (93, 177) | 147 (106, 205) |
| A1c, % | | 5.4 (5.3, 5.5) | 5.9 (5.7, 6.0) | 7.0 (6.5, 8.2) |
| Fasting glucose, mg/dL | | 93.7 (88.3, 99.1) | 100.9 (93.7, 108.1) | 133.3 (111.7, 169.4) |
| Duration of follow-up - months | | 2.9 (2.3, 3.5) | 2.8 (2.3, 3.4) | 2.7 (2.3, 3.4) |
| Eligible for ≥3 years of follow-up - no. (%) | | 2405 (45.9) | 3550 (43.1) | 2287 (42.0) |

| Cardiac intervention - no. (%) | | | | |
|---|---|---|---|---|
| | Percutaneous coronary intervention for index acute coronary syndrome | 3929 (75.1) | 6009 (72.9) | 3739 (68.7) |
| | Prior coronary artery bypass graft | 228 (4.4) | 395 (4.8) | 424 (7.8) |

| Cardiac medication - no. (%) | | | | |
|---|---|---|---|---|
| | Beta-blocker | 4285 (81.9) | 7031 (85.3) | 4674 (85.9) |
| | Angiotensin-converting enzyme inhibitor or angiotensin receptor blocker | 3832 (73.2) | 6427 (77.9) | 4457 (81.9) |
| | Aspirin | 5048 (96.4) | 7877 (95.5) | 5161 (94.8) |
| | P2Y₁₂ inhibitor | 4633 (88.5) | 7314 (88.7) | 4594 (84.4) |

| Diabetes mediation* - no. (%) | | | | |
|---|---|---|---|---|
| | Insulin | 0 | 0 | 1268 (23.3) |
| | Injectable diabetes medications other than insulin | 0 | 0 | 47 (0.9) |
| Oral diabetes medications | 4 (0.1) | 39 (0.5) | 3276 (60.2) | |

| | | | | |
|---|---|---|---|---|
| NSTEMI: Non ST-segment elevation myocardial infarction; STEMI: ST-segment elevation myocardial infarction; UA: unstable angina. | | | | |

**Table 29. Baseline Characteristics Across Glycemic Strata By Treatment Group**

| | | **Normoglycemia (N=5234)** | | **Prediabetes (N=8246)** | | **Diabetes (N=5444)** | |
|---|---|---|---|---|---|---|---|
| | | **Alirocumab (N=2639)** | **Placebo (N=2595)** | **Alirocumab (N=4130)** | **Placebo (N=4116)** | **Alirocumab (N=2693)** | **Placebo (N=2751)** |
| Age - yr | | 56 (50-63) | 56 (50-63) | 59 (52-65) | 59 (52-65) | 59 (53-66) | 59 (53-66) |
| Female sex - no. (%) | | 538 (20.4) | 540 (20.8) | 996 (24.1) | 952 (23.1) | 856 (31.8) | 880 (32.0) |

| Index acute coronary syndrome event - no. (%) | | | | | | | |
|---|---|---|---|---|---|---|---|
| | ST-segment elevation myocardial infarction | 977 (37.1) | 945 (36.5) | 1507 (36.6) | 1464 (35.6) | 817 (30.4) | 826 (30.1) |
| | Non-ST-segment elevation myocardial infarction | 1240 (47.1) | 1238 (47.8) | 1957 (47.5) | 1964 (47.7) | 1377 (51.3) | 1399 (51.0) |
| | Unstable angina | 418 (15.9) | 408 (15.8) | 658 (16.0) | 686 (16.7) | 492 (18.3) | 520 (18.9) |

| Laboratory values at randomization* | | | | | | | |
|---|---|---|---|---|---|---|---|
| | LDL cholesterol - mg/dL | 86 (73-103) | 87 (74-104) | 88 (75-105) | 87 (74-104) | 85 (71-103) | 85 (71-105) |
| | Non-HDL cholesterol - mg/dL | 112 (97-134) | 113 (98-134) | 116 (100-136) | 115 (99-137) | 117 (100-140) | 117 (102-141) |
| | Total cholesterol - mg/dL | 158 (141-182) | 160 (142-181) | 161 (144-183) | 160 (142-183) | 159 (141-184) | 159 (141-184) |
| | HDL cholesterol - mg/dL | 44 (38-53) | 44 (37-52) | 43 (37-51) | 43 (37-50) | 41 (35-48) | 41 (35-47) |
| | Triglycerides - mg/dL | 116 (86-161) | 118 (87-166) | 127 (93-177) | 127 (93-177) | 147 (106-205) | 147 (105-204) |
| | HbA1c - % | 5.4 (5.3-5.5) | 5.4 (5.3-5.5) | 5.9 (5.7-6.0) | 5.9 (5.7-6.0) | 7.0 (6.5-8.3) | 7.0 (6.5-8.2) |
| | Fasting glucose - mg/d L | 93.6 (88.2-99.0) | 93.6 (88.2-97.2) | 100.8 (93.6-108.0) | 100.8 (93.6-109.8) | 133.2 (111.6-167.4) | 131.4 (109.8-169.2) |

### b. Cardiovascular Events in the Placebo Group by Glycometabolic Status

After 16 weeks of treatment (intent-to-treat population), the levels of LDL-C, non-HDL-C, HDL-C, and triglyceride were similar between the three normoglycemic, pre-diabetic, and diabetic patient subgroups in both the alirocumab arm and the placebo arm. In the placebo group, the rate of the primary endpoint was 6.5, 3.4, and 3.1 per 100 person-years, respectively, for those with diabetes, prediabetes, and normoglycemia at baseline **(****Figure 26****).** In the placebo group, despite having similar lipid parameters, the diabetic patients had an increased incidence of MACE. The corresponding unadjusted hazard ratios for the primary endpoint among those with diabetes versus normoglycemia and prediabetes versus normoglycemia were 2.09 (95% CI, 1.78 to 2.46) and 1.10 (95% CI, 0.93 to 1.30), respectively **(****Figure 27****).** Analogous data for individual components of the primary endpoint are provided in **Table 30.**

**Table 30. Hazard Ratios for Individual Components of Primary Endpoint in the Placebo Group**

| | **Diabetes vs prediabetes** | **Diabetes vs normal** |
|---|---|---|
| **MACE:** | HR 1.90 (95% CI 1.65, 2.17); P<0.0001 | HR 2.09 (95% CI 1.78, 2.46); P<0.0001 |
| **Coronary heart disease death:** | HR 2.14 (95% CI 1.60, 2.88); P<0.0001 | HR 2.54 (95% CI 1.77, 3.64); P<0.0001 |
| **Nonfatal myocardial infarction:** | HR 1.88 (1.59, 2.21); P<0.0001 | HR 1.98 (95% CI 1.63, 2.40); P<0.0001 |
| **Ischemic stroke:** | HR 2.10 (95% CI 1.48, 2.99); P<0.0001 | HR 2.89 (95% CI 1.84, 4.56); P<0.0001 |
| **Unstable angina:** | HR 1.75 (95% CI 0.98, 3.15); P=0.0573 | HR 1.56 (95% CI 0.82, 2.98); P=0.1720 |

| | | |
|---|---|---|
| HR denotes hazard ratio; MACE, major adverse cardiovascular event; MI, myocardial infarction; UA, unstable angina; Q, quartile. | | |

### c. Effect of Alirocumab on Lipids

**Figure 26** shows the median changes in lipid parameters at month 4 in each treatment group, stratified by baseline glycometabolic category. Changes from baseline in LDL-C at month 4 were similar in each glycometabolic category (median -64% to -65% with alirocumab; +1.0% to 0% with placebo).

In alirocumab-treated patients, median (quartile 1, quartile 3) LDL-C values at month 4 were 31 mg/dL (20, 47), 31 mg/dL (21, 49), and 31 mg/dL (21, 50) among patients with diabetes, prediabetes, and normoglycemia, respectively, compared with 87 mg/dL (71, 107), 87 mg/dL (72, 107), and 88 mg/dL (73, 107) among patients allocated to placebo.

### d. Effect of Alirocumab on Endpoint Events According to Glucometabolic Status

**Table 31** shows a pre-specified analysis of incidence of MACE by assigned treatment and baseline glucometabolic status. Alirocumab produced a similar relative reduction in risk of the primary endpoint in patients with diabetes (HR, 0.84; 95% CI, 0.74 to 0.97), prediabetes (HR, 0.86; 95% CI, 0.74 to 1.00), and normoglycemia (HR, 0.85; 95% CI, 0.70 to 1.03), with no significant interaction of glycometabolic state and treatment. However, among the patients with diabetes, at baseline the risk of recurrent ischemic events after ACS was high, despite intensive statin treatment. As such, the substantially higher absolute risk at baseline among patients with diabetes resulted in greater absolute risk reduction with alirocumab treatment (-2.3%) compared to those with prediabetes (-1.2%) or normoglycemia (-1.2%; P_{interaction}=0.0019) **(Table 31).** Therefore, patients with recent ACS and diabetes derived the greatest absolute benefit from alirocumab added to maximum-tolerated statin.

**Table 31. Pre-Specified Analyses of Diabetic, Pre-diabetic, and Normoglycemic Patients**

| **Category** | **N (% of cohort)** | **MACE cumulative incidence** | | **Absolute risk reduction (95%CI)** | **Relative risk reduction (hazard ratio) (95% CI)** |
|---|---|---|---|---|---|
| | | **Alirocumab n/N (%)** | **Placebo n/N (%)** | | |
| All subjects | 18,924 (100) | 903/9462 (9.5) | 1052/9462 (11.1) | 1.6% (0.7%, 2.4%) | 0.85 (0.78, 0.93) |
| Diabetes | 5444 (28.8) | 380/2693 (14.1) | 452/2751 (16.4) | 2.3% (0.4%, 4.2%) | 0.84 (0.74, 0.97) |
| Pre-diabetes | 8246 (43.6) | 331/4130 (8.0) | 380/4116 (9.2) | 1.2% (0%, 2.4%) | 0.86 (0.74, 1.00) |
| Normoglycemia | 5234 (27.7) | 192/2639 (7.3) | 220/2595 (8.5) | 1.2% (-0.3%, 2.7%) | 0.85 (0.70, 1.03) |

| | | | | | |
|---|---|---|---|---|---|
| Median follow-up: 34 months. ARR: absolute risk reduction; NA: not applicable. | | | | | |

### e. Glycemic Safety of Alirocumab

**Figure 28** shows the effects of alirocumab on HbA1c, fasting glucose, and incidence of new-onset diabetes in patients without diabetes at baseline, and separately in patients with prediabetes or normoglycemia. Among patients without diabetes at baseline, alirocumab treatment resulted in a lower mean HbA1c (5.78% vs. 5.80%, P=0.0008), a similar mean fasting glucose (5.67 vs. 5.68 mmol/L, P=0.84), and no excess risk of new-onset diabetes (HR, 1.00; 95% CI, 0.89 to 1.11) compared with placebo. The results were similar when considering the separate categories of prediabetes or normoglycemia at baseline. As such, no evidence of increased fasting serum glucose or HbA1c was observed with alirocumab compared with placebo. Furthermore, no overall increase in new-onset diabetes, a pre-specified safety endpoint, was observed with alirocumab compared with placebo over the duration of this study (see **Figure 28** and **Table 4**).

### CONCLUSION

In a pre-specified analysis of diabetic, pre-diabetic, and normoglycemic patients with recent ACS, the greatest absolute benefit from alirocumab added to maximum-tolerated statins was derived in patients with recent ACS and diabetes. For these patients with recent ACS and diabetes, treatment with alirocumab targeting LDL-C levels 25-50 mg/dl provides approximately twice the absolute reduction in cardiovascular events among patients with diabetes versus patients with prediabetes or normoglycemia. With a median follow-up of 2.8 years and with 2405 patients without diabetes eligible for 3-5 years of follow-up, alirocumab did not adversely affect measures of glycemia (HbA1c, fasting glucose) or increase the risk of new-onset diabetes.

### Example 9: Secondary Analysis - Effects of Alirocumab in Patients with Peripheral Artery Disease, Cerebrovascular Disease, or both

Patients with atherosclerosis evident in more than one vascular bed have a high risk of major adverse cardiovascular events (MACE) and death. A secondary analysis of the ODYSSEY OUTCOMES trial analyzed whether the benefit of alirocumab was influenced by the presence of polyvascular disease, defined as coronary artery disease with concurrent peripheral artery disease (PAD), cerebrovascular disease (CeVD), or both. Median follow-up was 2.8 years.

### a. Baseline Characteristics

In this analysis, three subgroups of patients with recent ACS were defined based upon the distribution of other evident vascular disease: 1) monovascular disease (coronary artery disease without known PAD or CeVD); 2) polyvascular disease in two vascular beds (coronary artery disease and either PAD or CeVD); and 3) polyvascular disease in three vascular beds (coronary artery disease with both PAD and CeVD). An additional sensitivity analysis was performed with four subgroups of patients with ACS: 1) neither PAD nor CeVD; 2) PAD, with or without concurrent CeVD; 3) CeVD, with or without concurrent PAD; and 4) both PAD and CeVD. PAD included arterial disease of the extremities or abdominal aortic aneurysm. CeVD was defined as a history of carotid endarterectomy, carotid stenting, prior stroke or transient ischemic attack.

**Table 32** summarizes the baseline characteristics of patients with monovascular (coronary artery) disease, polyvascular disease in two beds (split by PAD only and CeVD only), and polyvascular disease in three beds. At baseline, 17,370 patients had monovascular disease (91.8%), 1,405 patients had polyvascular disease in two vascular beds (7.4%; 3.2% only PAD and 4.2% only CeVD), and 149 had polyvascular disease in three vascular beds (0.8%). Compared to patients with monovascular disease, those with coronary artery disease and PAD, coronary artery disease and CeVD, and three-bed polyvascular disease were older (median ages 58, 62, 62, and 66 years, p<0.0001); those with coronary and PAD or coronary and CeVD were somewhat more likely to be female (26.7% and 33.2%, respectively) than those with monovascular disease (24.7%, p<0.0001). Of all patients with CeVD, 526 (66.2%) had a history of stroke. Patients with polyvascular disease in three beds had more comorbidities including a history of hypertension, myocardial infarction and coronary artery bypass grafting, compared to patients with monovascular (coronary) disease (all p<0.0001). Furthermore, patients with polyvascular disease in three beds versus patients with monovascular disease had a higher prevalence of diabetes (43.6% vs. 27.7%; p<0.0001) and were more likely to be current or former smokers (81.9% vs. 64.9%; p<0.0001). More patients with polyvascular disease in three beds versus patients with monovascular disease had a glomerular filtration rate (GFR) of <60ml/min/1.73m² (39.6% vs. 12.3%) with median GFRs of 78.5, 73.5, 72.3, and 67.0 ml/min/1.73m² in patients with monovascular (coronary) disease, coronary and PAD, coronary and CeVD, and polyvascular disease in three beds, respectively (p<0.0001). **Table 33** shows the baseline characteristics for the four overlapping vascular groups based on PAD or CeVD.

**Table 32. Baseline Characteristics by History of PAD or CeVD Category**

| | | **Monovascular Disease (coronary without PAD or CeVD) (n=17,370)** | **Disease in Two Vascular Beds (coronary and PAD) (n=610)** | **Disease in Two Vascular Beds (coronary and CeVD) (n=795)** | **Disease in Three Vascular Beds (coronary, PAD, and CeVD) (n=149)** | **p Value** |
|---|---|---|---|---|---|---|
| Age, yrs | | 58 (51,65) | 62 (56, 68) | 62 (56, 69) | 66 (60, 71) | <0.0001 |
| Age category | | | | | | <0.0001 |
| | <65 yrs | 12,956 (74.6) | 368 (60.3) | 456 (57.4) | 60 (40.3) | |
| | 65 to <75 yrs | 3,575 (20.6) | 178 (29.2) | 252 (31.7) | 72 (48.3) | |
| | ≥75 yrs | 839 (4.8) | 64 (10.5) | 87 (10.9) | 17 (11.4) | |
| Female | | 4,298 (24.7) | 163 (26.7) | 264 (33.2) | 37 (24.8) | <0.0001 |
| Region | | | | | | <0.0001 |
| | Western Europe | 3,852 (22.2) | 152 (24.9) | 142 (17.9) | 29 (19.5) | |
| | Eastern Europe | 4,993 (28.7) | 189 (31.0) | 215 (27.0) | 40 (26.8) | |
| | North America | 2,513 (14.5) | 134 (22.0) | 170 (21.4) | 54 (36.2) | |
| | South America | 2,413 (13.9) | 64 (10.5) | 101 (12.7) | 10 (6.7) | |
| | Asia | 2,170 (12.5) | 22 (3.6) | 92 (11.6) | 9 (6.0) | |
| | Rest of world | 1,429 (8.2) | 49 (8.0) | 75 (9.4) | 7 (4.7) | |
| Index event | | | | | | <0.0001 |
| | NSTEMI | 8,300 (47.9) | 342 (56.3) | 439 (55.4) | 94 (63.1) | |
| | STEMI | 6,080 (35.1) | 195 (31.1) | 227 (28.6) | 34 (22.8) | |
| | Unstable angina | 2,963 (17.1) | 71 (11.7) | 127 (16.0) | 21 (14.1) | |
| Time from index event to randomization, months | | 2.6 (1.7, 4.3) | 3.0 (1.8, 5.4) | 2.7 (1.7, 4.8) | 3.0 (2.1, 3.9) | 0.0003 |
| Lipid-lowering therapy at randomization | | | | | | <0.0001 |
| | High-dose atorvastatin/ rosuvastatin | 15,486 (89.2) | 525 (86.1) | 679 (85.4) | 121 (81.2) | |
| | Other LLT | 1,734 (10.0) | 75 (12.3) | 102 (12.8) | 24 (16.1) | |
| | No LLT | 150 (0.9) | 10 (1.6) | 14 (1.8) | 4 (2.7) | |
| LDL-C, mg/dl | | 86 (73, 103) | 91 (76, 108) | 90 (75, 109) | 95 (80, 115) | <0.0001 |
| LDL-C ≥100 mg/dl | | 5,060 (29.1) | 218 (35.7) | 290 (36.5) | 61 (40.9) | <0.0001 |
| HDL-C, mg/dl | | 42 (36, 50) | 42 (36, 50) | 43 (36, 51) | 43 (37, 51) | NS |
| Non-HDL-C, mg/dl | | 114 (99, 136) | 121 (105, 143) | 120 (103, 144) | 124 (108, 143) | <0.0001 |
| Triglycerides, mg/dl | | 128 (94, 181) | 134 (99, 187) | 136 (98, 190) | 135 (94, 182) | 0.002 |
| Apolipoprotein B, mg/dl | | 79 (69, 93) | 83 (72, 96) | 83 (71, 96) | 82 (75, 95) | <0.0001 |
| Lipoprotein(a), mg/dl | | 20.8 (6.6, 59.4) | 25.5 (7.5, 68.1) | 23.0 (7.1, 61.7) | 29.4 (9.4, 74.5) | 0.004 |
| C-reactive protein, mg/dl | | 0.16 (0.08, 3.73) | 0.26 (0.11, 0.55) | 0.22 (0.10, 0.48) | 0.21 (0.11, 0.49) | <0.0001 |
| Body mass index, kg/m² | | 27.9 (25.2, 31.1) | 27.7 (24.9, 31.0) | 28.1 (25.4, 31.5) | 27.7 (24.5, 30.7) | NS |
| HbA1c, % | | 5.8 (5.5, 6.3) | 6.0 (5.6, 6.7) | 6.1 (5.7, 7.0) | 6.0 (5.7, 6.7) | <0.0001 |
| GFR, ml/min per 1.73 m² | | 78.5 (68.1, 90.4) | 74.1 (61.6, 86.7) | 72.9 (59.5, 85.8) | 67.0 (52.2, 84.4) | <0.0001 |
| GFR <60 ml/min per 1.73 m² | | 2139 (12.3) | 135 (22.1) | 206 (25.9) | 59 (39.6) | <0.0001 |
| Diabetes status | | | | | | <0.0001 |
| | Diabetes | 4,805 (27.7) | 225 (36.9) | 349 (43.9) | 65 (43.6) | |
| | Pre-diabetes | 7,630 (43.9) | 260 (42.6) | 299 (37.6) | 57 (38.3) | |
| | Normoglycemia | 4,935 (28.4) | 125 (20.5) | 147 (18.5) | 27 (18.1) | |
| Smoking status | | | | | | <0.0001 |
| | Current | 4,181 (24.1) | 189 (31.0) | 147 (18.5) | 43 (28.9) | |
| | Former | 7,095 (40.8) | 302 (49.5) | 335 (42.1) | 79 (53.0) | |
| | Never | 6,093 (35.1) | 119 (19.5) | 313 (39.4) | 27 (18.1) | |

| Medical history prior to index event | | | | | | |
|---|---|---|---|---|---|---|
| | Hypertension | 10,930 (62.9) | 489 (80.2) | 694 (87.3) | 136 (91.3) | <0.0001 |
| | Myocardial infarction | 3,147 (18.1) | 204 (33.4) | 226 (28.4) | 62 (41.6) | <0.0001 |
| | Stroke | 0 | 0 | 526 (66.2) | 85 (57.0) | <0.0001 |
| | Malignant disease | 458 (2.6) | 28 (4.6) | 34 (4.3) | 12 (8.1) | <0.0001 |
| | COPD | 613 (3.5) | 64 (10.5) | 46 (5.8) | 23 (15.4) | <0.0001 |
| | CABG | 826 (4.8) | 82 (13.4) | 91 (11.4) | 48 (32.2) | <0.0001 |
| | PAD | 0 | 610 (100) | 0 | 149 (100) | <0.0001 |
| | CeVD | 0 | 0 | 795 (100) | 149 (100) | <0.0001 |
| Revascularization for index event | | 12,596 (72.5) | 436 (71.5) | 540 (67.9) | 105 (70.5) | 0.04 |

| Medications | | | | | | |
|---|---|---|---|---|---|---|
| | Aspirin | 16,647 (95.8) | 564 (92.5) | 737 (92.7) | 138 (92.6) | <0.0001 |
| | P2Y₁₂ antagonist | 15,223 (87.6) | 525 (86.1) | 664 (83.5) | 129 (86.6) | 0.005 |
| | ACE inhibitor/ARB | 13,444 (77.4) | 494 (81.0) | 655 (82.4) | 123 (82.6) | 0.0008 |
| | Beta-blocker | 14,687 (84.6) | 507 (83.1) | 672 (84.5) | 124 (83.2) | NS |
| | Ezetimibe | 473 (2.7) | 38 (6.2) | 30 (3.8) | 13 (8.7) | <0.0001 |
| Treatment Variables Among Alirocumab Treated Patients | | n=8,683 | n=302 | n=406 | n=71 | |
| | % switched to placebo | 691 (8.0) | 12 (4.0) | 25 (6.2) | 2 (2.8) | 0.01 |

| | | | | | | |
|---|---|---|---|---|---|---|
| Values are median (quartile 1, quartile 3) or n (%). NS = p > 0.05. CABG = coronary artery bypass graft; CeVD = cerebrovascular disease; COPD = chronic obstructive pulmonary disease; CV = cardiovascular; GFR = glomerular filtration rate; LDL-C = low-density lipoprotein cholesterol; LLT = lipid-lowering therapy; NSTEMI = non-ST-elevation myocardial infarction; PAD = peripheral artery disease; STEMI = ST-elevation myocardial infarction. | | | | | | |

**Table 33. Baseline Characteristics by History of PAD or CeVD Category**

| | | **No PAD or CeVD (N=17,370)** | **PAD (including CeVD) (N=759)** | **CeVD (including PAD) (N=944)** | **PAD and CeVD (N=149)** |
|---|---|---|---|---|---|
| Age, yrs | | 58 (51, 65) | 62 (57, 69) | 63 (57, 70) | 66 (60, 71) |

| Age category | | | | | |
|---|---|---|---|---|---|
| | <65 yrs | 12,956 (74.6) | 428 (56.4) | 516 (54.7) | 60 (40.3) |
| | 65 to <75 yrs | 3,575 (20.6) | 250 (32.9) | 324 (34.3) | 72 (48.3) |
| | ≥75 yrs | 839 (4.8) | 81 (10.7) | 104 (11.0) | 17 (11.4) |
| Female | | 4,298 (24.7) | 200 (26.4) | 301 (31.9) | 37 (24.8) |

| Region | | | | | |
|---|---|---|---|---|---|
| | Western Europe | 3,852 (22.2) | 181 (23.8) | 171 (18.1) | 29 (19.5) |
| | Eastern Europe | 4,993 (28.7) | 229 (30.2) | 255 (27.0) | 40 (26.8) |
| | North America | 2,513 (14.5) | 188 (24.8) | 224 (23.7) | 54 (36.2) |
| | South America | 2,413 (13.9) | 74 (9.7) | 111 (11.8) | 10 (6.7) |
| | Asia | 2,170 (12.5) | 31 (4.1) | 101 (10.7) | 9 (6.0) |
| | Rest of world | 1,429 (8.2) | 56 (7.4) | 82 (8.7) | 7 (4.7) |

| Index event | | | | | |
|---|---|---|---|---|---|
| | NSTEMI | 8,300 (47.9) | 533 (56.6) | 436 (57.6) | 94 (63.1) |
| | STEMI | 6,080 (35.1) | 261 (27.7) | 229 (30.3) | 34 (22.8) |
| | Unstable angina | 2,963 (17.1) | 148 (15.7) | 92 (12.2) | 21 (14.1) |
| Time from index event to randomization, months | | 2.6 (1.7, 4.3) | 3.0 (1.9, 5.2) | 2.8 (1.8, 4.7) | 3.0 (2.1, 3.9) |

| Lipid-lowering therapy at Randomization | | | | | |
|---|---|---|---|---|---|
| | High-dose atorvastatin or rosuvastatin | 15,486 (89.2) | 646 (85.1) | 800 (84.7) | 121 (81.2) |
| | Other LLT | 1,734 (10.0) | 99 (13.0) | 126 (13.3) | 24 (16.1) |
| | No LLT | 150 (0.9) | 14 (1.8) | 18 (1.9) | 4 (2.7) |
| LDL-C, mg/dL | | 86 (73, 103) | 92 (77, 109) | 91 (76, 110) | 95 (80, 115) |
| LDL-C ≥100 mg/dL | | 5,060 (29.1) | 279 (36.8) | 351 (37.2) | 61 (40.9) |
| HDL-C, mg/dL | | 42 (36, 50) | 42 (36, 50) | 43 (36, 51) | 43 (37, 51) |
| Non-HDL-C, mg/dL | | 114 (99, 136) | 121 (106, 143) | 121 (104, 144) | 124 (108, 143) |
| Triglycerides, mg/dL | | 128 (94, 181) | 134 (98, 186) | 136 (97, 190) | 135 (94, 182) |
| Apolipoprotein B, mg/dL | | 79 (69, 93) | 83 (73, 96) | 83 (72, 96) | 82 (75, 95) |
| Lipoprotein(a), mg/dL | | 20.8 (6.6, 59.4) | 26.3 (7.6, 69.9) | 23.6 (7.3, 63.4) | 29.4 (9.4, 74.5) |
| C-reactive protein, mg/dL | | 0.16 (0.08, 3.73) | 0.25 (0.11, 0.53) | 0.22 (0.10, 0.48) | 0.21 (0.11, 0.49) |
| Body mass index, kg/m² | | 27.9 (25.2, 31.1) | 27.7 (24.9, 30.9) | 28.1 (25.2, 31.3) | 27.7 (24.5, 30.7) |
| HbA1c, % | | 5.8 (5.5, 6.3) | 6.0 (5.7, 6.7) | 6.1 (5.7, 7.0) | 6.0 (5.7, 6.7) |
| GFR, ml/min per 1.73 m² | | 78.5 (68.1, 90.4) | 73.5 (59.1, 85.9) | 72.3 (57.0, 85.5) | 67.0 (52.2, 84.4) |
| GFR <60 ml/min per 1.73 m² | | 2,139 (12.3) | 194 (25.6) | 265 (28.1) | 59 (39.6) |
| Diabetes status | | | | | |
| | Diabetes | 4,805 (27.7) | 290 (38.2) | 414 (43.9) | 65 (43.6) |
| | Pre-diabetes | 7,630 (43.9) | 317 (41.8) | 356 (37.7) | 57 (38.3) |
| | Normoglycemia | 4,935 (28.4) | 152 (20.0) | 174 (18.4) | 27 (18.1) |
| Smoking status | | | | | |
| | Current | 4,181 (24.1) | 232 (30.6) | 190 (20.1) | 43 (28.9) |
| | Former | 7,095 (40.8) | 381 (50.2) | 414 (43.9) | 79 (53.0) |
| | Never | 6,093 (35.1) | 146 (19.2) | 340 (36.0) | 27 (18.1) |
| Medical history prior to index event | | | | | |
| | Hypertension | 10,930 (62.9) | 625 (82.3) | 830 (87.9) | 136 (91.3) |
| | Myocardial infarction | 3,147 (18.1) | 266 (35.0) | 288 (30.5) | 62 (41.6) |
| | Stroke | 0 | 85 (11.2) | 611 (64.7) | 85 (57.0) |
| | Malignant disease | 458 (2.6) | 40 (5.3) | 46 (4.9) | 12 (8.1) |
| | COPD | 613 (3.5) | 87 (11.5) | 69 (7.3) | 23 (15.4) |
| | CABG | 826 (4.8) | 130 (17.1) | 139 (14.7) | 48 (32.2) |
| | PAD | 0 | 759 (100) | 149 (15.8) | 149 (100) |
| | CeVD | 0 | 149 (19.6) | 944 (100) | 149 (100) |
| Revascularization for index event | | 12,596 (72.5) | 541 (71.3) | 645 (68.3) | 105 (70.5) |
| Medications | | | | | |
| | Aspirin | 16,647 (95.8) | 702 (92.5) | 875 (92.7) | 138 (92.6) |
| | P2Y₁₂ antagonist | 15,223 (87.6) | 654 (86.2) | 793 (84.0) | 129 (86.6) |
| | ACE inhibitor/ARB | 13,444 (77.4) | 617 (81.3) | 778 (82.4) | 123 (82.6) |
| | Beta-blocker | 14,687 (84.6) | 631 (83.1) | 796 (84.3) | 124 (83.2) |
| | Ezetimibe | 473 (2.7) | 51 (6.7) | 43 (4.6) | 13 (8.7) |
| Treatment variables among alirocumab treated patients | | n=8,683 | n=477 | n=373 | n=71 |
| | % switched to placebo | 691 (8.0) | 27 (5.7) | 14 (3.8) | 2 (2.8) |

| | | | | | |
|---|---|---|---|---|---|
| Values are median (quartile 1, quartile 3) or column percentages. CABG, coronary artery bypass graft; CeVD, cerebrovascular disease; COPD, chronic obstructive pulmonary disease; GFR, glomerular filtration rate; LDL-C, low-density lipoprotein cholesterol; LLT, lipid-lowering therapy; NSTEMI, non-ST-elevation myocardial infarction; PAD, peripheral artery disease; STEMI, ST-elevation myocardial infarction. | | | | | |

### b. LDL-C Lowering

At baseline, median LDL-C (quartile 1, quartile 3) was higher in patients with polyvascular disease, with values of 86 (73, 103) in patients with monovascular (coronary) disease, 91 (76, 108) in coronary and PAD disease, 90 (75, 109) in coronary and CeVD, and 95 mg/dl (80, 115) with polyvascular disease in three beds (p<0.0001). In the placebo group, median LDL-C at 4 months was 87 (72, 106) in patients with monovascular disease; 90 (73, 108) in only PAD; 90 (73, 115) in only CeVD; and 93 mg/dl (78, 118) in polyvascular disease in three beds. In patients treated with alirocumab, median LDL-C at 4 months was 30 (20, 47); 34 (23, 50); 34 (21, 52); and 31 (20, 42) in the same four vascular disease categories.

### c. Primary MACE Endpoint and All-Cause Death

Overall in the ODYSSEY OUTCOMES trial, the incidence of MACE in the placebo and alirocumab groups was 11.1% and 9.5%, respectively, with a corresponding absolute risk reduction (ARR) of 1.6% (95% confidence interval [Cl], 0.7%, 2.4%; p=0.0003) (13). **Figure 29A** shows that this overall efficacy on MACE reflects a gradient of absolute risk and ARR according to the number of diseased vascular beds. For patients in the placebo group with one, two, or three diseased vascular beds, the incidence of MACE was 10.0%, 22.2%, and 39.7%, respectively. The corresponding ARR with alirocumab was 1.4% (0.6%, 2.3%), 1.9% (-2.4%, 6.2%), and 13.0% (-2.0, 28.0%), with a 3-way interaction p=0.0012. The corresponding numbers needed to treat (NNT) to prevent one MACE are 64 for all patients, 69 for patients with monovascular (coronary) disease, 53 for patients with polyvascular disease in two beds, and 8 for patients with polyvascular disease in three beds.

The overall incidence of all-cause death in the placebo and alirocumab groups was 4.1% and 3.5%, respectively, with a corresponding ARR of 0.6% (95% CI 0.2%, 1.2%). Similar to MACE, Figure 29B shows that the overall efficacy on all-cause death reflects a gradient of absolute risk and ARR with alirocumab according to the number of diseased vascular beds. In the placebo group, the incidence of death with one, two, or three diseased vascular beds was 3.5%, 10.0%, and 21.8%, respectively. With alirocumab, the corresponding ARR was 0.4% (-0.1, 1.0), 1.3% (-a1.8%, 4.3%), and 16.2% (5.5, 26.8), with a 3-way interaction p=0.0025. The corresponding NNT to prevent one death were 163 for all patients, 236 for patients with monovascular (coronary) disease, 78 for patients with polyvascular disease in two beds, and 6 for patients with polyvascular disease in three beds.

Details of primary MACE endpoint and all-cause death are shown in **Table 34** and **Figure 30****,** including the total number of events with corresponding HR and ARR of alirocumab versus placebo for primary endpoint and all-cause death for patients with monovascular (coronary) disease, polyvascular disease in two beds (split by PAD or CeVD) and polyvascular disease in three beds. Table 35 shows these details for the four overlapping vascular groups based on PAD or CeVD.

**Table 34. Primary MACE Endpoint and All-Cause Death by History of PAD or CeVD Category**

| | | **Alirocumab n/N (%)** | **Placebo n/N (%)** | **HR (95% CI)** | **HR 3-Way Interaction p Value** | **ARR (95% CI)** | **ARR 3-Way Interaction p Value** | **NNT*** |
|---|---|---|---|---|---|---|---|---|
| Primary composite | | | | | | | | |
| | Monovascular disease (coronary without PAD or CeVD) | 740/8,683 (8.5) | 866/8,687 (10.0) | 0.84 (0.76, 0.93) | 0.0153 | 1.4 (0.6, 2.3) | 0.0012 | 69 |
| | Disease in two vascular beds (coronary and PAD) | 69/302 (22.8) | 73/308 (23.7) | 0.98 (0.72, 1.34) | | 0.9 (-5.9, 7.6) | | 117 |
| | Disease in two vascular beds (coronary and CeVD) | 75/406 (18.5) | 82/389 (21.1) | 0.92 (0.68, 1.24) | | 2.6 (-2.9, 8.2) | | 38 |
| | Disease in three vascular beds (coronary, PAD, and CeVD) | 19/71 (26.8) | 31/78 (39.7) | 0.54 (0.32, 0.89) | | 13.0 (-2.0, 28.0) | | 8 |
| | All patients | 903/9,462 (9.5) | 1,052/9,462 (11.1) | 0.85 (0.78, 0.93) | | 1.6 (0.7, 2.4) | | 64 |

| All-cause death | | | | | | | | |
|---|---|---|---|---|---|---|---|---|
| | Monovascular disease (coronary without PAD or CeVD) | 268/8,683 (3.1) | 305/8,687 (3.5) | 0.87 (0.74, 1.03) | 0.0058 | 0.4 (-0.1, 1.0) | 0.0025 | 236 |
| | Disease in two vascular beds (coronary and PAD) | 28/302 (9.3) | 27/308 (8.8) | 1.11 (0.68, 1.81) | | -0.5 (-5.1, 4.0) | | NA |
| | Disease in two vascular beds (coronary and CeVD) | 34/406 (8.4) | 43/389 (11.1) | 0.76 (0.49, 1.17) | | 2.7 (-6.8, 1.4) | | 37 |
| | Disease in three vascular beds (coronary, PAD, and CeVD) | 4/71 (5.6) | 17/78 (21.8) | 0.20 (0.07, 0.57) | | 16.2 (5.5, 26.8) | | 6 |
| | All patients | 334/9,462 (3.5) | 392/9,462 (4.1) | 0.85 (0.77, 0.98) | | 0.6 (0.1, 1.2) | | 163 |

| | | | | | | | | |
|---|---|---|---|---|---|---|---|---|
| HRs reflect stratification by geographic region in models with interaction between treatment, prior PAD, and prior CeVD. *Based on observed incidences over a median of 2.8 years of follow-up. ARR = absolute risk reduction; CeVD = cerebrovascular disease; CI = confidence interval; HR = hazard ratio; MACE = major adverse cardiovascular event; NNT = number needed to treat; PAD = peripheral artery disease. | | | | | | | | |

**Table 35. Primary MACE endpoint and all-cause death by History of PAD or CeVD Category**

| | | **Alirocumab n/N (%)** | **Placebo n/N (%)** | **HR (95% CI)** | **Log-rank p-value** | **ARR (95% CI)** | **NNT*** |
|---|---|---|---|---|---|---|---|
| Primary MACE | | | | | | | |
| | No PAD or CeVD | 740/8,683 (8.5) | 866/8,687 (10.0) | 0.85 (0.77, 0.93) | 0.0008 | 1.4 (0.6, 2.3) | 69 |
| | PAD (including CeVD) | 88/373 (23.4) | 104/386 (26.9) | 0.83 (0.62, 1.10) | 0.19 | 3.4 (-2.8, 9.5) | 30 |
| | CeVD (including PAD) | 94/477 (19.7) | 113/467 (24.2) | 0.80 (0.61, 1.05) | 0.10 | 4.5 (-0.8, 9.8) | 22 |
| | PAD and CeVD | 19/71 (26.8) | 31/78 (39.7) | 0.63 (0.35, 1.12) | 0.11 | 13.0 (-2.0, 28.0) | 8 |
| | All patients | 903/9,462 (9.5) | 1,052/9,462 (11.1) | 0.85 (0.78, 0.93) | 0.0003 | 1.6 (0.7, 2.4) | 64 |
| All-cause death | | | | | | | |
| | No PAD or CeVD | 268/8,683 (3.1) | 305/8,687 (3.5) | 0.88 (0.75, 1.04) | 0.12 | 0.4 (-0.1, 1.0) | 236 |
| | PAD (including CeVD) | 32/373 (8.6) | 44/386 (11.4) | 0.71 (0.45, 1.12) | 0.14 | 2.8 (-1.4, 7.1) | 35 |
| | CeVD (including PAD) | 38/477 (8.0) | 60/467 (12.8) | 0.56 (0.37, 0.84) | 0.0047 | 4.9 (1.0, 8.8) | 20 |
| | PAD and CeVD | 4/71 (5.6) | 17/78 (21.8) | 0.23 (0.08, 0.68) | 0.0038 | 16.2 (5.5, 26.8) | 6 |
| | All patients | 334/9,462 (3.5) | 392/9,462 (4.1) | 0.85 (0.77, 0.98) | 0.0261 | 0.6 (0.1, 1.2) | 163 |

| | | | | | | | |
|---|---|---|---|---|---|---|---|
| HRs and p-values reflect stratification by geographic region. ARR, absolute risk reduction; CeVD, cerebrovascular disease; CI, confidence interval; HR, hazard ratio; NNT, number needed to treat; PAD, peripheral artery disease. | | | | | | | |

### d. Safety Outcomes

Overall, there were no differences in the incidence of adverse events or laboratory abnormalities between alirocumab and placebo groups, with the exception of local injection-site reactions, which occurred more often in the alirocumab group. **Table 36** shows all safety endpoints for alirocumab versus placebo for patients with monovascular (coronary) disease, polyvascular disease in two beds (split by PAD or CeVD), and polyvascular disease in three beds. No major differences were observed between the groups.

**Table 36. Safety Endpoints**

| | **Monovascular Disease** | | **Disease in Two Vascular Beds** | | **Disease in Two Vascular Beds** | | **Disease in Three Vascular Beds** | |
|---|---|---|---|---|---|---|---|---|
| | **(coronary without PAD or CeVD)** | | **(coronary and PAD)** | | **(coronary and CeVD)** | | **(coronary, PAD, and CeVD)** | |
| | **Alirocum ab (n=8,672)** | **Placebo (n=8,66 8)** | **Alirocum ab (n=302)** | **Placeb o (n=308 )** | **Alirocum ab (n=406)** | **Placeb o (n=389 )** | **Alirocum ab (n=71)** | **Placeb o (n=78)** |
| Any adverse event | 6,532 (75.3) | 6,619 (76.4) | 250 (82.8) | 262 (85.1) | 321 (79.1) | 328 (84.3) | 62 (87.3) | 73 (93.6) |
| Serious adverse event | 1,905 (22.0) | 2,012 (23.2) | 124 (41.1) | 142 (46.1) | 136 (33.5) | 151 (38.8) | 37 (52.1) | 45 (57.7) |
| Adverse event that led to death | 143 (1.6) | 175 (2.0) | 15 (5.0) | 15 (4.9) | 22 (5.4) | 24 (6.2) | 1 (1.4) | 8 (10.3) |
| Adverse event that led to treatment discontinuati on | 298 (3.4) | 285 (3.3) | 21 (7.0) | 15 (4.9) | 19 (4.7) | 18 (4.6) | 5 (7.0) | 6 (7.7) |
| Local injection-site reaction | 339 (3.9) | 185 (2.1) | 8 (2.6) | 4 (1.3) | 9 (2.2) | 11 (2.8) | 4 (5.6) | 3 (3.8) |
| General allergic reaction | 670 (7.7) | 643 (7.4) | 31 (10.3) | 38 (12.3) | 41 (10.1) | 45 (11.6) | 6 (8.5) | 10 (12.8) |
| Diabetes worsening or diabetic complication in patients with diabetes at baseline | 444/2,369 (18.7) | 521/2,4 27 (21.5) | 23/99 (2.3) | 28/126 (22.2) | 29/188 (15.4) | 32/161 (19.9) | 10/32 (31.3) | 2/33 (6.1) |
| New-onset diabetes among patients without diabetes at baseline* | 595/6,303 (9.4) | 617/6,2 41 (9.9) | 26/203 (12.8) | 22/182 (12.1) | 24/218 (11.0) | 32/228 (14.0) | 3/39 (7.7) | 5/45 (11.1) |
| Neurocogniti ve disorder | 120 (1.4) | 143 (1.6) | 6 (2.0) | 10 (3.2) | 9 (2.2) | 10 (2.6) | 8 (11.3) | 4 (5.1) |
| Hepatic disorder | 450 (5.2) | 493 (5.7) | 19 (6.3) | 18 (5.8) | 24 (5.9) | 21 (5.4) | 7 (9.9) | 2 (2.6) |
| Cataracts | 99 (1.1) | 117 (1.3) | 8 (2.6) | 9 (2.9) | 10 (2.5) | 5 (1.3) | 3 (4.2) | 3 (3.8) |
| Hemorrhagic stroke, adjudicated (fatal and nonfatal) | 10 (0.1) | 13 (0.1) | 1 (0.3) | 1 (0.3) | 2 (0.5) | 3 (0.8) | 0 | 0 |

| | | | | | | | | |
|---|---|---|---|---|---|---|---|---|
| Values are n/N (%) or n (%). * New-onset diabetes was defined according to the presence of one or more of the following, with confirmation of the diagnosis by blinded external review by experts in the field of diabetes: an adverse-event report, a new prescription for diabetes medication, a glycated hemoglobin level of ≥6.5% on two occasions (and a baseline level of <6.5%), or a fasting serum glucose level of ≥126 mg/dl (7.0 mmol/l) on two occasions (and a baseline level of <126 mg/dl). | | | | | | | | |

### CONCLUSION

In patients with ACS and dyslipidemia despite intensive statin therapy, patients with polyvascular disease, including a history of concurrent PAD, CeVD, or both, was associated with markedly higher risk of MACE and death. In these patients, treatment with alirocumab resulted in larger ARR than in patients without concurrent PAD or CeVD (**Figure 30**). Therefore, patients with polyvascular disease should be considered preferred candidates for alirocumab treatment after ACS.

### Example 10: Secondary Analysis - Effects of Alirocumab in Patients with Prior Coronary Artery Bypass Grafting

### a. Baseline Characteristics by Prior CABG Status

Patients with prior coronary artery bypass grafting (CABG) have extensive native coronary and bypass graft atherosclerosis and are at increased risk for CV events and death. In a secondary analysis of the ODYSSEY OUTCOMES trial, patients were categorized by CABG status: (1) no CABG (n=16896); (2) CABG after the index ACS but before randomization, including 44 patients who had also undergone prior CABG before the index ACS (index CABG; n=1025); or (3) CABG prior to the index ACS (prior CABG; n=1003) (**Table 37**). Compared with patients with no prior CABG or index CABG, those with prior CABG were older, less frequently female, more frequently had prior MI, stroke, PAD, diabetes, and hypertension. Further, those with prior CABG more frequently had a non-ST-segment elevation MI (NSTEMI) as their index ACS, were more likely to be receiving a renin-angiotensin system (RAS) inhibitor and less likely to receive a beta-blocker. Prior CABG patients were less frequently receiving high dose statin and more frequently receiving other lipid-lowering therapy (including ezetimibe) with higher baseline LDL-C, non-HDL-C, apoB, and lipoprotein(a) values. Within each CABG category, there were no significant differences in baseline characteristics between those assigned to alirocumab or to placebo treatment. Among those receiving alirocumab with no prior CABG (27.2%), those undergoing CABG for the index ACS (28.3%), and those with prior CABG (34.5%), the dose was blindly increased from 75 to 150 mg after randomization per protocol (**Table 37**). The numbers of patients undergoing blinded adjustment of alirocumab dose according to protocol was similar in each of the CABG categories (**Table 37**).

**Table 37. Baseline Characteristics by Prior CABG Status**

| | | **No CABG (n=16896)** | **Index CABG (n=1025)** | **Prior CABG (n=1003)** | **P-value** |
|---|---|---|---|---|---|
| Age, years | | 58 (51, 65) | 60 (53, 65) | 63 (57, 70) | <0.0001 |
| Age category | | | | | <0.0001 |
| | <65 years | 12563 (74.4) | 726 (70.8) | 551 (54.9) | |
| | 65 to <75 years | 3479 (20.6) | 255 (24.9) | 343 (34.2) | |
| | ≥75 years | 854 (5.0) | 44 (4.3) | 109 (10.9) | |
| Female sex | | 4354 (25.8) | 211 (20.6) | 197 (19.6) | <0.0001 |
| Region | | | | | <0.0001 |
| | Western Europe | 3767 (22.3) | 230 (22.4) | 178 (17.7) | |
| | Eastern Europe | 5049 (29.9) | 180 (17.6) | 208 (20.7) | |
| | North America | 2273 (13.5) | 247 (24.1) | 351 (35.0) | |
| | South America | 2293 (13.6) | 180 (17.6) | 115 (11.5) | |
| | Asia | 2196 (13.0) | 56 (5.5) | 41 (4.1) | |
| | Rest of world | 1318 (84.5) | 132 (8.5) | 110 (11.0) | |
| Index event | | | | | <0.0001 |
| | NSTEMI | 7913 (46.9) | 612 (59.8) | 650 (64.9) | |
| | STEMI | 6156 (36.5) | 214 (20.9) | 166 (16.6) | |
| | Unstable angina | 2798 (16.6) | 198 (19.3) | 186 (18.6) | |
| Time from index event to randomization, months | | 2.6 (1.7, 4.2) | 3.8 (2.7, 6.8) | 2.5 (1.7, 4.1) | <0.0001 |
| Lipid-lowering therapy at Randomization | | | | | <0.0001 |
| | High dose atorvastatin/ rosuvastatin | 15077 (89.2) | 900 (87.8) | 834 (83.2) | |
| | Other Lipid Lowering Therapy | 1690 (10.0) | 107 (10.4) | 138 (13.8) | |
| | No Lipid Lowering Therapy | 129 (0.8) | 18 (1.8) | 31 (3.1) | |
| LDL-C, mg/dL | | 86 (73, 103) | 87 (74, 107) | 94 (78, 116) | <0.0001 |
| LDL-C ≥100 mg/dL | | 4878 (28.9) | 333 (32.5) | 418 (41.7) | <0.0001 |
| HDL-C, mg/dL | | 42 (36, 50) | 42 (36, 50) | 42 (35, 50) | 0.0166 |
| Non-HDL-C, mg/dL | | 114 (99, 136) | 117 (102, 140) | 126 (107, 153) | <0.0001 |
| Triglyceride, mg/dL | | 128 (94, 181) | 137 (98, 192) | 139 (102, 195) | <0.0001 |
| Apolipoprotein B, mg/dL | | 79 (69, 92) | 82 (70, 96) | 85 (75, 103) | <0.0001 |
| Lipoprotein(a), mg/dL | | 20.3 (6.6, 58.1) | 27.8 (7.5, 69.0) | 33.2 (8.1, 79.9) | <0.0001 |
| C-Reactive Protein, mg/dL | | 0.16 (0.08, 0.38) | 0.19 (0.09, 0.47) | 0.18 (0.08, 0.41) | <0.0001 |
| Body Mass Index, kg/m² | | 27.9 (25.2, 31.0) | 27.9 (25.2, 31.0) | 29.2 (26.4, 32.4) | <0.0001 |
| Hemoglobin A1c, % | | 5.8 (5.5, 6.3) | 5.9 (5.5, 6.4) | 6.0 (5.6, 6.7) | <0.0001 |
| Estimated GFR <60 mL/min per 1.73 m² | | 2138 (12.7) | 141 (13.8) | 260 (25.9) | <0.0001 |
| Diabetes status | | | | | <0.0001 |
| | Diabetes | 4691 (27.8) | 346 (33.8) | 407 (40.6) | |
| | Pre-diabetes | 7482 (44.3) | 382 (37.3) | 382 (38.1) | |
| | Normoglycemia | 4723 (28.0) | 297 (29.0) | 214 (21.3) | |
| Smoking status | | | | | <0.0001 |
| | Current | 4258 (25.2) | 136 (13.3) | 166 (16.6) | |
| | Former | 6795 (40.2) | 526 (51.3) | 490 (48.9) | |
| | Never | 5842 (34.6) | 363 (35.4) | 347 (34.6) | |
| Medical history prior to index event | | | | | |
| | Hypertension | 10672 (63.2) | 703 (68.6) | 874 (87.1) | <0.0001 |
| | Myocardial infarction | 2748 (16.3) | 194 (18.9) | 697 (69.5) | <0.0001 |
| | Stroke | 501 (3.0) | 40 (3.9) | 70 (7.0) | <0.0001 |
| | Malignant disease | 450 (2.7) | 30 (2.9) | 52 (5.2) | <0.0001 |
| | COPD | 621 (3.7) | 51 (5.0) | 74 (7.4) | <0.0001 |
| | CABG | 0 | 44 (4.3) | 1003 (100) | <0.0001 |
| | PAD | 577 (3.4) | 58 (5.7) | 124 (12.4) | <0.0001 |
| | CeVD | 735 (4.4) | 74 (7.2) | 135 (13.5) | <0.0001 |
| Revascularization for index event | | 12070 (71.4) | 1025 (100) | 582 (58.0) | <0.0001 |
| | CABG | 0 | 1025 (100) | 0 | <0.0001 |
| | PCI | 12070 (71.4) | 117 (11.4) | 582 (58.0) | <0.0001 |
| Medications | | | | | |
| | Aspirin | 16160 (95.6) | 978 (95.4) | 948 (94.5) | n.s. |
| | P2Y₁₂ antagonist | 15280 (90.4) | 410 (40.0) | 851 (84.8) | <0.0001 |
| | ACE inhibitor/Angiotensin Receptor Blocker | 13245 (78.4) | 656 (64.0) | 815 (81.3) | <0.0001 |
| | Beta-blocker | 14227 (88.0) | 895 (87.3) | 868 (86.5) | 0.0045 |
| | Ezetimibe | 425 (2.5) | 40 (3.9) | 89 (8.9) | <0.0001 |
| Treatment Variables Among Alirocumab Treated Patients | | n=8466 | n=494 | n=502 | |
| | % increased to 150 mg SC Q2W | 2302 (27.2) | 140 (28.3) | 173 (34.5) | 0.0018 |
| | % switched to placebo | 665 (7.9) | 37 (7.5) | 28 (5.6) | n.s. |

| | | | | | |
|---|---|---|---|---|---|
| Values are median (quartile 1, quartile 3) or column percentages. n.s.: p>0.05 NSTEMI, Non-ST-elevation myocardial infarction; STEMI, ST-elevation myocardial infarction; LDL-C, low-density lipoprotein cholesterol; HDL-C, high-density lipoprotein cholesterol; GFR, glomerular filtration rate; COPD, chronic obstructive pulmonary disease; CABG, coronary artery bypass graft surgery; PAD, peripheral artery disease; CeVD, cerebrovascular disease; SC, subcutaneous; Q2W, every two weeks. | | | | | |

### b. Endpoints by Prior CABG Status

In each category, the relative hazard ratio (HR), absolute risk reductions (ARR), and number needed to treat for 2.8 years (NNT) to prevent 1 primary endpoint (MACE) or death with alirocumab was determined. The cumulative incidence of the primary composite endpoint was significantly higher among patients with prior CABG (27.7%) than among patients who underwent CABG during the index ACS hospitalization (7.1%) or those without CABG (9.5%) (**Table 38** and **Figure 31**). Patients with prior CABG also had higher rates of the individual components of the primary endpoint and of the secondary endpoints (**Table 38**).

**Table 38. Primary and secondary endpoints by prior CABG status**

| | | **n (%)** | | | **Index vs. No CABG** | **Prior vs. No CABG** |
|---|---|---|---|---|---|---|
| | | **No CABG (n=16896)** | **Index CABG (n=1025)** | **Prior CABG (n=1003)** | **HR (95% CI)*** | **HR (95% CI)*** |
| Primary endpoint | | 1604 (9.5) | 73 (7.1) | 278 (27.7) | 0.56 (0.44, 0.72) | 1.46 (1.27, 1.69) |
| | CHD death | 347 (2.1) | 17 (1.7) | 63 (6.3) | 0.54 (0.33, 0.89) | 1.34 (1.00, 1.81) |
| | Nonfatal MI | 1083 (6.4) | 50 (4.9) | 215 (21.4) | 0.59 (0.44, 0.79) | 1.62 (1.38, 1.91) |
| | Ischemic Stroke | 219 (1.3) | 11 (1.1) | 33 (3.3) | 0.69 (0.37, 1.29) | 1.14 (0.76, 1.71) |
| | Unstable Angina | 79 (0.5) | 5 (0.5) | 13 (1.3) | 0.80 (0.31, 2.05) | 1.64 (0.85, 3.17) |
| Secondary Endpoints | | | | | | |
| | Ischemia-driven coronary revascularization procedure | 1311 (7.8) | 57 (5.6) | 191 (19.0) | 0.65 (0.49, 0.85) | 1.57 (1.33, 1.86) |
| | CV death | 415 (2.5) | 22 (2.1) | 74 (7.4) | 0.60 (0.39, 0.93) | 1.33 (1.01, 1.75) |
| | All-cause death | 592 (3.5) | 40 (3.9) | 94 (9.4) | 0.81 (0.58, 1.13) | 1.24 (0.98, 1.58) |
| | Investigator-reported PAD event | 192 (1.1) | 18 (1.8) | 36 (3.6) | 1.20 (0.71, 2.03) | 1.05 (0.71, 1.57) |

| | | | | | | |
|---|---|---|---|---|---|---|
| * Reflects adjustment for sex, age, geographical region, diabetes status, smoking status, baseline LDL-C, baseline HDL-C, baseline BMI, renal function (GFR <60 vs. >= 60 mL/min per 1.73 m²), history of MI, history of stroke, history of COPD, history of PAD, PCI for index event | | | | | | |

Alirocumab reduced the primary endpoint in the overall study population (HR 0.85 [0.78-0.93]) with consistent relative risk reduction in each CABG category (**Table 39** and **Figure 31**). In contrast, absolute risk reduction for the primary endpoint with alirocumab was most pronounced among patients with prior CABG (6.4% [0.9%, 12.0%], NNT_{2.8 years}=16) compared with index CABG (0.9% [-2.3%, 4.0%]) and no prior CABG patients (1.3% [0.5%, 2.2%]), respectively (**Table 39** and **Figure 32**).

Findings were similar for secondary endpoints. Relative risk reduction with alirocumab was without heterogeneity according to CABG category, except for ischemia-driven coronary revascularization. Absolute reductions in the risk of secondary endpoints were numerically greater among patients with prior CABG, compared to the other two CABG categories, including CHD death (3.0% vs. 0.5% and 0%), non-fatal MI (2.2% vs. 1.2% and 0%), ischemic stroke (2.2% vs. -0.3% and 0.4%), coronary revascularization (4.6% vs. 3.7% and 0.7%), investigator-reported PAD events (2.8% vs. 0.3% and 0.3%), CV death (3.6% vs. 1.0% and 0.1%), and all-cause death (3.6% vs. 0.5% and 0.4%). Among patients with prior CABG, the NNT_{2.8 years} to prevent one death was 28. For each secondary endpoint except CHD death, there was a significant interaction of treatment and CABG category on absolute risk reduction (**Table 39** and **Figure 32**).

**Table 39. Primary and secondary endpoints by randomized treatment and according to prior CABG status**

| | | **Alirocumab n/N (%)** | **Placebo n/N (%)** | **HR (95% CI)** | **HR Interaction p-value** | **ARR (95% CI)** | **ARR Interaction p-value** |
|---|---|---|---|---|---|---|---|
| **Primary Composite Endpoint** | | | | | | | |
| | No CABG | 747/8466 (8.8) | 857/8430 (10.2) | 0.86 (0.78, 0.95) | 0.71 | 1.3 (0.5, 2.2) | 0.0007 |
| | Index CABG | 33/494 (6.7) | 40/531 (7.5) | 0.85 (0.54, 1.35) | | 0.9 (-2.3, 4.0) | |
| | Prior CABG | 123/502 (24.5) | 155/501 (30.9) | 0.77 (0.61, 0.98) | | 6.4 (0.9, 12.0) | |
| | All patients | 903/9462 (9.5) | 1052/9462 (11.1) | 0.85 (0.78, 0.93) | | 1.6 (0.7, 2.4) | |

| **CHD Death** | | | | | | | |
|---|---|---|---|---|---|---|---|
| | No CABG | 174/8466 (2.1) | 173/8430 (2.1) | 1.00 (0.81, 1.23) | 0.20 | 0 (-0.4, 0.4) | 0.09 |
| | Index CABG | 7/494 (1.4) | 10/531 (1.9) | 0.73 (0.28, 1.91) | | 0.5 (-1.1, 2.0) | |
| | Prior CABG | 24/502 (4.8) | 39/501 (7.8) | 0.62 (0.37, 1.03) | | 3.0 (0, 6.0) | |
| | All patients | 205/9462 (2.2) | 222/9462 (2.3) | 0.92 (0.76, 1.11) | | 0.2 (-0.2, 0.6) | |

| **Non-fatal MI** | | | | | | | |
|---|---|---|---|---|---|---|---|
| | No CABG | 503/8466 (5.9) | 580/8430 (6.9) | 0.86 (0.76, 0.97) | 0.87 | 0.9 (0.2, 1.7) | 0.0161 |
| | Index CABG | 21/494 (4.3) | 29/531 (5.5) | 0.74 (0.42, 1.30) | | 1.2 (-1.4, 3.8) | |
| | Prior CABG | 102/502 (20.3) | 113/501 (22.6) | 0.88 (0.67, 1.15) | | 2.2 (-2.8, 7.3) | |
| | All patients | 626/9462 (6.6) | 722 (7.6) | 0.86 (0.77, 0.96) | | 1.0 (0.3, 1.8) | |
| | | | | | | | |

| **Ischemic Stroke** | | | | | | | |
|---|---|---|---|---|---|---|---|
| | No CABG | 94/8466 (1.1) | 125 (1.5) | 0.75 (0.57, 0.98) | 0.37 | 0.4 (0, 0.7) | 0.0118 |
| | Index CABG | 6/494 (1.2) | 5/531 (0.9) | 1.24 (0.38, 4.05) | | -0.3 (-1.5, 1.0) | |
| | Prior CABG | 11/502 (2.2) | 22/501 (4.4) | 0.49 (0.24, 1.01) | | 2.2 (0, 4.4) | |
| | All patients | 111/9462 (1.2) | 152/9462 (1.6) | 0.73 (0.57, 0.93) | | 0.4 (0.1, 0.8) | |

| **Unstable Angina requiring hospitalization** | | | | | | | |
|---|---|---|---|---|---|---|---|
| | No CABG | 29/8466 (0.3) | 50/8430 (0.6) | 0.57 (0.36, 0.91) | 0.81 | 0.3 (0, 0.5) | 0.0397 |
| | Index CABG | 2/494 (0.4) | 3/531 (0.6) | 0.69 (0.12, 4.10) | | 0.2 (-0.7, 1.0) | |
| | Prior CABG | 6/502 (1.2) | 7/501 (1.4) | 0.85 (0.28, 2.52) | | 0.2 (-1.2, 1.6) | |
| | All patients | 37/9462 (0.4) | 60/9462 (0.6) | 0.61 (0.41, 0.92) | | 0.2 (0.1, 0.5) | |

| **Coronary Revascularization** | | | | | | | |
|---|---|---|---|---|---|---|---|
| | No CABG | 629/8466 (7.4) | 682/8430 (8.1) | 0.92 (0.82, 1.02) | 0.0319 | 0.7 (-0.2, 1.5) | 0.0013 |
| | Index CABG | 18/494 (3.6) | 39/531 (7.3) | 0.47 (0.27, 0.82) | | 3.7 (0.9, 6.5) | |
| | Prior CABG | 84/502 (16.7) | 107/501 (21.4) | 0.74 (0.55, 0.98) | | 4.6 (-0.2, 9.5) | |
| | All patients | 731/9462 (7.7) | 828/9462 (8.8) | 0.88 (0.79, 0.97) | | 1.0 (0.2, 1.8) | |

| **CV Death** | | | | | | | |
|---|---|---|---|---|---|---|---|
| | No CABG | 204/8466 (2.4) | 211/8430 (2.5) | 0.96 (0.79, 1.16) | 0.14 | 0.1 (-0.4, 0.6) | 0.0341 |
| | Index CABG | 8/494 (1.6) | 14/531 (2.6) | 0.59 (0.25, 1.42) | | 1.0 (-0.7, 2.8) | |
| | Prior CABG | 28/502 (5.6) | 46/501 (9.2) | 0.61 (0.38, 0.97) | | 3.6 (0.4, 6.8) | |
| | All patients | 240/9462 (2.5) | 271/9462 (2.9) | 0.88 (0.74, 1.05) | | 0.3 (-0.1, 0.8) | |

| | | | | | | | |
|---|---|---|---|---|---|---|---|
| | | | | | | | |

| **All-Cause Death** | | | | | | | |
|---|---|---|---|---|---|---|---|
| | No CABG | 278/8466 (3.3) | 314/8430 (3.7) | 0.88 (0.75, 1.03) | 0.48 | 0.4 (-0.1, 1.0) | 0.0304 |
| | Index CABG | 18/494 (3.6) | 22/531 (4.1) | 0.85 (0.46, 1.59) | | 0.5 (-1.9, 2.9) | |
| | Prior CABG | 38/502 (7.6) | 56/501 (11.2) | 0.67 (0.44, 1.01) | | 3.6 (0, 7.2) | |
| | All patients | 334/9462 (3.5) | 392/9462 (4.1) | 0.85 (0.77, 0.98) | | 0.6 (0.1, 1.2) | |

| **Investigator-Reported PAD Event** | | | | | | | |
|---|---|---|---|---|---|---|---|
| | No CABG | 82/8466 (1.0) | 110/8430 (1.3) | 0.74 (0.56, 0.99) | 0.30 | 0.3 (0, 0.7) | 0.0053 |
| | Index CABG | 8/494 (1.6) | 10/531 (1.9) | 0.81 (0.32, 2.06) | | 0.3 (-1.3, 1.9) | |
| | Prior CABG | 11/502 (2.2) | 25/501 (5.0) | 0.41 (0.20, 0.84) | | 2.8 (0.5, 5.1) | |
| | All patients | 101/9462 (1.1) | 145/9462 (1.5) | 0.69 (0.54, 0.89) | | 0.5 (0.1, 0.8) | |

| | | | | | | | |
|---|---|---|---|---|---|---|---|
| HR and HR interaction p-value reflect stratification by geographical region. | | | | | | | |

### CONCLUSION

Among patients with recent ACS and elevated atherogenic lipoproteins despite intensive statin therapy, those with prior CABG derived a large absolute reduction in fatal and non-fatal cardiovascular events with alirocumab treatment.

### Example 11: Secondary Analysis - Effects of Alirocumab in Patients with Type 1 and Type 2 Myocardial Infarctions

LDL cholesterol (LDL-C) reduction with statins and PCSK9 inhibitors reduces risk of myocardial infarction (MI), but effects on specific types of MI are unknown. In a pre-specified analysis of the ODYSSEY OUTCOMES trial, the occurrence and outcomes of the types of Mls (according to the 3rd Universal Definition) in patients with recent acute coronary syndrome (ACS) and elevated LDL-C despite intensive statin therapy who were randomised to receive the PCSK9 inhibitor alirocumab or placebo and followed for cardiovascular outcomes. Overall a post-randomization MI occurred in 1383 (7.3%) patients (1860 total MIs). Of these, 991 (71.7%) patients had a total of 1223 type 1 MIs (42 were considered fatal), 287 (20.1%) had a total of 386 type 2 MIs (1 was considered fatal), 225 (16.3%) had a type 4 MI, and the remainder of 7 (<0.01%) had type 3 or type 5 MI.

Baseline characteristics of patients with type 1 and type 2 MI are shown in **Table 40.** Patients with type 1 MI, compared to patients with type 2 MI, were older, more likely female, more likely to be black, to be from North America or Europe, and to have a history of hypertension, congestive heart failure, pulmonary disease, diabetes, and malignant disease. LDL-C, Lp(a), and high sensitivity C-reactive protein (hsCRP) levels were higher in patients with MI than without MI. LDL-C and hsCRP did not differ between those with type 1 or type 2 MI, but Lp(a) was higher in patients with type 2 MI.

**Table 40. Baseline characteristics of patients with type 1 and 2 myocardial infarctions**

| **Characteristics** | | **(A) No Event (n=17719)** | **(B) First Event = Type 1 (n=963)** | **(C) First Event = Type 2 (n=242)** | **(A) vs. (B) vs. (C) P-value** | **(B) vs. (C) P-value** |
|---|---|---|---|---|---|---|
| Age, years | | 58 (52, 65) | 59 (52, 66) | 65 (59, 72) | <0.0001 | <0.0001 |
| Age category | | | | | <0.0001 | <0.0001 |
| | <65 years | 13069 (73.8) | 651 (67.6) | 120 (49.6) | | |
| | 65 to <75 years | 3757 (21.2) | 243 (25.2) | 77 (31.8) | | |
| | ≥75 years | 893 (5.0) | 69 (7.2) | 45 (18.6) | | |
| Female sex | | 4416 (24.9) | 267 (27.7) | 79 (32.6) | 0.0039 | n.s. |
| Race | | | | | <0.0001 | n.s. |
| | White | 14039 (79.2) | 788 (81.8) | 197 (81.4) | | |
| | Asian | 2390 (13.5) | 86 (8.9) | 22 (9.1) | | |
| | Black | 411 (2.3) | 44 (4.6) | 18 (7.4) | | |
| | Other | 879 (5.0) | 45 (4.7) | 5 (2.1) | | |
| Region | | | | | <0.0001 | 0.0290 |
| | Western Europe | 3894 (22.0) | 232 (24.1) | 49 (20.2) | | |
| | Eastern Europe | 5185 (29.3) | 199 (20.7) | 53 (21.9) | | |
| | North America | 2555 (14.4) | 234 (24.3) | 82 (33.9) | | |
| | South America | 2469 (13.9) | 103 (10.7) | 16 (6.6) | | |
| | Asia | 2194 (12.4) | 80 (8.3) | 19 (7.9) | | |
| | Rest of world | 1422 (8.0) | 115 (11.9) | 23 (9.5) | | |
| Index event | | | | | <0.0001 | n.s. |
| | NSTEMI | 8443 (47.7) | 587 (61.0) | 145 (59.9) | | |
| | STEMI | 6209 (35.1) | 259 (26.9) | 68 (28.1) | | |
| | Unstable angina | 3037 (17.2) | 116 (12.1) | 29 (12.0) | | |
| Time from index event to randomization, months | | 2.6 (1.7, 4.4) | 2.5 (1.7, 3.9) | 2.5 (1.7, 4.2) | 0.0369 | n.s. |
| Lipid lowering therapy at Randomization | | | | | <0.0001 | n.s. |
| | High dose atorvastatin/rosuvastatin | 15766 (89.0) | 845 (87.7) | 200 (82.6) | | |
| | Other LLT | 1802 (10.2) | 98 (10.2) | 35 (14.5) | | |
| | No LLT | 151 (0.9) | 20 (2.1) | 7 (2.9) | | |
| LDL-C, mg/dL | | 86 (73, 103) | 91 (76, 113) | 91 (75, 109) | <0.0001 | n.s. |
| LDL-C ≥100 mg/dL | | 5177 (29.2) | 365 (37.9) | 87 (36.0) | <0.0001 | n.s. |
| HDL-C, mg/dL | | 43 (36, 50) | 41 (35, 49) | 45 (37, 54) | <0.0001 | <0.0001 |
| Non-HDL-C, mg/dL | | 114 (99, 136) | 123 (105, 149) | 121 (103, 137) | <0.0001 | n.s. |
| Triglyceride, mg/dL | | 128 (94, 181) | 138 (100, 201) | 130 (90, 178) | <0.0001 | 0.0117 |
| ApoB, mg/dL | | 79 (69, 92) | 84 (72. 101) | 82 (71, 95) | <0.0001 | n.s. |
| Lp(a), mg/dL | | 20.8 (6.6, 59.0) | 25.4 (7.3, 70.0) | 34.9 (9.3, 76.8) | <0.0001 | n.s. |
| CRP, mg/dL | | 0.16 (0.08, 0.38) | 0.23 (0.10, 0.53) | 0.26 (0.11, 0.58) | <0.0001 | n.s. |
| BMI, kg/m2) | | 27.9 (25.2, 31.0) | 28.6 (25.7, 31.7) | 28.7 (25.7, 32.8) | <0.0001 | n.s. |
| HbA1c, % | | 5.8 (5.5, 6.3) | 6.0 (5.6, 6.8) | 6.0 (5.6, 6.9) | <0.0001 | n.s. |
| GFR, mL/min per 1.73 m² | | 78.5 (67.8, 90.4) | 76.0 (62.5, 88.3) | 67.4 (53.5, 83.7) | <0.0001 | <0.0001 |
| GFR <60 mL/min per 1.73 m² | | 2256 (12.7) | 199 (20.7) | 84 (34.7) | <0.0001 | <0.0001 |
| Current smoker | | 4261 (24.0) | 252 (26.2) | 47 (19.4) | n.s. | 0.0305 |

| Medical history prior to index event | | | | | | |
|---|---|---|---|---|---|---|
| | Diabetes | 4924 (27.8) | 404 (42.0) | 116 (47.9) | <0.0001 | n.s. |
| | Hypertension | 11277 (63.6) | 758 (78.1) | 214 (88.4) | <0.0001 | 0.0005 |
| | Myocardial infarction | 3174 (17.9) | 373 (38.7) | 92 (38.0) | <0.0001 | n.s. |
| | PCI | 2805 (15.8) | 347 (36.0) | 89 (36.8) | <0.0001 | n.s. |
| | CABG | 844 (4.8) | 162 (16.8) | 41 (16.9) | <0.0001 | n.s. |
| | Stroke | 541 (3.1) | 55 (5.7) | 15 (6.2) | <0.0001 | n.s. |
| | Malignant disease | 475 (2.7) | 36 (3.7) | 21 (8.7) | <0.0001 | 0.0033 |
| | COPD | 637 (3.6) | 68 (7.1) | 41 (16.9) | <0.0001 | <0.0001 |
| | PAD | 628 (3.5) | 89 (9.2) | 42 (17.4) | <0.0001 | 0.0007 |
| | CHF | 2542 (14.3) | 195 (20.2) | 78 (32.2) | <0.0001 | 0.0001 |
| Revascularization for index event | | 12886 (72.7) | 630 (65.4) | 161 (66.5) | <0.0001 | n.s. |
| | PCI | 12024 (67.9) | 595 (61.8) | 150 (62.0) | <0.0001 | n.s. |
| | CABG | 976 (5.5) | 38 (3.9) | 11 (4.5) | n.s. | n.s. |
| Medications | | | | | | |
| | Aspirin | 16960 (95.7) | 910 (94.5) | 216 (89.3) | <0.0001 | 0.0054 |
| | P2Y₁₂ antagonist | 15482 (87.4) | 855 (88.8) | 204 (82.3) | n.s. | n.s. |
| | ACE inhibitor/ARB | 13728 (77.5) | 793 (82.3) | 195 (80.6) | 0.0011 | n.s. |
| | Beta-blocker | 14951 (84.4) | 833 (86.5) | 206 (85.1) | n.s. | n.s. |
| | Ezetimibe | 472 (2.7) | 68 (7.1) | 14 (5.8) | <0.0001 | n.s. |
| Randomized to Placebo | | 8808 (49.7) | 512 (53.2) | 142 (58.7) | 0.0028 | n.s. |

| | | | | | | |
|---|---|---|---|---|---|---|
| Values are median (quartile 1, quartile 3) or column percentages. n.s.: p>0.05 ACE, angiotensin converting enzyme; ARB, angiotensin II receptor blocker; CABG, coronary artery bypass grafting; CHF, congestive heart failure; COPD, chronic obstructive pulmonary disease; CV, cardiovascular; LDL-C = low-density lipoprotein cholesterol; LLT, lipid lowering therapy; NSTEMI, non-ST-elevation myocardial infarction; PAD, peripheral artery disease; PCI, percutaneous coronary intervention; STEMI, ST-elevation myocardial infarction. | | | | | | |

**Figure 33** shows a Kaplan-Meier curve of the occurrence of type 1 and type 2 MIs over time in patients treated with alirocumab and placebo. For type 1 MI the highest risk is in the first 12 months and then divergence of the curves and a lower annual risk thereafter. For type 2 MI there is a relatively consistent risk over time with divergence beginning approximately at 6 months.

Effects of alirocumab on each type of MI are shown in **Table 41.** Overall, alirocumab reduced the occurrence of all MIs compared with placebo (7.9% placebo versus 6.8% alirocumab, HR 0.85, 95% CI 0.77-0.95, p=0.003). Both type 1 MI (HR 0.87, 95% CI 0.77-0.99, p=0.0317) and type 2 Mls (HR 0.77, 95% CI 0.61-0.97, p=0.0251) were reduced. There was no observed effect of alirocumab treatment on MI types 3-5. For type 4 MI the HR was 0.94 (CI 0.72 - 1.22, p=0.62).

Most MI's (82.7%) were non-STEMI (ST-elevation myocardial infarction), and alirocumab significantly reduced these Mls (HR 0.82 (CI 0.72, 0.93), p=0.0020. There was no significant effect on STEMIs. Q wave MIs were a minority (10.5%) of patients with interpretable ECGs. There was no observed effect of alirocumab in Q wave Mls, whereas for non-Q wave Mls there was a reduction with alirocumab. (HR 0.86, CI 0.76, 0.97), p=0.0130. **Table 42** shows a sensitivity analysis of types of myocardial infarctions and effects of alirocumab, which includes patients dying on day 1 (n=7 alirocumab; n=11 placebo).

**Table 41: Types of myocardial infarctions and effects of alirocumab**

| | **Alirocumab** | | | | **Placebo** | | | **Treatment HR (95% CI)^{1,2}** | **P-value^{1,2}** |
|---|---|---|---|---|---|---|---|---|---|
| | **Patients with MI¹ n (%)** | **Total MIs** | | | **Patients with MI¹ n (%)** | **Total MIs** | | | |
| Any myocardial infarction (MI) | 639 (6.8) | 866 | | | 744 (7.9) | 994 | | 0.85 (0.77, 0.95) | 0.0031 |

| **Universal Classification** | | | | | | | | | |
|---|---|---|---|---|---|---|---|---|---|
| Type 1: spontaneous | 463 (4.9) | 560 | | | 528 (5.6) | 663 | | 0.87 (0.77, 0.99) | 0.0317 |
| Type 2: supply/demand imbalance | 125 (1.3) | 180 | | | 162 (1.7) | 206 | | 0.77 (0.61, 0.97) | 0.0251 |
| Type 3: cardiac death suggestive of MI without increased biomarkers | | 2 | | | | 0 | | - | - |
| Type 4A: peri-percutaneous coronary intervention | 22 (0.2) | 23 | | | 28 (0.3) | 29 | | 0.94 (0.72, 1.22) | 0.62 |
| Type 4B: stent thrombosis | 50 (0.5) | 55 | | | 46 (0.5) | 49 | | | |
| Type 4C: restenosis | 37 (0.4) | 44 | | | 42 (0.4) | 44 | | | |
| Type 5: peri-coronary artery bypass graft | | 2 | | | | 3 | | - | - |

| **ECG Classification:** | | | | | | | | | |
|---|---|---|---|---|---|---|---|---|---|
| NSTEMI | 437 (4.6) | | 576 | 529 (5.6) | | 692 | 0.82 (0.72, 0.93) | | 0.0020 |
| STEMI | 92 (0.5) | | 96 | 109 (1.2) | | 116 | 0.84 (0.64, 1.11) | | 0.22 |
| ECG not interpretable or not available | 161 (1.7) | | 194 | 162 (1.7) | | 186 | 1.01 (0.81, 1.25) | | 0.96 |

| **Q Wave Classification** | | | | | | | | | |
|---|---|---|---|---|---|---|---|---|---|
| Q Wave | 52 (0.5) | | 52 | 71 (0.9) | | 73 | 0.73 (0.51, 1.04) | | 0.08 |
| Non Q Wave | 483 (5.1) | | 634 | 560 (5.9) | | 725 | 0.86 (0.76, 0.97) | | 0.0130 |
| ECG not interpretable or available | 146 (1.5) | | 180 | 165 (1.7) | | 196 | 0.88 (0.71, 1.10) | | 0.27 |

| | | | | | | | | | |
|---|---|---|---|---|---|---|---|---|---|
| ¹ Corresponds to traditional time-to-first event analysis ² Reflects stratification by geographic region. NSTEMI, non-ST-elevation myocardial infarction; STEMI, ST-elevation myocardial infarction | | | | | | | | | |

**Table 42. Sensitivity analyses of types of myocardial infarctions and effects of alirocumab including events on date of death**

| | **Alirocumab** | **Placebo** | | |
|---|---|---|---|---|
| | **Patients with MI¹ n (%)** | **Patients with MI¹ n (%)** | **Treatment HR (95% CI)¹** | **P-value¹** |
| Any myocardial infarction (MI) | 646 (6.8) | 755 (8.0) | 0.85 (0.77, 0.94) | 0.0023 |
| Type 1: spontaneous | 464 (4.9) | 534 (5.6) | 0.86 (0.76, 0.98) | 0.0215 |
| Type 2: supply/demand imbalance | 126 (1.3) | 163 (1.7) | 0.77 (0.61, 0.97) | 0.0256 |
| Type 3: cardiac death suggestive of MI without increased biomarkers | 10 (<0.1) | 5 (<0.1) | - | - |
| Type 4A: peri-percutaneous coronary intervention | 22 (0.2) | 28 (0.3) | 0.94 (0.72, 1.22) | 0.62 |
| Type 4B: stent thrombosis | 50 (0.5) | 46 (0.5) | | |
| Type 4C: restenosis | 37 (0.4) | 42 (0.4) | | |
| Type 5: peri-coronary artery bypass grafting | 2 (<0.1) | 3 (<0.1) | - | - |

**Table 43** shows the effect of alirocumab on biomarker levels, predominantly cardiac troponin (92%) at various cut-points. Alirocumab treatment was associated with no apparent reduction in smaller Mls (with peak biomarker levels <3 times the upper limit of normal) but with large reductions in larger Mls as defined by peak biomarker value.

**Table 43. First MI by biomarker elevation level^{a}.**

| **Multiple of upper limit of normal** | | **Alirocumab** | **Placebo** | | |
|---|---|---|---|---|---|
| | **Events *n* (%)** | **Patients with MI¹ *n* (%)** | **Patients with MI^{a} *n* (%)** | **Treatment HR (95% CI)^{b}** | ***P*-value^{b}** |
| 1 to <3 | 406 (29.4) | 200 (2.1) | 206 (2.2) | 0.97 (0.79-1.17) | 0.72 |
| 3 to <10 | 304 (22.0) | 133 (1.4) | 171 (1.8) | 0.77 (0.62-0.97) | 0.026 |
| 10 to <100 | 468 (33.8) | 213 (2.3) | 255 (2.7) | 0.83 (0.69-1.00) | 0.044 |
| ≥100 | 167 (12.1) | 73 (0.8) | 94 (1.0) | 0.77 (0.57-1.04) | 0.09 |
| Missing | 38 (2.7) | 20 (0.2) | 18 (0.2) | 1.10 (0.58-2.08) | 0.77 |

| | | | | | |
|---|---|---|---|---|---|
| ^{a}For the 1383 first Mis: troponin available for 1272 (92.0%); CK-MB for 73 (5.3%); missing both troponin and CKMB for 38 (2.7%). ^{b}Analysis by Cox proportional hazards models, stratified by geographical region. CI, confidence interval; HR, hazard ratio; MI, myocardial infarction. | | | | | |

### a. Predictors of type 1 and type 2 myocardial infarctions

Candidate predictors for type 1 MI are age category, race, region, index event, lipid lowering therapy at randomization, LDL-C, HDL-C, Lp(a), BMI, GFR, diabetes, hypertension, myocardial infarction, stroke, PCI, COPD, CABG, PAD, CHF, revascularization for index event, and alirocumab treatment (significant predictors (p<0.05) in traditional Cox model).

Candidate predictors for type 2 Mls are age category, female sex, race, region, index event, lipid lowering therapy at randomization, LDL-C, HDL-C, Lp(a), BMI, GFR, current smoker, diabetes, hypertension, myocardial infarction, stroke, malignant disease, PCI, COPD, CABG, PAD, CHF, revascularization for index event, and alirocumab treatment (significant predictors (p<0.05) in traditional Cox model). **Tables 44** and **45** show the multivariable prediction models for type 1 and type 2 MI, respectively.

**Table 44. Multivariable prediction model for type 1 MI**

| | | **Type 1 MI** | |
|---|---|---|---|
| **Characteristic** | | **HR (95% CI)** | **p-value** |
| History of PCI | | 1.505 (1.266, 1.790) | <0.0001 |
| History of CABG | | 1.739 (1.446, 2.091) | <0.0001 |
| History of diabetes | | 1.572 (1.379, 1.793) | <0.0001 |
| History of PAD | | 1.609 (1.294, 2.002) | <0.0001 |
| History of hypertension | | 1.539 (1.307, 1.811) | <0.0001 |
| Region | | | <0.0001 |
| | Western Europe | Ref | |
| | Eastern Europe | 0.504 (0.413, 0.614) | |
| | North America | 0.930 (0.769, 1.125) | |
| | South America | 0.654 (0.512, 0.835) | |
| | Asia | 2.028 (0.966, 4.260) | |
| | Rest of world | 1.161 (0.924, 1.457) | |
| LDL-C, 1 mg/dL increment | | 1.004 (1.003, 1.006) | <0.0001 |
| History of myocardial infarction | | 1.463 (1.230, 1.740) | <0.0001 |
| History of CHF | | 1.327 (1.121, 1.571) | 0.0010 |
| GFR <60 mL/min per 1.73 m² | | 1.368 (1.167, 1.603) | 0.0001 |
| Revascularization for index event | | 0.796 (0.696, 0.910) | 0.0009 |
| Current smoker | | 1.228 (1.062, 1.420) | 0.0055 |
| Race | | | 0.0085 |
| | White | Ref | |
| | Asian | 0.376 (0.185, 0.765) | |
| | Black | 1.369 (1.005, 1.865) | |
| | Other | 0.968 (0.703, 1.333) | |
| Alirocumab treatment | | 0.870 0.768, 0.986) | 0.0288 |
| History of stroke | | 1.336 (1.023, 1.746) | 0.0337 |

**Table 45. Multivariable prediction model for type 2 MI**

| | | **Type 2 MI** | |
|---|---|---|---|
| **Characteristic** | | **HR (95% CI)** | **p-value** |
| GFR <60 mL/min per 1.73 m² | | 2.048 (1.569, 2.673) | <0.0001 |
| History of PAD | | 2.487 (1.826, 3.388) | <0.0001 |
| History of PCI | | 1.477 (1.089, 2.005) | 0.0123 |
| History of COPD | | 2.263 (1.629, 3.145) | <0.0001 |
| History of CHF | | 2.359 (1.803, 3.087) | <0.0001 |
| History of Diabetes | | 1.761 (1.379, 2.249) | <0.0001 |
| Region | | | <0.0001 |
| | Western Europe | Ref | |
| | Eastern Europe | 0.445 (0.304, 0.652) | |
| | North America | 1.163 (0.825, 1.637) | |
| | South America | 0.436 (0.250, 0.761) | |
| | Asia | 1.233 (0.361, 4.211) | |
| | Rest of world | 1.220 (0.768, 1.938) | |
| History of Hypertension | | 2.661 (1.785, 3.966) | <0.0001 |
| Age category | | | <0.0001 |
| | <65 years | Ref | |
| | 65 to <75 years | 1.455 (1.108, 1.913) | |
| | ≥75 years | 2.263 (1.584, 3.232) | |
| Race | | | 0.0211 |
| | White | Ref | |
| | Asian | 0.758 (0.243, 2.358) | |
| | Black | 1.915 (1.215, 3.016) | |
| | Other | 0.621 (0.265, 1.451) | |
| History of Myocardial infarction | | 1.491 (1.105, 2.011) | 0.0089 |
| HDL-C, 1 mg/dL increment | | 1.011 (1.002, 1.021) | 0.0173 |
| Alirocumab treatment | | 0.770 (0.610, 0.973) | 0.0288 |

Most of the significant factors were similar for both MI types. However, LDL-C, revascularization at the index event, current smoking, race, and previous stroke were significant for type 1 MI but were not significant for type 2 MI. In addition, age categories, history of chronic obstructive pulmonary disease, and baseline HDL-C levels were significant for type 2 MI and not for type 1 MI.

### b. Mortality of Patients with type 1 and type 2 myocardial infarctions

Mortality in patients with type 2 MI after randomization (n=283, 25.4%) was over twice that of patients with type 1 MI after randomization (n=73, 11.9%). Alirocumab reduced the frequency of type 2 MI, but did not reduce mortality following type 2 MI (24.8% vs 25.9%). In contrast, alirocumab compared with placebo reduced mortality following type 1 MI (10.2% versus 13.4%).

**Figure 34** shows a forest plot of type 1 MI or type 2 MI during follow-up as predictors for all-cause death. After adjustment for treatment and prognostic factors there was a significant effect on type 1 MI, p=0.03 but not for type 2 MI, p=0.61.

**Figure 35** shows forest plots for all-cause death, cardiovascular, and non-cardiovascular deaths before and after the occurrence of a type 1 or type 2 MI. The incidence of all-cause death before MI was similar for both type 1 and type 2 infarctions but approximately twice as high after type 2 MI as after type 1 MI. The reduction in all-cause death after type 1 MI with alirocumab was 47/463 (10.2%) vs 71/528 (13.4%) in the placebo group, HR 0.69 (CI 0.48, 1.00). After type 2 MI alirocumab had no apparent effect on mortality 31/125 alirocumab (24.8%) vs 42/162 (25.9%) HR 0.98 (CI 0.62, 1.56).

Cardiovascular mortality before MI was similar for both type 1 and type 2 MI. Cardiovascular mortality was higher after type 2 MI than after type 1 MI. There was no effect of alirocumab on cardiovascular mortality for either type of MI. For non-cardiovascular death, the rates were similar before MI for both type 1 and type 2 Mls. After MI, non-cardiovascular death rates were 3-4 higher in patients with type 2 MI than for patients with type 1 MI. There was no effect of alirocumab on non-cardiovascular deaths for type 1 or type 2 MI.

### CONCLUSION

In patients with ACS and elevated LDL-C despite intensive statin therapy, alirocumab reduced the incidence of both type 1 and type 2 Mls, and reduced mortality in type 1 MI, indicating that LDL-C reduction below levels achievable with statins is an effective preventive strategy for both types of MI.

### Example 12: Cardiovascular Efficacy of Alirocumab After Acute Coronary Syndromes in High-Risk Versus Very High-Risk Patients

The frequency of composite ischemic events and the efficacy of PCSK9 inhibition with alirocumab was examined in ODYSSEY OUTCOMES patients who were classified as "very high risk" for future atherosclerotic cardiovascular disease (ASCVD) events according to 2018 ACC/AHA guidelines. (Grundy SM, et al. JACC (2018) (in press - available online November 10, 2018)).

A total of 11,935 (63%) patients were categorized as very high-risk and 6,989 (37%) as not at very high-risk based upon 2018 ACC/AHA guidelines criteria. Criteria for "very high risk" included prior (before the index ACS event) ischemic events (of which 19.2% of patients had a prior myocardial infarction (MI), 2.8% had a prior ischaemic stroke, and 4.0% had prior PAD) or the presence of multiple high-risk clinical conditions (26.9% were ≥65 years of age, 28.8% had diabetes mellitus, 13.4% had chronic kidney disease, and 24.1% were current smokers).

Alirocumab treatment was associated with consistent relative reduction in the risk of MACE events in patients with very high risk features vs. patients without very high-risk features (Table 46). However, the absolute risk of MACE and the absolute reduction in MACE events with alirocumab were greater among patients classified as very high-risk with accentuated absolute benefit among those with multiple prior ischemic events compared with those with only other high-risk clinical conditions.

**Table 46. Time to first occurrence of primary endpoint**

| **Population** | | **Placebo n/N (%)** | **Alirocumab n/N (%)** | **Hazard ratio (95% CI)** | **Interaction p-value** |
|---|---|---|---|---|---|
| No additional very high-risk features | | 198/3525 (5.6%) | 168/3464 (4.8%) | 0.86 (0.70-1.06) | 0.83 |
| Additional very high-risk features | | 854/5937 (14.4%) | 735/5998 (12.3%) | 0.84 (0.76-0.92) | |
| Previous ASCVD events | | 458/2241 (20.4%) | 398/2209 (18.0%) | 0.86 (0.75-0.98) | 0.62 |
| | Myocardial infarction | 378/1843 (20.5%) | 333/1790 (18.6%) | 0.87 (0.75-1.01) | |
| | Ischemic stroke | 63/256 (24.6%) | 54/268 (20.1%) | 0.77 (0.54-1.12) | |
| | PAD | 104/386 (26.9%) | 88/373 (23.6%) | 0.83 (0.62-1.10) | |
| ≥1 high-risk clinical condition (no previous ASCVD event) | | 396/3696 (10.7%) | 337/3789 (8.9%) | 0.82 (0.71-0.95) | |

### CONCLUSION

Application of 2018 ACC/AHA cholesterol guidelines criteria for "very high risk" accurately identifies patients with ACS and dyslipidaemia who derive a large absolute benefit from alirocumab treatment. Patients categorized as very high-risk on the basis of multiple prior ischemic events derive a particularly large benefit from this approach.

### Example 13. Effects of Alirocumab According to Background Statin Treatment

In this analysis, the efficacy of alirocumab or placebo was compared among ODYSSEY OUTCOMES patients with varying statin therapy backgrounds. All 18,924 patients were categorized by background statin therapy at randomization: high-intensity (atorvastatin 40-80 mg/d or rosuvastatin 20-40 mg/d) (n=16,811, 88.8%); low- or moderate-intensity (atorvastatin <40 mg/d or rosuvastatin <20 mg/d) (n=1653, 8.7%); or no statin use (n=460, 2.4%). In the study, no background statin was allowed in cases of statin intolerance, which was established with documented intolerance to ≥2 statins. The relative risk (hazard ratio [HR]) and absolute risk reductions (ARR) for MACE were determined for all patient groups.

Hazard ratios (HRs) were consistent across statin categories (**Table 47**). Baseline LDL-C increased across high-intensity, low/moderate-intensity, and no statin categories. There was a gradient of the risk of MACE in the placebo group across these categories (10.8%, 10.7%, and 26%, respectively). With alirocumab treatment, the mean reduction in LDL-C from baseline to Month 4 was greater across the three categories (-1.37 mmol/L, -1.47 mmol/L, and -2.27 mmol/L, respectively) and the ARRs for MACE across categories were 1.3%, 3.2%, and 8.0%, respectively (P_{interaction} <.001).

**Table 47. LDL-C values and MACE events stratified by intensity of statin therapy.**

| | **All patients** | | **High-intensity statin** | | **Low-/moderate-intensity statin** | | **No statin** | | **Interaction P-value** |
|---|---|---|---|---|---|---|---|---|---|
| | **N=18,924 (100%)** | | **N=16,811 (88.8%)** | | **N=1653 (8.7%)** | | **N=460 (2.4%)** | | |
| | **PBO** | **ALI** | **PBO** | **ALI** | **PBO** | **ALI** | **PBO** | **ALI** | |
| LDL-C (SE) (mmol/L) at baseline [mg/dL] | 2.39 (0.01) [92 (31)] | 2.39 (0.01) [92 (31)] | 2.35 (0.01) [91 (29)] | 2.35 (0.01) [91 (29)] | 2.41 (0.03) [93 (29)] | 2.43 (0.03) [94 (29] | 3.76 (0.08) [145 (48)] | 3.82 (0.08) [148 (45)] | |
| LDL-C (SE) (mmol/L): month 4 change from baseline [mg/dL] | 0.03 (0.01) [1 (0)] | -1.4 (0.01) [-54 (0)] | 0.03 (0.01) [1 (0)] | -1.37 (0.01) [-53 (0)] | 0.01 (0.02) [0 (1)] | -1.47 (0.02) [-56 (1)] | -0.004 (0.06) [0 (2)] | -2.27 (0.06) [-86 (2)] | <0.001 |
| MACE, n (%) | 1052 (11.1) | 903 (9.5) | 907 (10.8) | 797 (9.5) | 86 (10.7) | 34 (7.5) | 59 (26.0) | 42 (18.0) | |
| HR (95% CI) | 0.85 (0.78-0.93) | | 0.88 (0.80-0.96) | | 0.69 (0.50-0.95) | | 0.65 (0.43-0.96) | | 0.14 |
| ARR (%) (95% CI) | 1.6 (0.7-2.4) | | 1.3 (0.3-2.2) | | 3.2 (0.4-5.9) | | 8.0 (0.4-15.5) | | <0.001 |

### CONCLUSION

Among ODYSSEY OUTCOMES patients, those who are not receiving statin treatment showed a higher mean baseline LDL-C (3.82 mmol/L) and a greater mean absolute reduction in LDL-C (-2.27 mmol/L) with alirocumab treatment than patients receiving statin treatment. Patients not receiving statin treatment also showed a greater absolute risk reduction in MACE (8.0% (0.4-15.5)) compared to those on high-intensity statin treatment (1.3% (0.3-2.2) P_{interaction} <.001). Thus, patients not receiving statin treatment, including patients who are statin-intolerant, or for whom a statin is contraindicated, may be an important group to consider for treatment with alirocumab after ACS.

## Claims

1. A proprotein convertase subtilisin/kexin type 9 (PCSK9) antibody or antigen-binding fragment thereof for use in a method for reducing the risk of major adverse cardiovascular events (MACE) comprising:
administering to a patient in need thereof a therapeutically effective amount of said PCKS9 antibody or antigen-binding fragment thereof for at least three years, wherein the antibody or antigen-binding fragment thereof comprises complementarity determining regions (CDRs) of a heavy chain variable region (HCVR) and a light chain variable region (LCVR) comprising amino acid sequences set forth in SEQ ID NOs: 1 and 6, respectively; and
wherein the patient is a high cardiovascular risk patient who had an acute coronary syndrome (ACS) event between about 1 to about 12 months prior to the administration of the antibody or antigen binding fragment thereof,
wherein the patient has an LDL-C level greater than or equal to 100 mg/dL despite treatment with maximally tolerated statin therapy.

2. The PCSK9 antibody or antigen-binding fragment thereof for use of claim 1, wherein the MACE comprises coronary heart disease, myocardial infarction, ischemic stroke, or unstable angina.

3. The PCSK9 antibody or antigen-binding fragment thereof for use of any one of claims 1 to 2, wherein the patient has:
(a) clinical atherosclerotic cardiovascular disease (ASCVD);
(b) peripheral artery disease (PAD);
(c) cerebrovascular disease (CeVD); or
(d) polyvascular disease.

4. The PCSK9 antibody or antigen-binding fragment thereof for use of any one of claims 1 to 3, wherein the patient has had a prior coronary artery bypass graft (CABG).

5. The PCSK9 antibody or antigen-binding fragment thereof for use of any one of claims 1 to 4 wherein the antibody or antigen-binding fragment thereof comprises heavy and light chain CDR amino acid sequences having SEQ ID NOs: 2, 3, 4, 7, 8, and 10.

6. The PCSK9 antibody or antigen-binding fragment thereof for use of any one of claims 1 to 5, wherein the administration step comprises:
(i) administering to the patient one or more initial doses of 75 mg of the antibody or antigen-binding fragment thereof about every two weeks; and
(ii) administering to the patient one or more following doses of 150 mg of the antibody or antigen-binding fragment thereof about every two weeks if the LDL-C level in the patient after step (i) is greater than or equal to a threshold level.

7. The PCSK9 antibody or antigen-binding fragment thereof for use of claim 6, wherein the threshold level is 50 mg/dL.

8. The PCSK9 antibody or antigen-binding fragment thereof for use of any one of claims 1 to 5, wherein the administering step comprises administering to the patient:
(a) one or more doses of 75 mg of the antibody or antigen-binding fragment thereof about every two weeks; or
(b) one or more doses of 150 mg of the antibody or antigen-binding fragment thereof about every two weeks; or
(c) one or more doses of 300 mg of the antibody or antigen-binding fragment thereof about every four weeks.

9. The PCSK9 antibody or antigen-binding fragment thereof for use of any one of claims 1 to 8, wherein the antibody or antigen-binding fragment is administered to the patient in combination with maximally tolerated statin therapy.

10. The PCSK9 antibody or antigen-binding fragment thereof for use of claim 9, wherein the maximally tolerated statin therapy comprises:
(a) a daily dose of about 40 mg to about 80 mg of atorvastatin;
(b) a daily dose of about 20 mg to about 40 mg of rosuvastatin;
(c) a low or moderate dose of atorvastatin or rosuvastatin; or
(d) a statin other than atorvastatin or rosuvastatin.

## Patentansprüche

1. Proprotein-Convertase-Subtilisin/Kexin-Typ-9(PCSK9)-Antikörper oder antigenbindendes Fragment davon zur Verwendung bei einem Verfahren zum Verringern des Risikos für schwerwiegende unerwünschte kardiovaskuläre Ereignisse (MACE) umfassend:
Verabreichen einer therapeutisch wirksamen Menge des PCKS9-Antikörpers oder antigenbindenden Fragments davon an einen Patienten mit Bedarf daran über wenigstens drei Jahre, wobei der Antikörper oder das antigenbindende Fragment davon komplementaritätsbestimmende Regionen (CDRs) einer variablen Region der schweren Kette (HCVR) und einer variablen Region der leichten Kette (LCVR) umfasst, umfassend Aminosäuresequenzen dargelegt in SEQ ID NO: 1 bzw. 6; und
wobei der Patient ein Patient mit hohem kardiovaskulären Risiko ist, der ein akutes Koronarsyndrom(ACS)-Ereignis zwischen etwa 1 und etwa 12 Monaten vor der Verabreichung des Antikörpers oder antigenbindenden Fragments davon erfahren hat,
wobei der Patient trotz Behandlung mit maximal tolerierter Statin-Therapie einen LDL-C-Spiegel von höher als oder gleich 100 mg/dl aufweist.

2. PCSK9-Antikörper oder antigenbindendes Fragment davon zur Verwendung nach Anspruch 1, wobei das MACE koronare Herzerkrankung, Myokardinfarkt, ischämischen Schlaganfall oder instabile Angina umfasst.

3. PCSK9-Antikörper oder antigenbindendes Fragment davon zur Verwendung nach einem der Ansprüche 1 bis 2, wobei der Patient aufweist:
(a) klinische atherosklerotische kardiovaskuläre Erkrankung (ASCVD);
(b) periphere Arterienerkrankung (PAD);
(c) zerebrovaskuläre Erkrankung (CeVD); oder
(d) polyvaskuläre Erkrankung.

4. PCSK9-Antikörper oder antigenbindendes Fragment davon zur Verwendung nach einem der Ansprüche 1 bis 3, wobei der Patient ein früheres Koronararterien-Bypass-Transplantat (CABG) hatte.

5. PCSK9-Antikörper oder antigenbindendes Fragment davon zur Verwendung nach einem der Ansprüche 1 bis 4, wobei der Antikörper oder das antigenbindende Fragment davon CDR-Aminosäuresequenzen der schweren und leichten Kette mit SEQ ID NO: 2, 3, 4, 7, 8 und 10 umfasst.

6. PCSK9-Antikörper oder antigenbindendes Fragment davon zur Verwendung nach einem der Ansprüche 1 bis 5, wobei der Verabreichungsschritt umfasst:
(i) Verabreichen einer oder mehrerer Anfangsdosen von 75 mg des Antikörpers oder antigenbindenden Fragments davon an den Patienten etwa alle zwei Wochen; und
(ii) Verabreichen einer oder mehrerer nachfolgender Dosen von 150 mg des Antikörpers oder antigenbindenden Fragments davon an den Patienten etwa alle zwei Wochen, wenn der LDL-C-Spiegel in dem Patienten nach Schritt (i) höher als oder gleich einem Schwellenwert ist.

7. PCSK9-Antikörper oder antigenbindendes Fragment davon zur Verwendung nach Anspruch 6, wobei der Schwellenwert 50 mg/dl beträgt.

8. PCSK9-Antikörper oder antigenbindendes Fragment davon zur Verwendung nach einem der Ansprüche 1 bis 5, wobei der Verabreichungsschritt Verabreichen an den Patienten umfasst:
(a) eine oder mehrere Dosen von 75 mg des Antikörpers oder antigenbindenden Fragments davon etwa alle zwei Wochen; oder
(b) eine oder mehrere Dosen von 150 mg des Antikörpers oder antigenbindenden Fragments davon etwa alle zwei Wochen; oder
(c) eine oder mehrere Dosen von 300 mg des Antikörpers oder antigenbindenden Fragments davon etwa alle vier Wochen.

9. PCSK9-Antikörper oder antigenbindendes Fragment davon zur Verwendung nach einem der Ansprüche 1 bis 8, wobei der Antikörper oder das antigenbindende Fragment davon an den Patienten in Kombination mit maximal tolerierter Statin-Therapie verabreicht wird.

10. PCSK9-Antikörper oder antigenbindendes Fragment davon zur Verwendung nach Anspruch 9, wobei die maximal tolerierte Statin-Therapie umfasst:
(a) eine tägliche Dosis von etwa 40 mg bis etwa 80 mg Atorvastatin;
(b) eine tägliche Dosis von etwa 20 mg bis etwa 40 mg Rosuvastatin;
(c) eine niedrige oder mäßige Dosis von Atorvastatin oder Rosuvastatin; oder
(d) ein von Atorvastatin und Rosuvastatin verschiedenes Statin.

## Revendications

1. Anticorps de proprotéine convertase subtilisine/kexine de type 9 (PCSK9) ou fragment de liaison à un antigène de celui-ci pour une utilisation dans un procédé pour réduire le risque d'événements cardiovasculaires indésirables majeurs (MACE) comprenant :
l'administration à un patient nécessitant celle-ci d'une quantité thérapeutiquement efficace dudit anticorps PCKS9 ou fragment de liaison à un antigène de celui-ci pendant au moins trois ans, dans lequel l'anticorps ou fragment de liaison à un antigène de celui-ci comprend des régions déterminant la complémentarité (CDR) d'une région variable de chaîne lourde (HCVR) et une région variable de chaîne légère (LCVR) comprenant des séquences d'acides aminés décrites dans SEQ ID NO: 1 et 6, respectivement ; et
dans lequel le patient est un patient à risque cardiovasculaire élevé qui a présenté un événement de syndrome coronarien aigu (SCA) entre environ 1 et environ 12 mois avant l'administration de l'anticorps ou du fragment de liaison à un antigène de celui-ci,
dans lequel le patient a un taux de LDL-C supérieur ou égal à 100 mg/dl malgré un traitement avec une thérapie de statine tolérée maximale.

2. Anticorps PCSK9 ou fragment de liaison à un antigène de celui-ci pour utilisation de la revendication 1, dans lequel le MACE comprend une coronaropathie, un infarctus du myocarde, un accident vasculaire cérébral ischémique ou une angine instable.

3. Anticorps PCSK9 ou fragment de liaison à un antigène de celui-ci pour utilisation de l'une quelconque des revendications 1 à 2, dans lequel le patient a :
(a) maladie cardiovasculaire athérosclérotique clinique (ASCVD) ;
(b) maladie des artères périphériques (PAD) ;
(c) maladie cérébrovasculaire (CeVD) ; ou
(d) maladie polyvasculaire.

4. Anticorps PCSK9 ou fragment de liaison à un antigène de celui-ci pour utilisation de l'une quelconque des revendications 1 à 3, dans lequel le patient a eu une greffe de pontage de l'artère coronaire antérieure (CABG).

5. Anticorps de PCSK9 ou fragment de liaison à un antigène de celui-ci pour utilisation de l'une quelconque des revendications 1 à 4, dans lequel l'anticorps ou fragment de liaison à un antigène de celui-ci comprend des séquences d'acides aminés de CDR de chaîne lourde et légère ayant SEQ ID NO: 2, 3, 4, 7, 8, et 10.

6. Anticorps de PCSK9 ou fragment de liaison à un antigène de celui-ci pour utilisation selon l'une quelconque des revendications 1 à 5, dans lequel l'étape d'administration comprend :
(i) l'administration au patient d'une ou plusieurs doses initiales de 75 mg de l'anticorps ou fragment de liaison à un antigène de celui-ci environ toutes les deux semaines ; et
(ii) l'administration au patient d'une ou plusieurs doses suivantes de 150 mg de l'anticorps ou fragment de liaison à un antigène de celui-ci environ toutes les deux semaines si le taux de LDL-C chez le patient après l'étape (i) est supérieur ou égal à un taux seuil.

7. Anticorps PCSK9 ou fragment de liaison à un antigène de celui-ci pour utilisation de la revendication 6, dans lequel le taux seuil est de 50 mg/dl.

8. Anticorps PCSK9 ou fragment de liaison à un antigène de celui-ci pour utilisation de l'une quelconque des revendications 1 à 5, dans lequel l'étape d'administration comprend l'administration au patient de :
(a) une ou plusieurs doses de 75 mg de l'anticorps ou fragment de liaison à un antigène de celui-ci environ toutes les deux semaines ; ou
(b) une ou plusieurs doses de 150 mg de l'anticorps ou fragment de liaison à un antigène de celui-ci environ toutes les deux semaines ; ou
(c) une ou plusieurs doses de 300 mg de l'anticorps ou fragment de liaison à un antigène de celui-ci environ toutes les quatre semaines.

9. Anticorps PCSK9 ou fragment de liaison à un antigène de celui-ci pour une utilisation selon l'une quelconque des revendications 1 à 8, dans lequel l'anticorps ou le fragment de liaison à un antigène est administré au patient en combinaison avec une thérapie de statine tolérée maximale.

10. Anticorps PCSK9 ou fragment de liaison à un antigène de celui-ci pour utilisation de la revendication 9, dans lequel la thérapie de statine tolérée maximale comprend :
(a) une dose quotidienne d'environ 40 mg à environ 80 mg d'atorvastatine ;
(b) une dose quotidienne d'environ 20 mg à environ 40 mg de rosuvastatine ;
(c) une dose faible ou modérée d'atorvastatine ou de rosuvastatine ; ou
(d) une statine autre que l'atorvastatine ou la rosuvastatine.
